# EUROPEAN PATENT SPECIFICATION

(11) **EP 1 208 204 B1**
(45) Date of publication and mention of the grant of the patent: **27.08.2008**
(21) Application number: 00957908.7
(22) Date of filing: 30.08.2000
(51) Int. Cl.: C12N 15/29, C07K 14/415, C12N 15/82, C12N 5/10, A01H 1/00, A01H 5/10

(54) **PLANT REPRODUCTION PROTEINS**
VERMEHRUNGSPROTEINE VON PFLANZEN
PROTEINES DE REPRODUCTION DESTINEES AUX PLANTES

(30) Priority: 31.08.1999 US 151575 P
(43) Date of publication of application: 29.05.2002
(73) Proprietor: E.I. DU PONT DE NEMOURS AND COMPANY, Wilmington DE 19898 (US); PIONEER HI-BRED INTERNATIONAL, INC., Des Moines, Iowa 50309 (US)
(72) Inventor: BUTLER, Karlene H., Newark, DE 19713 (US); DANILEVSKAYA, Olga, Johnston, IA 50131 (US); MIAO, Guo-Hua, Johnston, IA 50131 (US); MORGANTE, Michele, Wilmington, DE 19810 (US); SAKAI, Hajime, Newark DE 19711 (US); SIMMONS, Carl R., Des Moines, IA 50310 (US); WENG, Zude, Des Plaines, IL 60016 (US)
(74) Representative: Bentham, Andrew
(86) International application number: PCT/US2000/023735
(87) International publication number: WO 2001/016325

(56) References cited:
- OHAD ET AL.: "Mutations in FIE, a WD polycomb group gene, allow endosperm development without fertilization" THE PLANT CELL, vol. 11, March 1999 (1999-03), pages 407-415, XP002158154
- WALBOT, V.: "605065H09.x1 605 - Endosperm cDNA library from Schmidt lab Zea mays cDNA, mRNA sequence" EMBL SEQUENCE DATABASE, 9 June 1999 (1999-06-09), XP002158155 HEIDELBERG DE
- SASAKI, T.: "Rice cDNA, partial sequence" EMBL SEQUENCE DATABASE, 6 August 1997 (1997-08-06), XP002158156 HEIDELBERG DE
- BLEWITT ET AL.: "BNLGHi6867 Six-day cotton fiber Gossypium hirsutum cDNA 5' similar to (AF003604) extra sex combs [Schistocerca americana], mRNA sequence" EMBL SEQUENCE DATABASE, 12 June 1999 (1999-06-12), XP002158157 HEIDELBERG DE
- OHAD NIR ET AL: "A mutation that allows endosperm development without fertilization" PROCEEDINGS OF THE NATIONAL ACADEMY OF SCIENCES OF USA,NATIONAL ACADEMY OF SCIENCE. WASHINGTON,US, vol. 93, no. 11, 1996, pages 5319-5324, XP002136432 ISSN: 0027-8424
- CORDONNIER-PRATT ET AL.: "DG1_87_G07.b1_A002 Dark grown 1 (DG1) Sorgum bicolor cDNa, mRNA sequence" EMBL SEQUENCE DATABASE, 28 July 2000 (2000-07-28), XP002158158 HEIDELBERG DE

## Description

### FIELD OF THE INVENTION

This invention is in the field of plant molecular biology. More specifically, this invention pertains to nucleic acid fragments encoding reproduction proteins in plants and seeds.

### BACKGROUND OF THE INVENTION

Apomixis is the formation of seeds without fertilization. Most seed formation in plants involves sexual gamete exchange between different plants or within the same plant, however, apomixis does naturally occur especially in polyploid plant varieties/species.

The control of seed formation through apomixis is anticipated to have an enormous impact to agriculture. Generally apomixis has not been exploited in agriculture, however if apomixis could be controlled in various crops it would revolutionize agriculture because genetically identical seed could be produced without the need of gamete fertilization. One could easily visualize immortalization of a high yielding crop plant varieties and apomixis could be exploited by the seed industry to reduce the costs of maintaining pure inbred lines. Also, seed formation without fertilization avoids factors that could affect the efficiency of seed set, such as pollen count and pollen viability, and stigma or tassel emergence or viability. Apomixis would also allow the immediate stable incorporation of transgenes without the need for selfing to produce homozygotes.

The use of the feterilization-independent endosperm gene and other related genes could be to form fertilization independent endosperms without necessarily forming viable embyros. Such seed would not germinate because it doesn't have an embryo. The endosperms, if sufficiently formed, could nonetheless be used for human and animal food and for commercial milling and extraction. Such embyro-less seeds would have the added advantage of allowing containment of genetically modified organisms to satisfy environmental and regulatory concerns. Such seeds could also be independently modified to produce novel products in the endosperm such as pharmaceuticals, nutraceuticals, and industrial compounds and polymers.

Identification of specific genes involved in apomixis, such as fertilization-independent endosperm genes, should offer new ways of producing apomictic plants. Such approaches may involve selective mutagenesis of fertilization-independent endosperm genes and then tracking of the mutant alleles in a (molecular) breeding program, or by transgenic methods. Accordingly, the availability of nucleic acid sequences encoding all or a portion of a fertilization-independent endosperm protein would facilitate studies to better understand development regulation in plants, provide genetic tools to control apomixis.

### SUMMARY OF THE INVENTION

The present invention provides an isolated polynucleotide encoding a polypeptide that has the effect of altering endosperm formation, comprising at least 250 amino acids, wherein:
(a) the amino acid sequence of the polypeptide and SEQ ID NO: 2 have at least 80% identity based on the Clustal alignment method; or
(b) the nucleotide sequence of the polynucleotide and SEQ ID NO: I have at least 80% identity based on the Clustal alignment method.
Preferably:
(a) the amino acid sequence of the polypeptide and SEQ ID NO: 2 have at least 85% or 95% identity based on the Clustal alignment method; or
(b) the nucleotide sequence and SEQ ID NO: 1 or SEQ ID NO: 29 have at least 85% or 95% identity based on the Clustal alignment method.

In one embodiment, the polypeptide comprises SEQ ID NO: 2.

In another embodiment, the polynucleotide comprises SEQ ID NO: 1 or SEQ ID NO: 29.

Preferably, the polypeptide is a fertilization independent endosperm (FIE) protein.

The invention also provide a complement of the polynucleotide of the invention, wherein the complement and the polynucleotide contain the same number of nucleotides and are 100% complementary.

The invention also provides a method for transforming a cell comprising introducing into a cell the polynucleotide of the invention that encodes a polypeptide as defined above that has the effect of altering endosperm formation.

The invention also provides a method for transforming a cell comprising introducing the complement defined above into a cell.

Preferably, in either of these methods, the cell is a plant cell. The plant cell may be a monocot or a dicot plant cell.

The invention also provides the isolated polypeptide encoded by the polynucleotide of the invention. Preferably, said polynucleotide is a fertilization independent endosperm (FIE) protein.

The invention also provides a chimeric gene in which the polynucleotide of the invention is operably linked to a promoter.

The invention also provides a plasmid vector comprising an isolated polynucleotide of the invention or a chimeric gene of the invention.

The invention also provides a plant expression vector comprising a polynucleotide of the invention under the transcriptional control of 5' and 3' regulatory sequences and a dominant selectable marker.

The invention also provides a host cell comprising a polynucleotide or chimeric gene of the invention. Preferably, the host cell is a plant cell. The plant cell may be a monocot plant cell or a dicot plant cell.

The invention also provides a method for transforming a plant comprising the transfer of a polynucleotide of the invention into, said plant resulting in stable inheritance. The plant may be a monocot or a dicot.

The invention also provides a transgenic host plant stably transformed with a polynucleotide of the invention. Preferably, said host plant is a host plant in which a polypeptide of the invention is present at higher levels than normal or in cell types or development or stages in which it is not normally found; and wherein said polypeptide has an effect of altering endosperm function. Said plant may be a monocot or dicot plant.

The invention also provides a plant in which expression of an endogenous gene encoding a polypeptide of the invention is reduced or eliminated, such that said polypeptide is present at lower levels than normal and endosperm formation is altered. In some embodiments, said reduction for elimination may be via antisense inhibition or co-suppression. Said plant may be a monocot or a dicot plant.

Where the present invention concerns an isolated host cell comprising a chimeric gene of the present invention or an isolated polynucleotide of the present invention, the host cell may be eukaryotic, such as a yeast or a plant cell, or prokaryotic, such as a bacterial cell. The present invention also relates to a virus, preferably a baculovirus, comprising an isolated polynucleotide of the present invention or a chimeric gene of the present invention.

The invention also relates to a process for producing and isolated host cell comprising a chimeric gene of the present invention or an isolated polynucleotide of the present invention, the process comprising either transforming or transfecting an isolated compatible host cell with a chimeric gene or isolated polynucleotide of the present invention.

The inventiont also relates to a method of selecting an isolated polynucleotide that affects the level of expression of a fertilization-independent endosperm protein polypeptide or enzyme activity in a host cell, preferably a plant cell, the method comprising the steps of: (a) constructing an isolated polynucleotide of the present invention or an isolated chimeric gene of the present invention; (b) introducing the isolated polynucleotide or the isolated chimeric gene into a host cell; (c) measuring the level of the fertilization-independent endosperm protein polypeptide or enzyme activity in the host cell containing the isolated polynucleotide; and (d) comparing the level of the fertilization-independent endosperm protein polypeptide or enzyme activity in the host cell containing the isolated polynucleotide with the level of the fertilization-independent endosperm protein polypeptide or enzyme activity in the host cell that does not contain the isolated polynucleotide.

The invention also relates to a method of obtaining a nucleic acid fragment encoding a substantial portion of a fertilization-independent endosperm polypeptide, preferably a plant fertilization-independent endosperm polypeptide, comprising the steps of : synthesizing an oligonucleotide primer comprising a nucleotide sequence of at least one of 60 (preferably at least one of 40, most preferably at least one of 30) contiguous nucleotides derived from a nucleotide sequence selected from of SEQ ID No: 1, 29 or 39, 41, 43, 45, 47, and the complement of such nucleotide sequences; and amplifying a nucleic acid fragment (preferably a cDNA inserted in a cloning vector) using the oligonucleotide primer. The amplified nucleic acid fragment preferably will encode a substantial portion of a fertilization-independent endosperm protein amino acid sequence.

This invention also relates to a method of obtaining a nucleic acid fragment encoding all or a substantial portion of the amino acid sequence encoding a fertilization-independent endosperm polypeptide comprising the steps of: probing a cDNA or genomic library with an isolated polynucleotide of the present invention; identifying a DNA clone that hybridizes with an isolated polynucleotide of the present invention; isolating the identified DNA clone; and sequencing the cDNA or genomic fragment that comprises the isolated DNA clone.

This invention also relates to a composition, such as a hybridization mixture, comprising an isolated polynucleotide of the present invention.

This invention also relates to a method for positive selection of a transformed cell comprising: (a) transforming a host cell with the chimeric gene of the present invention or an expression cassette of the present invention; and (b) growing the transformed host cell, preferably a plant cell, such as a monocot or a dicot, under conditions which allow expression of the fertilization-independent endosperm polynucleotide in an amount sufficient to complement a null mutant to provide a positive selection means.

This invention also relates to a method of altering the level of expression of a reproduction protein in a host cell comprising: (a) transforming a host cell with a chimeric gene of the present invention; and (b) growing the transformed host cell under conditions that are suitable for expression of the chimeric gene wherein expression of the chimeric gene results in production of altered levels of the reproduction protein in the transformed host cell.

### BRIEF DESCRIPTION OF THE SEQUENCE LISTINGS

The invention can be more fully understood from the following detailed description and the accompanying Sequence Listing which form a part of this application.

Table I lists the polypeptides that are described herein, the designation of the cDNA clones and chromosomal sequences that comprise the nucleic acid fragments encoding polypeptides representing all or a substantial portion of these polypeptides, and the corresponding identifier (SEQ ID NO:) as used in the attached Sequence Listing.

**TABLE 1**

| Reproduction Proteins | | | |
|---|---|---|---|
| | | SEQ ID NO: | |
| Protein | Clone Designation | (Nucleotide) | (Amino Acid) |
| Fertilization-independent endosperm protein | ccase-b.pk0026.g4 (CGS) | 1 | 2 |
| Fertilization-independent endosperm protein | cen1.mn0001.g10 (CGS) | 3 | 4 |
| Fertilization-independent | cen3n.pk0076.b8 (CGS) | 5 | 6 |
| endosperm protein | | | |
| Fertilization-independent | cpb1c.pk001.d10 (FIS) | 7 | 8 |
| endosperm protein | | | |
| Fertilization-independent | eec1c.pk003.e23 (CGS) | 9 | 10 |
| endosperm protein | | | |
| Fertilization-independent | hlplc.pk003.e8 (FIS) | 11 | 12 |
| endosperm protein | | | |
| Fertilization-independent | ncs.pk0019.h3 (CGS) | 13 | 14 |
| endosperm protein | | | |
| Fertilization-independent | p0003.cgpfn34f (EST) | 15 | 16 |
| endosperm protein | | | |
| Fertilization-independent | p0003.cgped29rb (CGS) | 17 | 18 |
| endosperm protein | | | |
| Fertilization-independent | p0003.cgpfn34rb (EST) | 19 | 20 |
| endosperm protein | | | |
| Fertilization-independent | p0037.crwao47r (FIS) | 21 | 22 |
| endosperm protein | | | |
| Fertilization-independent | p0041.crtaw93r (FIS) | 23 | 24 |
| endosperm protein | | | |
| Fertilization-independent | p0101.cgamg48r (CGS) | 25 | 26 |
| endosperm protein | | | |
| Fertilization-independent | p0104.cabbn62r (CGS) | 27 | 28 |
| endosperm protein | | | |
| Fertilization-independent | p0107.cbcai79r (CGS) | 29 | 30 |
| endosperm protein | | | |
| Fertilization-independent | p0119.cmtoh49r (CGS) | 31 | 32 |
| endosperm protein | | | |
| Fertilization-independent | p0120.cdebd48r (FIS) | 33 | 34 |
| endosperm protein | | | |
| Fertilization-independent | real1c.pk0001.d2 (CGS) | 35 | 36 |
| endosperm protein | | | |
| Fertilization-independent | ses2w.pk0015.b10 (CGS) | 37 | 38 |
| endosperm protein | | | |
| Fertilization-independent | wkm1c.pk0003.f4 (CGS) | 39 | 40 |
| endosperm protein | | | |
| Fertilization-independent | ccase-b.pk0026.g4 (EST) | 41 | 42 |
| endosperm protein | | | |
| Fertilization-independent | cen1.mn0001.g10 (EST) | 43 | 44 |
| endosperm protein | | | |
| Fertilization-independent | cpb1c.pk001.d10 (EST) | 45 | 46 |
| endosperm protein | | | |
| Fertilization-independent | eec1c.pk003.e23 (EST) | 47 | 48 |
| endosperm protein | | | |
| Fertilization-independent | hlp1c.pk003.e8 (EST) | 49 | 50 |
| endosperm protein | | | |
| Fertilization-independent | ncs.pk0019.h3 (EST) | 51 | 52 |
| endosperm protein | | | |
| Fertilization-independent | p0003.cgped29rb (EST) | 53 | 54 |
| endosperm protein | | | |
| Fertilization-independent | p0037.crwao47r (EST) | 55 | 56 |
| endosperm protein | | | |
| Fertilization-independent | p0041.crtaw93r (EST) | 57 | 58 |
| endosperm protein | | | |
| Fertilization-independent | p0104.cabbn62r (EST) | 59 | 60 |
| endosperm protein | | | |
| Fertilization-independent | p0107.cbcai79r (CGS) | . 61 | 62 |
| endosperm protein | | | |
| Fertilization-independent | p0120.cdebd48r (EST) | 63 | 64 |
| endosperm protein | | | |
| Fertilization-independent | rcal 1c.pk0001.d2 (EST) | 65 | 66 |
| endosperm protein | | | |
| Fertilization-independent | ses2w.pk0015.b10 (EST) | 67 | 68 |
| endosperm protein | | | |
| Fertilization-independent | wkm1c.pk0003.f4 (EST) | 69 | 70 |
| endosperm protein | | | |
| Fertilization-independent | Genomic Sequence | 71 | |
| endosperm protein | | | |
| Fertilization-independent | Genomic Sequence | 72 | |
| endosperm protein | | | |

SEQ ID NOs: 71 and 72 represent two different genes.

The Sequence Listing contains the one letter code for nucleotide sequence characters and the three letter codes for amino acids as defined in conformity with the IUPAC-IUBMB standards described in Nucleic Acids Res. 13:3021-3030 (1985) and in the Biochemical J. 219 (No. 2):345-373 (1984).

### DETAILED DESCRIPTION OF THE INVENTION

In the context of this disclosure, a number of terms shall be utilized. The terms "polynucleotide", "polynucleotide sequence", "nucleic acid sequence", and "nucleic acid fragment"/"isolated nucleic acid fragment" are used interchangeably herein. These terms encompass nucleotide sequences and the like. A polynucleotide may be a polymer of RNA or DNA that is single- or double-stranded, that optionally contains synthetic, non-natural or altered nucleotide bases. A polynucleotide in the form of a polymer of DNA may be comprised of one or more segments of cDNA, genomic DNA, synthetic DNA, or mixtures thereof.

The term "isolated" polynucleotide refers to a polynucleotide that is substantially free from other nucleic acid sequences, such as and not limited to other chromosomal and extrachromosomal DNA and RNA. Isolated polynucleotides may be purified from a host cell in which they naturally occur. Conventional nucleic acid purification methods known to skilled artisans may be used to obtain isolated polynucleotides. The term also embraces recombinant polynucleotides and chemically synthesized polynucleotides.

The term "recombinant" means, for example, that a nucleic acid sequence is made by an artificial combination of two otherwise separated segments of sequence, e.g., by chemical synthesis or by the manipulation of isolated nucleic acids by genetic engineering techniques.

As used herein, "contig" refers to a nucleotide sequence that is assembled from two or more constituent nucleotide sequences that share common or overlapping regions of sequence homology. For example, the nucleotide sequences of two or more nucleic acid fragments can be compared and aligned in order to identify common or overlapping sequences. Where common or overlapping sequences exist between two or more nucleic acid fragments, the sequences (and thus their corresponding nucleic acid fragments) can be assembled into a single contiguous nucleotide sequence.

As used herein, "substantially similar" refers to nucleic acid fragments wherein changes in one or more nucleotide bases results in substitution of one or more amino acids, but do not affect the functional properties of the polypeptide encoded by the nucleotide sequence. "Substantially similar" also refers to nucleic acid fragments wherein changes in one or more nucleotide bases does not affect the ability of the nucleic acid fragment to mediate alteration of gene expression by gene silencing through for example antisense or co-suppression technology. "Substantially similar" also refers to modifications of the nucleic acid fragments of the instant invention such as deletion or insertion of one or more nucleotides that do not substantially affect the functional properties of the resulting transcript vis-A-vis the ability to mediate gene silencing or alteration of the functional properties of the resulting protein molecule. It is therefore understood that the invention encompasses more than the specific exemplary nucleotide or amino acid sequences and includes functional equivalents thereof. The terms "substantially similar" and "corresponding substantially" are used interchangeably herein.

Substantially similar nucleic acid fragments may be selected by screening nucleic acid fragments representing subfragments or modifications of the nucleic acid fragments of the instant invention, wherein one or more nucleotides are substituted, deleted and/or inserted, for their ability to affect the level of the polypeptide encoded by the unmodified nucleic acid fragment in a plant or plant cell. For example, a substantially similar nucleic acid fragment representing at least one of 30 contiguous nucleotides derived from the instant nucleic acid fragment can be constructed and introduced into a plant or plant cell. The level of the polypeptide encoded by the unmodified nucleic acid fragment present in a plant or plant cell exposed to the substantially similar nucleic fragment can then be compared to the level of the polypeptide in a plant or plant cell that is not exposed to the substantially similar nucleic acid fragment.

For example, it is well known in the art that antisense suppression and co-suppression of gene expression may be accomplished using nucleic acid fragments representing less than the entire coding region of a gene, and by using nucleic acid fragments that do not share 100% sequence identity with the gene to be suppressed. Moreover, alterations in a nucleic acid fragment which result in the production of a chemically equivalent amino acid at a given site, but do not effect the functional properties of the encoded polypeptide, are well known in the art. Thus, a codon for the amino acid alanine, a hydrophobic amino acid, may be substituted by a codon encoding another less hydrophobic residue, such as glycine, or a more hydrophobic residue, such as valine, leucine; or isoleucine. Similarly, changes which result in substitution of one negatively charged residue for another, such as aspartic acid for glutamic acid, or one positively charged residue for another, such as lysine for arginine, can also be expected to produce a functionally equivalent product. Nucleotide changes which result in alteration of the N-terminal and C-terminal portions of the polypeptide molecule would also not be expected to alter the activity of the polypeptide. Each of the proposed modifications is well within the routine skill in the art, as is determination of retention of biological activity of the encoded products. Consequently, an isolated polynucleotide comprising a nucleotide sequence of at least one of 60 (preferably at least one of 40, most preferably at least one of 30) contiguous nucleotides derived from a nucleotide sequence selected from the group consisting of SEQ ID Nos:1, 29 and 39, and the complement of such nucleotide sequences may be used in methods of selecting an isolated polynucleotide that affects the expression of a fertilization-independent endosperm polypeptide in a host cell. A method of selecting an isolated polynucleotide that affects the level of expression of a polypeptide in a virus or in a host cell (eukaryotic, such as plant or yeast, prokaryotic such as bacterial) may comprise the steps of: constructing an isolated polynucleotide or an isolated chimeric gene comprising the desired sequence introducing the isolated polynucleotide or the isolated chimeric gene into a host cell; measuring the level of a polypeptide or enzyme activity in the host cell containing the isolated polynucleotide; and comparing the level of a polypeptide or enzyme activity in the host cell containing the isolated polynucleotide with the level of a polypeptide or enzyme activity in a host cell that does not contain the isolated polynucleotide.

Moreover, substantially similar nucleic acid fragments may also be characterized by their ability to hybridize. Estimates of such homology are provided by either DNA-DNA or DNA-RNA hybridization under conditions of stringency as is well understood by those skilled in the art (Hames and Higgins, Eds. (1985) Nucleic Acid Hybridisation, IRL Press, Oxford, U.K.). Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions. One set of preferred conditions uses a series of washes starting with 6X SSC, 0.5% SDS at room temperature for 15 min, then repeated with 2X SSC, 0.5% SDS at 45°C for 30 min, and then repeated twice with 0.2X SSC, 0.5% SDS at 50°C for 30 min. A more preferred set of stringent conditions uses higher temperatures in which the washes are identical to those above except for the temperature of the final two 30 min washes in 0.2X SSC, 0.5% SDS was increased to 60°C. Another preferred set of highly stringent conditions uses two final washes in 0.1X SSC, 0.1 % SDS at 65°C.

Substantially similar nucleic acid fragments of the instant invention may also be characterized by the percent identity of the amino acid sequences that they encode to the amino acid sequences disclosed herein, as determined by algorithms commonly employed by those skilled in this art. Suitable nucleic acid fragments (isolated polynucleotides of the present invention) encode polypeptides that are at least 80% preferably about 85%, more preferably at least about 90% identical to the amino acid sequence of SEQ ID NO:2. Most preferred are nucleic acid fragments that encode amino acid sequences that are at least about 95% identical to the amino acid sequence of SEQ ID NO:2. Suitable nucleic acid fragments not only have the above identities but typically encode a polypeptide having at least 250 amino acids. Sequence alignments and percent identity calculations were performed using the Megalign program of the LASERGENE bioinformatics computing suite (DNASTAR Inc., Madison, WI). Multiple alignment of the sequences was performed using the Clustal method of alignment (Higgins and Sharp (1989) *CABIOS. 5*:151-153) with the default parameters (GAP PENALTY=10, GAP LENGTH PENALTY=10). Default parameters for pairwise alignments using the Clustal method were KTUPLE 1, GAP PENALTY=3, WINDOW=5 and DIAGONALS SAVED=5.

A "substantial portion" of an amino acid or nucleotide sequence comprises an amino acid or a nucleotide sequence that is sufficient to afford putative identification of the protein or gene that the amino acid or nucleotide sequence comprises. Amino acid and nucleotide sequences can be evaluated either manually by one skilled in the art, or by using computer-based sequence comparison and identification tools that employ algorithms such as BLAST (Basic Local Alignment Search Tool; Altschul et al. (1993) J. Mol. Biol. 215:403-410; see also www.ncbi.nlm.nih.gov/BLAST/). In general, a sequence of ten or more contiguous amino acids or thirty or more contiguous nucleotides is necessary in order to putatively identify a polypeptide or nucleic acid sequence as homologous to a known protein or gene. Moreover, with respect to nucleotide sequences, gene-specific oligonucleotide probes comprising: 30 or more contiguous nucleotides may be used in sequence-dependent methods of gene identification (e.g., Southern hybridization) and isolation (e.g., *in situ* hybridization of bacterial colonies or bacteriophage plaques). In addition, short oligonucleotides of 12 or more nucleotides may be used as amplification primers in PCR in order to obtain a particular nucleic acid fragment comprising the primers. Accordingly, a "substantial portion" of a nucleotide sequence comprises a nucleotide sequence that will afford specific identification and/or isolation of a nucleic acid fragment comprising the sequence. The instant specification teaches amino acid and nucleotide sequences encoding polypeptides that comprise one or more particular plant proteins. The skilled artisan, having the benefit of the sequences as reported herein, may now use all or a substantial portion of the disclosed sequences for purposes known to those skilled in this art. Accordingly, the instant invention comprises the complete sequences as reported in the accompanying Sequence Listing, as well as substantial portions of those sequences as defined above.

"Codon degeneracy" refers to divergence in the genetic code permitting variation of the nucleotide sequence without effecting the amino acid sequence of an encoded polypeptide. Accordingly, the instant invention relates to any nucleic acid fragment comprising a nucleotide sequence that encodes all or a substantial portion of the amino acid sequences set forth herein. The skilled artisan is well aware of the "codon-bias" exhibited by a specific host cell in usage of nucleotide codons to specify a given amino acid. Therefore, when synthesizing a nucleic acid fragment for improved expression in a host cell, it is desirable to design the nucleic acid fragment such that its frequency of codon usage approaches the frequency of preferred codon usage of the host cell.

"Synthetic nucleic acid fragments" can be assembled from oligonucleotide building blocks that are chemically synthesized using procedures known to those skilled in the art. These building blocks are ligated and annealed to form larger nucleic acid fragments which may then be enzymatically assembled to construct the entire desired nucleic acid fragment. "Chemically synthesized", as related to a nucleic acid fragment, means that the component nucleotides were assembled *in vitro.* Manual chemical synthesis of nucleic acid fragments may be accomplished using well established procedures, or automated chemical synthesis can be performed using one of a number of commercially available machines. Accordingly, the nucleic acid fragments can be tailored for optimal gene expression based on optimization of the nucleotide sequence to reflect the codon bias of the host cell. The skilled artisan appreciates the likelihood of successful gene expression if codon usage is biased towards those codons favored by the host. Determination of preferred codons can be based on a survey of genes derived from the host cell where sequence information is available.

"Gene" refers to a nucleic acid fragment that expresses a specific protein, including regulatory sequences preceding (5' non-coding sequences) and following (3' non-coding sequences) the coding sequence. "Native gene" refers to a gene as found in nature with its own regulatory sequences. "Chimeric gene" refers any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. "Endogenous gene" refers to a native gene in its natural location in the genome of an organism. A "foreign-gene" refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure.

"Coding sequence" refers to a nucleotide sequence that codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, translation leader sequences, introns, and polyadenylation recognition sequences.

"Promoter" refers to a nucleotide sequence capable of controlling the expression of a coding sequence or functional RNA. In general, a coding sequence is located 3' to a promoter sequence. The promoter sequence consists of proximal and more distal upstream elements, the latter elements often referred to as enhancers. Accordingly, an "enhancer" is a nucleotide sequence which can stimulate promoter activity and may be an innate element of the promoter or a heterologous element inserted to enhance the level or tissue-specificity of a promoter. Promoters may be derived in their entirety from a native gene, or may be composed of different elements derived from different promoters found in nature, or may even comprise synthetic nucleotide segments. It is understood by those skilled in the art that different promoters may direct the expression of a gene in different tissues or cell types, or at different stages of development, or in response to different environmental conditions. Promoters which cause a nucleic acid fragment to be expressed in most cell types at most times are commonly referred to as "constitutive promoters". New promoters of various types useful in plant cells are constantly being discovered; numerous examples may be found in the compilation by Okamuro and Goldberg (1989) Biochemistry of Plants 15:1-82. It is further recognized that since in most cases the exact boundaries of regulatory sequences have not been completely defined, nucleic acid fragments of different lengths may have identical promoter activity.

"Translation leader sequence" refers to a nucleotides sequence located between the promoter sequence of a gene and the coding sequence. The translation leader sequence is present in the fully processed mRNA upstream of the translation start sequence. The translation leader sequence may affect processing of the primary transcript to mRNA, mRNA stability or translation efficiency. Examples of translation leader sequences have been described (Turner and Foster (1995) Mol. Biotechnol. 3:225-236).

"3' non-coding sequences" refer to nucleotide sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al. (1989) Plant Cell 1:671-680.

"RNA transcript" refers to the product resulting from RNA polymerase-catalyzed transcription of a DNA sequence. When the RNA transcript is a perfect complementary copy of the DNA sequence, it is referred to as the primary transcript or it may be a RNA sequence derived from post-transcriptional processing of the primary transcript and is referred to as the mature RNA. "Messenger RNA (mRNA)" refers to the RNA that is without introns and that can be translated into polypeptides by the cell. "cDNA" refers to DNA that is complementary to and derived from an mRNA template. The cDNA can be single-stranded or converted to double stranded form using, for example, the Klenow fragment of DNA polymerase I. "Sense-RNA" refers to an RNA transcript that includes the mRNA and so can be translated into a polypeptide by the cell. "Antisense RNA" refers to an RNA transcript that is complementary to all or part of a target primary transcript or mRNA and that blocks the expression of a target gene (see U.S. Patent No. 5,107,065, ). The complementarity of an antisense RNA may be with any part of the specific nucleotide sequence, i.e., at the 5' non-coding sequence, 3' non-coding sequence, introns, or the coding sequence. "Functional RNA" refers to sense RNA, antisense RNA, ribozyme RNA, or other RNA that may not be translated but yet has an effect on cellular processes.

The term "operably linked" refers to the association of two or more nucleic acid fragments on a single polynucleotide so that the function of one is affected by the other. For example, a promoter is operably linked with a coding sequence when it is capable of affecting the expression of that coding sequence (i.e., that the coding sequence is under the transcriptional control of the promoter). Coding sequences can be operably linked to regulatory sequences in sense or antisense orientation.

The term "expression", as used herein, refers to the transcription and stable accumulation of sense (mRNA) or antisense RNA derived from the nucleic acid fragment of the invention. Expression may also refer to translation of mRNA into a polypeptide. "Antisense inhibition" refers to the production of antisense RNA transcripts capable of suppressing the expression of the target protein. "Overexpression" refers to the production of a gene product in transgenic organisms that exceeds levels of production in normal or non-transformed organisms. "Co-suppression" refers to the production of sense RNA transcripts capable of suppressing the expression of identical or substantially similar foreign or endogenous genes (U.S. Patent No. 5,231,020).

A "protein" or "polypeptide" is a chain of amino acids .arranged in a specific order determined by the coding sequence in a polynucleotide encoding the polypeptide. Each protein or polypeptide has a unique function.

"Altered levels" or "altered expression" refers to the production of gene product(s) in transgenic organisms in amounts or proportions that differ from that of normal or non-transformed organisms.

"Null mutant" refers here to a host cell which either lacks the expression of a certain polypeptide or expresses a polypeptide which is inactive or does not have any detectable expected enzymatic function.

"Mature protein" or the term "mature" when used in describing a protein refers to a post-translationally processed polypeptide; i.e., one from which any pre- or propeptides present in the primary translation product have been removed. "Precursor protein" or the term "precursor" when used in describing a protein refers to the primary product of translation of mRNA; i.e., with pre- and propeptides still present. Pre- and propeptides may be but are not limited to intracellular localization signals.

A "chloroplast transit peptide" is an amino acid sequence which is translated in conjunction with a protein and directs the protein to the chloroplast or other plastid types present in the cell in which the protein is made. "Chloroplast transit sequence" refers to a nucleotide sequence that encodes a chloroplast transit peptide. A "signal peptide" is an amino acid sequence which is translated in conjunction with a protein and directs the protein to the secretory system (Chrispeels (1991) Ann. Rev. Plant Phys. Plant Mol. Biol. 42:21-53). If the protein is to be directed to a vacuole, a vacuolar targeting signal *(supra)* can further be added, or if to the endoplasmic reticulum, an endoplasmic reticulum retention signal *(supra)* may be added. If the protein is to be directed to the nucleus, any signal peptide present should be removed and instead a nuclear localization signal included (Raikhel (1992) Plant Phys. 100:1627-1632).

"Transformation" refers to the transfer of a nucleic acid fragment into the genome of a host organism, resulting in genetically stable inheritance. Host organisms containing the transformed nucleic acid fragments are referred to as "transgenic" organisms. Examples of methods of plant transformation include *Agrobacterium*-mediated transformation (De Blaere et al. (1987) Meth. Enzymol. 143:277) and particle-accelerated or "gene gun" transformation technology (Klein et al. (1987) Nature (London) 327:70-73; U.S. Patent No. 4,945,050, ). Thus, isolated polynucleotides of the present invention can be incorporated into recombinant constructs, typically DNA constructs, capable of introduction into and replication in a host cell. Such a construct can be a vector that includes a replication system and sequences that are capable of transcription and translation of a polypeptide-encoding sequence in a given host cell. A number of vectors suitable for stable transfection of plant cells or for the establishment of transgenic plants have been described in, e.g., Pouwels et al., Cloning Vectors: A Laboratory Manual, 1985, supp. 1987; Weissbach and Weissbach, Methods for Plant Molecular Biology, Academic Press, 1989; and Flevin et al., Plant Molecular Biology Manual, Kluwer Academic Publishers, 1990. Typically, plant expression vectors include, for example, one or more cloned plant genes under the transcriptional control of 5' and 3' regulatory sequences and a dominant selectable marker. Such plant expression vectors also can contain a promoter regulatory region (e.g., a regulatory region controlling inducible or constitutive, environmentally- or developmentally-regulated, or cell- or tissue-specific expression), a transcription initiation start site, a ribosome binding site, an RNA processing signal, a transcription termination site, and/or a polyadenylation signal.

Standard recombinant DNA and molecular cloning techniques used herein are well known in the art and are described more fully in Sambrook et al. Molecular Cloning: A Laboratory Manual; Cold Spring Harbor Laboratory Press: Cold Spring Harbor, 1989 (hereinafter "Maniatis").

"PCR" or "polymerase chain reaction" is well known by those skilled in the art as a technique used for the amplification of specific DNA segments (U.S. Patent Nos. 4,683,195 and 4,800,159).

Nucleic acid fragments encoding at least a portion of several reproduction proteins have been isolated and identified by comparison of random plant cDNA sequences to public databases containing nucleotide and protein sequences using the BLAST algorithms well known to those skilled in the art. The nucleic acid fragments of the instant invention may be used to isolate cDNAs and genes encoding homologous proteins from the same or other plant species. Isolation of homologous genes using sequence-dependent protocols is well known in the art. Examples of sequence-dependent protocols include, but are not limited to, methods of nucleic acid hybridization, and methods of DNA and RNA amplification as exemplified by various uses of nucleic acid amplification technologies (e.g., polymerase chain reaction, ligase chain reaction).

For example, genes encoding other fertilization-independent endosperm proteins, either as cDNAs or genomic DNAs, could be isolated directly by using all or a portion of the instant nucleic acid fragments as DNA hybridization probes to screen libraries from any desired plant employing methodology well known to those skilled in the art. Specific oligonucleotide probes based upon the instant nucleic acid sequences can be designed and synthesized by methods known in the art (Maniatis). Moreover, an entire sequence can be used directly to synthesize DNA probes by methods known to the skilled artisan such as random primer DNA labeling, nick translation, end-labeling techniques, or RNA probes using available *in vitro* transcription systems. In addition, specific primers can be designed and used to amplify a part or all of the instant sequences. The resulting amplification products can be labeled directly during amplification reactions or labeled after amplification reactions, and used as probes to isolate full length cDNA or genomic fragments under conditions of appropriate stringency.

In addition, two short segments of the instant nucleic acid fragments may be used in polymerase chain reaction protocols to amplify longer nucleic acid fragments encoding homologous genes from DNA or RNA. The polymerase chain reaction may also be performed on a library of cloned nucleic acid fragments wherein the sequence of one primer is derived from the instant nucleic acid fragments, and the sequence of the other primer takes advantage of the presence of the polyadenylic acid tracts to the 3' end of the mRNA precursor encoding plant genes. Alternatively, the second primer sequence may be based upon sequences derived from the cloning vector. For example, the skilled artisan can follow the RACE protocol (Frohman et al. (1988) Proc. Natl. Acad Sci. USA 85:8998-9002) to generate cDNAs by using PCR to amplify copies of the region between a single point in the transcript and the 3' or 5' end. Primers oriented in the 3' and 5' directions can be designed from the instant sequences. Using commercially available 3' RACE or 5' RACE systems (BRL), specific 3' or 5' cDNA fragments can be isolated (Ohara et al. (1989) Proc. Natl. Acad Sci. USA 86:5673-5677; Loh et al. (1989) Science 243:217-220). Products generated by the 3' and 5' RACE procedures can be combined to generate full-length cDNAs (Frohman and Martin (1989) Techniques 1:165). Consequently, a polynucleotide comprising a nucleotide sequence of at least one of 60 (preferably one of at least 40, most preferably one of at least 30) contiguous nucleotides derived from a nucleotide sequence selected from the group consisting of SEQ ID Nos: 1, 29 and 39, and the complement of such nucleotide sequences may be used in such methods to obtain a nucleic acid fragment encoding a substantial portion of an amino acid sequence of a polypeptide.

The present invention relates to a method of obtaining a nucleic acid fragment encoding a substantial portion of a fertilization-independent endosperm polypeptide, preferably a substantial portion of a plant fertilization-independent endosperm polypeptide, comprising the steps of : synthesizing an oligonucleotide primer comprising a nucleotide sequence of at least one of 60 (preferably at least one of 40, most preferably at least one of 30) contiguous nucleotides derived from a nucleotide sequence selected from the group consisting of SEQ ID Nos: 1, 29 and 39, and the complement of such nucleotide sequences; and amplifying a nucleic acid fragment (preferably a cDNA inserted in a cloning vector) using the oligonucleotide primer. The amplified nucleic acid fragment preferably will encode a portion of a fertilization-independent endosperm polypeptide.

Availability of the instant nucleotide and deduced amino acid sequences facilitates immunological screening of cDNA expression libraries. Synthetic peptides representing portions of the instant amino acid sequences may be synthesized. These peptides can be used to immunize animals to produce polyclonal or monoclonal antibodies with specificity for peptides or proteins comprising the amino acid sequences. These antibodies can be then be used to screen cDNA expression libraries to isolate full-length cDNA clones of interest (Lerner (1984) Adv. lmmunol. 36:1-34; Maniatis).

In another embodiment, this invention concerns viruses and host cells comprising either the chimeric genes of the invention as described herein or an isolated polynucleotide of the invention as described herein. Examples of host cells which can be used to practice the invention include, but are not limited to, yeast, bacteria, and plants.

As was noted above, the nucleic acid fragments of the instant invention may be used to create transgenic plants in which the disclosed polypeptides are present at higher or lower levels than normal or in cell types or developmental stages in which they are not normally found. This would have the effect of altering endosperm formation in those cells.

Overexpression of the proteins of the instant invention may be accomplished by first constructing a chimeric gene in which the coding region is operably linked to a promoter capable of directing expression of a gene in the desired tissues at the desired stage of development. The chimeric gene may comprise promoter sequences and translation leader sequences derived from the same genes. 3' Non-coding sequences encoding transcription termination signals may also be provided. The instant chimeric gene may also comprise one or more introns in order to facilitate gene expression.

Plasmid vectors comprising the instant isolated polynucleotide (or chimeric gene) may be constructed. The choice of plasmid vector is dependent upon the method that will be used to transform host plants. The skilled artisan is well aware of the genetic elements that must be present on the plasmid vector in order to successfully transform, select and propagate host cells containing the chimeric gene. The skilled artisan will also recognize that different independent transformation events will result in different levels and patterns of expression (Jones et al. (1985) EMBO J 4:2411-2418; De Almeida et al. (1989) Mol. Gen. Genetics 218:78-86), and thus that multiple events must be screened in order to obtain lines displaying the desired expression level and pattern. Such screening may be accomplished by Southern analysis of DNA, Northern analysis of mRNA expression, Western analysis of protein expression, or phenotypic analysis.

For some applications it may be useful to direct the instant polypeptides to different cellular compartments, or to facilitate its secretion from the cell. It is thus envisioned that the chimeric gene described above may be further supplemented by directing the coding sequence to encode the instant polypeptides with appropriate intracellular targeting sequences such as transit sequences (Keegstra (1989) Cell 56:247-253), signal sequences or sequences encoding endoplasmic reticulum localization (Chrispeels (1991) Ann. Rev. Plant Phys. Plant Mol. Biol. 42:21-53), or nuclear localization signals (Raikhel (1992) Plant Phys.100:1627-1632) with or without removing targeting sequences that are already present. While the references cited give examples of each of these, the list is not exhaustive and more targeting signals of use may be discovered in the future.

It may also be desirable to reduce or eliminate expression of genes encoding the instant polypeptides in plants for some applications. In order to accomplish this, a chimeric gene designed for co-suppression of the instant polypeptide can be constructed by linking a gene or gene fragment encoding that polypeptide to plant promoter sequences. Alternatively, a chimeric gene designed to express antisense RNA for all or part of the instant nucleic acid fragment can be constructed by linking the gene or gene fragment in reverse orientation to plant promoter sequences. Either the co-suppression or antisense chimeric genes could be introduced into plants via transformation wherein expression of the corresponding endogenous genes are reduced or eliminated.

Molecular genetic solutions to the generation of plants with altered gene expression have a decided advantage over more traditional plant breeding approaches. Changes in plant phenotypes can be produced by specifically inhibiting expression of one or more genes by antisense inhibition or co-suppression (U.S. Patent Nos. 5,190,931, 5,107,065 and 5,283,323). An antisense or co-suppression construct would act as a dominant negative regulator of gene activity. While conventional mutations can yield negative regulation of gene activity these effects are most likely recessive. The dominant negative regulation available with a transgenic approach may be advantageous from a breeding perspective. In addition, the ability to restrict the expression of a specific phenotype to the reproductive tissues of the plant by the use of tissue specific promoters may confer agronomic advantages relative to conventional mutations which may have an effect in all tissues in which a mutant gene is ordinarily expressed.

The person skilled in the art will know that special considerations are associated with the use of antisense or cosuppression technologies in order to reduce expression of particular genes. For example, the proper level of expression of sense or antisense genes may require the use of different chimeric genes utilizing different regulatory elements known to the skilled artisan. Once transgenic plants are obtained by one of the methods described above, it will be necessary to screen individual transgenics for those that most effectively display the desired phenotype. Accordingly, the skilled artisan will develop methods for screening large numbers of transformants. The nature of these screens will generally be chosen on practical grounds. For example, one can screen by looking for changes in gene expression by using antibodies specific for the protein encoded by the gene being suppressed, or one could establish assays that specifically measure enzyme activity. A preferred method will be one which allows large numbers of samples to be processed rapidly, since it will be expected that a large number of transformants will be negative for the desired phenotype.

The instant polypeptides (or portions thereof) may be produced in heterologous host cells, particularly in the cells of microbial hosts, and can be used to prepare antibodies to these proteins by methods well known to those skilled in the art. The antibodies are useful for detecting the polypeptides of the instant invention *in situ* in cells or *in vitro* in cell extracts. Preferred heterologous host cells for production of the instant polypeptides are microbial hosts. Microbial expression systems and expression vectors containing regulatory sequences that direct high level expression of foreign proteins are well known to those skilled in the art. Any of these could be used to construct a chimeric gene for production of the instant polypeptides. This chimeric gene could then be introduced into appropriate microorganisms via transformation to provide high level expression of the encoded reproduction proteins. An example of a vector for high level expression of the instant polypeptides in a bacterial host is provided (Example 6).

All or a substantial portion of the polynucleotides of the instant invention may also be used as probes for genetically and physically mapping the genes that they are a part of, and used as markers for traits linked to those genes. Such information may be useful in plant breeding in order to develop lines with desired phenotypes. For example, the instant nucleic acid fragments may be used as restriction fragment length polymorphism (RFLP) markers. Southern blots (Maniatis) of restriction-digested plant genomic DNA may be probed with the nucleic acid fragments of the instant invention. The resulting banding patterns may then be subjected to genetic analyses using computer programs such as MapMaker (Lander et al. (1987) Genomics 1:174-181) in order to construct a genetic map. In addition, the nucleic acid fragments of the instant invention may be used to probe Southern blots containing restriction endonuclease-treated genomic DNAs of a set of individuals representing parent and progeny of a defined genetic cross. Segregation of the DNA polymorphisms is noted and used to calculate the position of the instant nucleic acid sequence in the genetic map previously obtained using this population (Botstein et al. (1980) Am. J. Hum. Genet. 32:314-331).

The production and use of plant gene-derived probes for use in genetic mapping is described in Bematzky and Tanksley (1986) Plant Mol. Biol. Reporter 4:37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

Nucleic acid probes derived from the instant nucleic acid sequences may also be used for physical mapping (i.e., placement of sequences on physical maps; *see* Hoheisel et al. In: Nonmammalian Genomic Analysis: A Practical Guide, Academic press 1996, pp. 319-346, and references cited therein).

In another embodiment, nucleic acid probes derived from the instant nucleic acid sequences may be used in direct fluorescence *in situ* hybridization (FISH) mapping (Trask (1991) Trends Genet. 7:149-154). Although current methods of FISH mapping favor use of large clones (several to several hundred KB; see Laan et al. (1995) Genome Res. 5:13-20), improvements in sensitivity may allow performance of FISH mapping using shorter probes.

A variety of nucleic acid amplification-based methods of genetic and physical mapping may be carried but using the instant nucleic acid sequences. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807)*.* For these methods, the sequence of a nucleic acid fragment is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

Loss of function mutant phenotypes may be identified for the instant cDNA clones either by targeted gene disruption protocols or by identifying specific mutants for these genes contained in a maize population carrying mutations in all possible genes (Ballinger and Benzer (1989) Proc. Natl. Acad Sci USA 86:9402-9406; Koes et al. (1995) Proc. Natl. Acad. Sci USA 92:8149-8153; Bensen et al. (1995) Plant Cell 7:75-84). The latter approach may be accomplished in two ways. First, short segments of the instant nucleic acid fragments may be used in polymerase chain reaction protocols in conjunction with a mutation tag sequence primer on DNAs prepared from a population of plants in which Mutator transposons or some other mutation-causing DNA element has been introduced (see Bensen, *supra*). The amplification of a specific DNA fragment with these primers indicates the insertion of the mutation tag element in or near the plant gene encoding the instant polypeptides. Alternatively, the instant nucleic acid fragment may be used as a hybridization probe against PCR amplification products generated from the mutation population using the mutation tag sequence primer in conjunction with an arbitrary genomic site primer, such as that for a restriction enzyme site-anchored synthetic adaptor. With either method, a plant containing a mutation in the endogenous gene encoding the instant polypeptides can be identified and obtained. This mutant plant can then be used to determine or confirm the natural function of the instant polypeptides disclosed herein.

### EXAMPLES

The present invention is further defined in the following Examples, in which parts and percentages are by weight and degrees are Celsius, unless otherwise stated. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various usages and conditions. Thus, various modifications of the invention in addition to those shown and described herein will be apparent to those skilled in the art from the foregoing description. Such modifications are also intended to fall within the scope of the appended claims.

The disclosure of each reference set forth herein is incorporated herein by reference in its entirety.

### EXAMPLE I

### Composition of cDNA Libraries: Isolation and Sequencing of cDNA Clones

cDNA libraries representing mRNAs from various catalpa, corn eucalyptus, rice, soybean, sunflower and wheat tissues were prepared. The characteristics of the libraries are described below.

**TABLE 2**

| cDNA Libraries from Catalpa, Corn, Eucalyptus, Rice, Soybean, Sunflower and Wheat | | |
|---|---|---|
| Library | Tissue | Clone |
| ccase-b | Corn callus, somatic embryo formed, | ccase-b.pk0026.g4 |
| cent 1 | Corn endosperm 10 to 11 days after pollination | cen1.mn0001.g10 |
| cen3n | Corn endosperm 20 days after pollination* | cen3n.pk0076.b8 |
| cpb1c | Corn pooled BMS treated with chemicals related to Ca⁺⁺ | cpb1c.pk001.d10 |
| | channel** | |
| eec1c | *Eucalyptus tereticornis* capsules (older flowers, lost | eeclc.pk003.e23 |
| | stamens, possibly fertiliseed) from adult tree | |
| hlp1c | *Helianthus* sp. leaf infected with phomopsis | hlplc.pk003.e8 |
| ncs | *Catalpa speciosa* developing seed | ncs.pk0019.h3 |
| p0003 | Corn premeiotic ear shoot, 0.2-4 cm | p0003.cgped29rb |
| | | p0003.cgpfn34f |
| | | p0003.cgpfn34rb |
| p0037 | Corn V5 stage*** roots infested with corn root worm | p0037.crwao47r |
| p0041 | Corn root tips smaller than 5 mm in length four days after | p0041.crtaw93r |
| | imbibition | |
| p0101 | Corn embryo sacs 4 days after pollination* | p0101.cgamg48r |
| p0104 | Corn roots V5, corn root worm infested* | p0104.cabbn62r |
| p0107 | Corn whole kernels 7 days after pollination* | p0107.cbcai79r |
| p0119 | Corn V 12 stage*** ear shoot with husk, night harvested* | p0119.cmtoh49r |
| p0120 | Pooled endosperm: 18, 21, 24, 27 and 29 days after | p0120.cdebd48r |
| | pollenation* | |
| rcal1c | Rice nipponbare callus | rcal1c.pk0001.d2 |
| ses2w | Soybean embryogenic suspension 2 weeks after | ses2w.pk0015.b10 |
| | subculture | |
| wkm1c | Wheat kernel malted 55 hours at 22 degrees celsius | wkm1c.pk0003.f4 |

| | | |
|---|---|---|
| *These libraries were normalized essentially as described in U.S. Patent No. 5,482,845, incorporated herein by reference. ** Chemicals used included caffeine, BHQ, cyclopiazonic acid, nifedipine, verapamil, fluphenizine-N-2-chloroethane, calmidazoilum chloride *** Corn developmental stages are explained in the publication "How a corn plant develops" from the Iowa State University Coop. Ext. Service Special Report No. 48 reprinted June 1993. | | |

cDNA libraries may be prepared by any one of many methods available. For example, the cDNAs may be introduced into plasmid vectors by first preparing the cDNA libraries in Uni-ZAP^{™} XR vectors according to the manufacturer's protocol (Stratagene Cloning Systems, La Jolla, CA). The Uni-ZAP^{™} XR libraries are converted into plasmid libraries according to the protocol provided by Stratagene. Upon conversion, cDNA inserts will be contained in the plasmid vector pBluescript. In addition, the cDNAs may be introduced directly into precut Bluescript II SK(+) vectors (Stratagene) using T4 DNA ligase (New England Biolabs), followed by transfection into DH10B cells according to the manufacturer's protocol (GIBCO BRL Products). Once the cDNA inserts are in plasmid vectors, plasmid DNAs are prepared from randomly picked bacterial colonies containing recombinant pBluescript plasmids, or the insert cDNA sequences are amplified via polymerase chain reaction using primers specific for vector sequences flanking the inserted cDNA sequences. Amplified insert DNAs or plasmid DNAs are sequenced in dye-primer sequencing reactions to generate partial cDNA sequences (expressed sequence tags or "ESTs"; see Adams et al., (1991) Science 252:1651-1656). The resulting ESTs are analyzed using a Perkin Elmer Model 377 fluorescent sequencer.

### EXAMPLE 2

### Identification of cDNA Clones

cDNA clones encoding reproduction proteins were identified by conducting BLAST (Basic Local Alignment Search Tool; Altschul et al. (1993) J. Mol. Biol. 215:403-410; see also www.ncbi.nlm.nih.gov/BLAST/ searches for similarity to sequences contained in the BLAST "nr" database (comprising all non-redundant GenBank CDS translations, sequences derived from the 3-dimensional structure Brookhaven Protein Data Bank, the last major release of the SWISS-PROT protein sequence database, EMBL, and DDBJ databases). The cDNA sequences obtained in Example 1 were analyzed for similarity to all publicly available DNA sequences contained in the "nr" database using the BLASTN algorithm provided by the National Center for Biotechnology Information (NCBI). The DNA sequences were translated in all reading frames and compared for similarity to all publicly available protein sequences contained in the "nr" database using the BLASTX algorithm (Gish and States (1993) Nat. Genet. 3:266-272) provided by the NCBI. For convenience, the P-value (probability) of observing a match of a cDNA sequence to a sequence contained in the searched databases merely by chance as calculated by BLAST are reported herein as "pLog" values, which represent the negative of the logarithm of the reported P-value. Accordingly, the greater the pLog value, the greater the likelihood that the cDNA sequence and, the BLAST "hit" represent homologous proteins.

### EXAMPLE 3

### Characterization of cDNA Clones Encoding Fertilization-Independent Endosperm Protein

The BLASTX search using the EST sequences from clones listed in Table 3 revealed similarity of the polypeptides encoded by the cDNAs to fertilization-independent endosperm protein from *Arabidopsis thaliana* NCBI Identifier No. gi 4567095). Shown in Table 3 are the BLAST results for individual ESTs ("EST"), the sequences of the entire cDNA inserts comprising the indicated cDNA clones ("FIS"), the sequences of contigs assembled from two or more ESTs ("Contig"), sequences of contigs assembled from an FIS and one or more ESTs ("Contig*"), or sequences encoding an entire protein derived from an FIS, a contig, or an FIS and PCR ("CGS"):

**TABLE 3**

| BLAST Results for Sequences Encoding Polypeptides Homologous to *Arabidopsis thaliana* Fertilization-Independent Endosperm Protein | | |
|---|---|---|
| | | BLAST pLog Score to |
| Clone | Status | gi 4567095 |
| ccase-b.pk0026.g4 | EST | 23.40 |
| cen1.mn0001.g10 | EST | 33.60 |
| cpb1c.pk001.d10 | EST | 60.15 |
| eec1c.pk003.e23 | EST | 25.10 |
| hlp1c.pk003.e8 | EST | 54.00 |
| ncs.pk0019.h3 | EST | 50.40 |
| p0003.cgpfn34rb | EST | 50.52 |
| p0003.cgped29rb | EST | 88.70 |
| p0037.crwao47r | EST | 82.00 |
| p0041.crtaw93r | EST | 18.00 |
| p0104.cabbn62r | EST | 20.70 |
| p0107.cbcai79r | CGS | 133.00 |
| p0120.cdebd48r | EST | 41.40 |
| rcal1c.pk0001.d2 | EST | 80.00 |
| ses2w.pk0015.b10 | EST | 89.71 |
| wkm1c.pk0003.f4 | EST | 33.00 |

The sequence of the entire cDNA insert in the clones listed in Table 3 was determined. Further sequencing and searching of the DuPont proprietary database allowed the identification of other corn, rice, soybean and/or wheat clones encoding fertilization-independent endosperm proteins. The BLASTX search using the EST sequences from clones listed in Table 4 revealed similarity of the polypeptides encoded by the cDNAs to fertilization-independent endosperm protein from *Arabidopsis thaliana* NCBI Identifier No. gi 4567095). Shown in Table 4 are the BLAST results for individual ESTs ("EST"), the sequences of the entire cDNA inserts comprising the indicated cDNA clones ("FIS"), sequences of contigs assembled from two or more ESTs ("Contig"), sequences of contigs assembled from an FIS and one or more ESTs ("Contig*"), or sequences encoding the entire protein derived from an FIS, a contig, or an FIS and PCR ("CGS"):

**TABLE 4**

| BLAST Results for Sequences Encoding Polypeptides Homologous to Fertilization-Independent Endosperm Protein | | |
|---|---|---|
| | | BLAST pLog Score |
| Clone | Status | to gi 4567095 |
| ccase-b.pk0026.g4 | CGS | 155.00 |
| cen1.mn0001.g10 | CGS | 134.00 |
| cen3n.pk0076.b8 | CGS | 134.00 |
| cpb1c.pk001.d10 | FIS | 60.40 |
| eeclc.pk003.e23 | CGS | 177.00 |
| hlp1c.pk003.e8 | FIS | 49.70 |
| ncs.pk0019.h3 | CGS | 176.00 |
| p0003.cgpfn34f | EST | 54.00 |
| p0003.cgped29rb | CGS | 148.00 |
| p0037.crwao47r | FIS | 121.00 |
| p0041.crtaw93r | FIS | 15.10 |
| p0101.cgamg48r | CGS | 112.00 |
| p0104.cabbn62r | CGS | 155.00 |
| p0107.cbcai79r | CGS | 134.00 |
| p0119.cmtoh49r | CGS | 155.00 |
| p0120.cdebd48r | FIS | 40.70 |
| rcal1c.pk0001.d2 | CGS | 154.00 |
| ses2w.pk0015.b10 | CGS | 167.00 |
| wkm1c.pk0003.f4 | CGS | 155.00 |

The data in Table 5 represents a calculation of the percent identity of the amino acid sequences set forth in SEQ ID NOs:2, 4, 6, 8, 10, 12, 14, 16, 18, 20, 22, 24, 26, 28; 30, 32, 34, 36, 38, 40, 42, 44, 46, 48, 50, 52, 54, 56, 58, 60, 62, 64, 66, 68 and 70 and the *Arabidopsis thaliana* sequence.

**TABLE 5**

| Percent Identity of Amino Acid Sequences Deduced From the Nucleotide Sequences of cDNA Clones Encoding Polypeptides Homologous to *Arabidopsis thaliana* Fertilization-Independent Endosperm Protein | |
|---|---|
| | Percent Identity to |
| SEQ ID NO. | gi 4567095 |
| 2 | 66% |
| 4 | 57% |
| 6 | 57% |
| 8 | 66% |
| 10 | 75% |
| 12 | 70% |
| 14 | 74% |
| 16 | 68% |
| 18 | 64% |
| 20 | 72% |
| 22 | 55% |
| 24 | 62% |
| 26 | 57% |
| 28 | 57% |
| 30 | 68% |
| 32 | 49% |
| 34 | 67% |
| 36 | 71% |
| 38 | 67% |
| 40 | 55% |
| 42 | 53% |
| 44 | 70% |
| 46 | 66% |
| 48 | 70% |
| 50 | 65% |
| 52 | 68% |
| 54 | 80% |
| 56 | 79% |
| 58 | 66% |
| 60 | 66% |
| 62 | 58% |
| 64 | 66% |
| 66 | 80% |
| 78 | 79% |
| 70 | 49% |

Sequence alignments and percent identity calculations were performed using the Megalign program of the LASERGENE bioinformatics computing suite (DNASTAR Inc., Madison, WI). Multiple alignment of the sequences was performed using the Clustal method of alignment (Higgins and Sharp (1989) CABIOS. 5:151-153) with the default parameters (GAP PENALTY=10, GAP LENGTH PENALTY=10). Default parameters for pairwise alignments using the Clustal method were KTUPLE 1, GAP PENALTY=3, WINDOW=5 and DIAGONALS SAVED=5. Sequence alignments and BLAST scores and probabilities indicate that the nucleic acid fragments comprising the instant cDNA clones encode a substantial portion of a fertilization-independent endosperm protein. These sequences represent the first *Catalpa,* eucalyptus, corn, rice, soybean, sunflower and wheat sequences encoding fertilization-independent endosperm proteins known to Applicant.

### EXAMPLE 4

### Expression of Chimeric Genes in Monocot Cells

A chimeric gene comprising a cDNA encoding the instant polypeptides in sense orientation with respect to the maize 27 kD zein promoter that is located 5' to the cDNA fragment, and the 10 kD zein 3' end that is located 3' to the cDNA fragment, can be constructed. The cDNA fragment of this gene may be generated by polymerase chain reaction (PCR) of the cDNA clone using appropriate oligonucleotide primers. Cloning sites (NcoI or SmaI) can be incorporated into the oligonucleotides to provide proper orientation of the DNA fragment when inserted into the digested vector pML103 as described below. Amplification is then performed in a standard PCR. The amplified DNA is then digested with restriction enzymes NcoI and SmaI and fractionated on an agarose gel. The appropriate band can be isolated from the gel and combined with a 4.9 kb NcoI-SmaI fragment of the plasmid pML103. Plasmid pML103 has been deposited under the terms of the Budapest Treaty at ATCC (American Type Culture Collection, 10801 University Blvd., Manassas, VA 20110-2209), and bears accession number ATCC 97366. The DNA segment from pML103 contains a 1.05 kb SalI-NcoI promoter fragment of the maize 27 kD zein gene and a 0.96 kb SmaI-SalI fragment from the 3' end of the maize 10 kD zein gene in the vector pGem9Zf(+) (Promega). Vector and insert, DNA can be ligated at 15°C overnight, essentially as described (Maniatis). The ligated DNA may then be used to transform *E*. *coli* XL1-Blue (Epicurian Coli XL-1 Blue^{™}; Stratagene). Bacterial transformants can be screened by restriction enzyme digestion of plasmid DNA and limited nucleotide sequence analysis using the dideoxy chain termination method (Sequenase^{™} DNA Sequencing Kit; U.S. Biochemical). The resulting plasmid construct would comprise a chimeric gene encoding, in the 5' to 3' direction, the maize 27 kD zein promoter, a cDNA fragment encoding the instant polypeptides; and the 10 kD zein 3' region.

The chimeric gene described above can then be introduced into corn cells by the following procedure. Immature corn embryos can be dissected from developing caryopses derived from crosses of the inbred corn lines H99 and LH132. The embryos are isolated 10 to 11 days after pollination when they are 1.0 to 1.5 mm long. The embryos are then placed with the axis-side facing down and in contact with agarose-solidified N6 medium (Chu et al. (1975) Sci. Sin. Peking 18:659-668). The embryos are kept in the dark at 27°C. Friable embryogenic callus consisting of undifferentiated masses of cells with somatic proembryoids and embryoids borne on suspensor structures proliferates from the scutellum of these immature embryos. The embryogenic callus isolated from the primary explant can be cultured on N6 medium and sub-cultured on this medium every 2 to 3 weeks.

The plasmid, p35S/Ac (obtained from Dr. Peter Eckes, Hoechst Ag, Frankfurt, Germany) may be used in transformation experiments in order to provide for a selectable marker. This plasmid contains the *Pat* gene (see European Patent Publication 0 242 236) which encodes phosphinothricin acetyl transferase (PAT). The enzyme PAT confers resistance to herbicidal glutamine synthetase inhibitors such as phosphinothricin. The *pat* gene in p35S/Ac is under the control of the 35S promoter from Cauliflower Mosaic Virus (Odell et al. (1985) Nature 313:810-812) and the 3' region of the nopaline synthase gene from the T-DNA of the Ti plasmid of *Agrobacterium tumefaciens.*

The particle bombardment method (Klein et al. (1987) Nature 327:70-73) may be used to transfer genes to the callus culture cells. According to this method, gold particles (1 µm in diameter) are coated with DNA using the following technique. Ten µg of plasmid DNAs are added to 50 µL of a suspension of gold particles (60 mg per mL). Calcium chloride (50 µL of a 2.5 M solution) and spermidine free base (20 µL of a 1.0 M solution) are added to the particles. The suspension is vortexed during the addition of these solutions. After 10 minutes, the tubes are briefly centrifuged (5 sec at 15,000 rpm) and the supernatant removed. The particles are resuspended in 200 µL of absolute ethanol, centrifuged again and the supernatant removed. The ethanol rinse is performed again and the particles resuspended in a final volume of 30 µL of ethanol. An aliquot (5 µL) of the DNA-coated gold particles can be placed in the center of a Kapton^{™} flying disc (Bio-Rad Labs). The particles are then accelerated into the corn tissue with a Biolistic^{™} PDS-1000/He (Bio-Rad Instruments, Hercules CA), using a helium pressure of 1000 psi, a gap distance of 0.5 cm and a flying distance of 1.0 cm.

For bombardment, the embryogenic tissue is placed on filter paper over agarose-solidified N6 medium. The tissue is arranged as a thin lawn and covered a circular area of about 5 cm in diameter. The petri dish containing the tissue can be placed in the chamber of the PDS-1000/He approximately 8 cm from the stopping screen. The air in the chamber is then evacuated to a vacuum of 28 inches of Hg. The macrocarrier is accelerated with a helium shock wave using a rupture membrane that bursts when the He pressure in the shock tube reaches 1000 psi.

Seven days after bombardment the tissue can be transferred to N6 medium that contains gluphosinate (2 mg per liter) and lacks casein or proline. The tissue continues to grow slowly on this medium. After an additional 2 weeks the tissue can be transferred to fresh N6 medium containing gluphosinate. After 6 weeks areas of about 1 cm in diameter of actively growing callus can be identified on some of the plates containing the glufosinate-supplemented medium. These calli may continue to grow when sub-cultured on the selective medium.

Plants can be regenerated from the transgenic callus by first transferring clusters of tissue to N6 medium supplemented with 0.2 mg per liter of 2.4-D. After two weeks the tissue can be transferred to regeneration medium (Fromm et al. (1990) Bio/Techonology 8:833-839).

### EXAMPLE 5

### Expression of Chimeric Genes in Dicot Cells

A seed-specific expression cassette composed of the promoter and transcription terminator from the gene encoding the β subunit of the seed storage protein phaseolin from the bean *Phaseolus vulgaris* (Doyle et al. (1986) J. Biol: Chem. 261:9228-9238) can be used for expression of the instant polypeptides in transformed soybean. The phaseolin cassette includes about 500 nucleotides upstream (5') from the translation initiation codon and about 1650 nucleotides downstream (3') from the translation stop codon of phaseolin. Between the 5' and 3' regions are the unique restriction endonuclease sites Nco I (which includes the ATG translation initiation codon). SmaI, Kpn I and Xba I. The entire cassette is flanked by Hind III sites.

The cDNA fragment of this gene may be generated by polymerase chain reaction (PCR) of the cDNA clone using appropriate oligonucleotide primers. Cloning sites can be incorporated into the oligonucleotides to provide proper orientation of the DNA fragment when inserted into the expression vector. Amplification is then performed as described above, and the isolated fragment is inserted into a pUC 18 vector carrying the seed expression cassette.

Soybean embryos may then be transformed with the expression vector comprising sequences encoding the instant polypeptides. To induce somatic embryos, cotyledons, 3-5 mm in length dissected from surface sterilized, immature seeds of the soybean cultivar A2872, can be cultured in the light or dark ai 26°C on an appropriate agar medium for 6-10 weeks. Somatic embryos which produce secondary embryos are then excised and placed into a suitable liquid medium. After repeated selection for clusters of somatic embryos which multiplied as early, globular staged embryos, the suspensions are maintained as described below.

Soybean embryogenic suspension cultures can be maintained in 35 mL liquid media on a rotary shaker, 150 rpm, at 26°C with florescent lights on a 16:8 hour day/night schedule. Cultures are subcultured every two weeks by inoculating approximately 35 mg of tissue into 35 mL of liquid medium.

Soybean embryogenic suspension cultures may then be transformed by the method of particle gun bombardment (Klein et al. (1987) Nature (London) 327:70-73, U.S. Patent No. 4,945,050). A DuPont Biolistic^{™} PDS1000/HE instrument (helium retrofit) can be used for these transformations.

A selectable marker gene which can be used to facilitate soybean transformation is a chimeric gene composed of the 35S promoter from Cauliflower Mosaic Virus (Odell et al. (1985) Nature 313:810-812), the hygromycin phosphotransferase gene from plasmid pJR225 (from *E*. *coli;* Gritz et al.(1983) Gene 25:179-188) and the 3' region of the nopaline synthase gene from the T-DNA of the Ti plasmid *of Agrobacterium tumefaciens.* The seed expression cassette comprising the phaseolin 5' region, the fragment encoding the instant polypeptides and the phaseolin 3' region can be isolated as a restriction fragment. This fragment can then be inserted into a unique restriction site of the vector carrying the marker gene.

To 50 µL of a 60 mg/mL 1 µm gold particle suspension is added (in order): 5 µL DNA (1 µg/µL), 20 µl spermidine (0.1 M), and 50 µL CaCl₂ (2.5 M). The particle preparation is then agitated for three minutes, spun in a microfuge for 10 seconds and the supernatant removed. The DNA-coated particles are then washed once in 400 µL 70% ethanol and resuspended in 40 µL of anhydrous ethanol. The DNA/particle suspension can be sonicated three times for one second each. Five µL of the DNA-coated gold particles are then loaded on each macro carrier disk.

Approximately 300-400 mg of a two-week-old suspension culture is placed in an empty 60x15 mm petri dish and the residual liquid removed from the tissue with a pipette. For each transformation experiment, approximately 5-10 plates of tissue are normally bombarded. Membrane rupture pressure is set at 1100 psi and the chamber is evacuated to a vacuum of 28 inches mercury. The tissue is placed approximately 3.5 inches away from the retaining screen and bombarded three times. Following bombardment, the tissue can be divided in half and placed back into liquid and cultured as described above.

Five to seven days post bombardment, the liquid media may be exchanged with fresh media, and eleven to twelve days post bombardment with fresh media containing 50 mg/mL hygromycin. This selective media can be refreshed weekly. Seven to eight weeks post bombardment, green, transformed tissue may be observed growing from untransformed, necrotic embryogenic clusters. Isolated green tissue is removed and inoculated into individual flasks to generate new, clonally propagated, transformed embryogenic suspension cultures. Each new line may be treated as an independent transformation event. These suspensions can then be subcultured and maintained as clusters of immature embryos or regenerated into whole plants by maturation and germination of individual somatic embryos.

### EXAMPLE 6

### Expression of Chimeric Genes in Microbial Cells

The cDNAs encoding the instant polypeptides can be inserted into the T7 *E*. *coli* expression vector pBT430. This vector is a derivative of pET-3a (Rosenberg et al. (1987) Gene 56:125-135) which employs the bacteriophage T7 RNA polymerase/T7 promoter system. Plasmid pBT430 was constructed by first destroying the EcoR I and Hind III sites in pET-3a at their original positions. An oligonucleotide adaptor containing EcoR I and Hind III sites was inserted at the BamH I site of pET-3a. This created pET-3aM with additional unique cloning sites for insertion of genes into the expression vector. Then, the Nde I site at the position of translation initiation was converted to an Nco I site using oligonucleotide-directed mutagenesis. The DNA sequence of pET-3aM in this region, 5'-CATATGG, was converted to 5'-CCCATGG in pBT430.

Plasmid DNA containing a cDNA may be appropriately digested to release a nucleic acid fragment encoding the protein. This fragment may then be purified on a 1% low melting agarose gel. Buffer and agarose contain 10 ug/ml ethidium bromide for visualization of the DNA fragment. The fragment can then be purified from the agarose gel by digestion with GELase^{™} (Epicentre Technologies, Madison, WI) according to the manufacturer's instructions, ethanol precipitated, dried and resuspended in 20 µL of water. Appropriate oligonucleotide adapters may be ligated to the fragment using T4 DNA ligase (New England Biolabs (NEB), Beverly, MA). The fragment containing the ligated adapters can be purified from the excess adapters using low melting agarose as described above. The vector pBT430 is digested, dephosphorylated with alkaline phosphatase (NEB) and deproteinized with phenol/chloroform as described above. The prepared vector pBT430 and fragment can then be ligated at 16°C for 15 hours followed by transformation into DH5 electrocompetent cells (GIBCO BRL). Transformants can be selected on agar plates containing LB media and 100 µg/mL ampicillin. Transformants containing the gene encoding the instant polypeptides are then screened for the correct orientation with respect to the T7 promoter by restriction enzyme analysis.

For high level expression, a plasmid clone with the cDNA insert in the correct orientation relative to the T7 promoter can be transformed into *E*. *coli* strain BL21 (DE3) (Studier et al. (1986) J. Mol. Biol. 189:113-130). Cultures are grown in LB medium containing ampicillin (100 mg/L) at 25°C. At an optical density at 600 nm of approximately 1, IPTG (isopropylthio-β-galactoside, the inducer) can be added to a final concentration of 0.4 mM and incubation can be continued for 3 h at 25°. Cells are then harvested by centrifugation and re-suspended in 50 µL of 50 mM Tris-HCl at pH 8.0 containing 0.1 mM DTT and 0.2 mM phenyl methylsulfonyl fluoride. A small amount of 1 mm glass beads can be added and the mixture sonicated 3 times for about 5 seconds each time with a microprobe sonicator. The mixture is centrifuged and the protein concentration of the supernatant determined. One µg of protein from the soluble fraction of the culture can be separated by SDS-polyacrylamide gel electrophoresis. Gels can be observed for protein bands migrating at the expected molecular weight.

## Claims

1. An isolated polynucleotide encoding a polypeptide that has the effect of altering endosperm formation, comprising at least 250 amino acids, wherein:
(a) the amino acid sequence of the polypeptide and SEQ ID NO: 2 have at least 80% identity based on the Clustal alignment method; or
(b) the nucleotide sequence of the polynucleotide and SEQ ID NO: 1 have at least 80% identity based on the Clustal alignment method.

2. The isolated polynucleotide of Claim 1, wherein:
(a) the amino acid sequence of the polypeptide and SEQ ID NO: 2 have at least 85% identity based on the Clustal alignment method; or
(b) the nucleotide sequence and SEQ ID NO: 1 or SEQ ID NO: 29 have at least 85% identity based on the Clustal alignment method.

3. The isolated polynucleotide of Claim 1, wherein:
(a) the amino acid sequence of the polypeptide and SEQ ID NO: 2 have at least 95% identity based on the Clustal alignment method; or
(b) the nucleotide sequence and SEQ iD NO: 1 or SEQ ID NO: 29 have at least 95% identity based on the Clustal alignment method.

4. The isolated polynucleotide of Claim 1, wherein the polypeptide comprises SEQ ID NO: 2.

5. The isolated polynucleotide of Claim 1 comprising SEQ ID NO: 1 or SEQ ID NO: 29.

6. The isolated polynucleotide of any one of Claims 1-5, wherein the polypeptide is a fertilization independent endosperm protein.

7. The complement of the polynucleotide of any of Claims 1-6, wherein the complement and the polynucleotide contain the same number of nucleotides and are 100% complementary.

8. A method for transforming a cell comprising introducing the polynucleotide of any one of Claims 1-5 into a cell.

9. A method for transforming a cell comprising introducing the complement of Claim 7 into a cell.

10. A method of claim 8 or 9 wherein said cell is a plant cell.

11. A method of claim 14 wherein said plant cell is a monocot plant cell.

12. A method of claim 10 wherein said plant cell is a dicot plant cell.

13. The isolated polypeptide encoded by the polynucleotide of any of Claims 1-6.

14. A polypeptide of claim 13 which is a fertilization independent Endosperm protein.

15. A chimeric gene in which the polynucleotide of any one of claims 1-6 is operably linked to a promoter.

16. A plasmid vector comprising an isolated polynucleotide of any one of claims 1-6 or a chimeric gene of Claim 15.

17. A plant expression vector comprising a polynucleotide of any one of claims 1-6 under the transcriptional control of 5' and 3' regulatory sequences and a dominant selectable marker.

18. A host cell comprising a polynucleotide of any one of claims 1-6 or a chimeric gene of Claim 15.

19. A host cell of claim 18 which is a plant cell.

20. A host plant cell of claim 19 which is:
(a) a monocot plant cell; or
(b) a dicot plant cell.

21. A method for transforming a host plant comprising the transfer of a polynucleotide of any one of claims 1-6 into said host plant, resulting in stable inheritance.

22. A method of claim 21 wherein said plant is:
(a) a monocot; or
(b) a dicot.

23. A transgenic plant stably transformed with a polynucleotide of any one of claims 1-6.

24. A transgenic plant of claim 23 in which a polypeptide of Claim 13 or 14 is present at higher levels than normal or in cell types or developmental stages in which it is not normally found; and wherein said polypeptide has an effect of altering endosperm formation.

25. A plant in which expression of an endogenous gene encoding a polypeptide defined in claim 13 or 14 is reduced or eliminated, such that said polypeptide is present at lower levels than normal and endosperm formation is altered; wherein said reduction or elimination is via antisense inhibition or co-suppression.

26. A plant of claim 24 or 25 which is a monocot plant.

27. A plant of claim 24 or 25 which is a dicot plant.

## Patentansprüche

1. Isoliertes Polynukleotid, das für ein Polypeptid codiert, das die Wirkung einer Veränderung der Endospermbildung hat, das wenigstens 250 Aminosäuren umfasst, wobei:
(a) die Aminosäuresequenz des Polypeptids und SEQ ID NO:2 wenigstens 80% Identität, basierend auf der Clustal-Alignment-Methode, haben oder
(b) die Nukleotidsequenz des Polynukleotids und SEQ ID NO:1 wenigstens 80% Identität, basierend auf der Clustal-Alignment-Methode, haben.

2. Isoliertes Polynukleotid nach Anspruch 1, wobei:
(a) die Aminosäuresequenz des Polypeptids und SEQ ID NO:2 wenigstens 85% Identität, basierend auf der Clustal-Alignment-Methode, haben oder
(b) die Nukleotidsequenz und SEQ ID NO:1 oder SEQ ID NO:29 wenigstens 85% Identität, basierend auf der Clustal-Alignment-Methode, haben.

3. Isoliertes Polynukleotid nach Anspruch 1, wobei:
(a) die Aminosäuresequenz des Polypeptids und SEQ ID NO:2 wenigstens 95% Identität, basierend auf der Clustal-Alignment-Methode, haben oder
(b) die Nukleotidsequenz und SEQ ID NO:1 oder SEQ ID NO:29 wenigstens 95% Identität, basierend auf der Clustal-Alignment-Methode, haben.

4. Isoliertes Polynukleotid nach Anspruch 1, wobei das Polypeptid SEQ ID NO:2 umfasst.

5. Isoliertes Polynukleotid nach Anspruch 1, das SEQ ID NO:1 oder SEQ ID NO:29 umfasst.

6. Isoliertes Polynukleotid nach einem der Ansprüche 1 bis 5, wobei das Polypeptid ein Befruchtungs-unabhängiges Endospermprotein ("fertilization independent endosperm protein") ist.

7. Komplement des Polynukleotids nach einem der Ansprüche 1 bis 6, wobei das Komplement und das Polynukleotid die selbe Anzahl von Nukleotiden enthalten und 100% komplementär sind.

8. Verfahren zum Transformieren einer Zelle, das Einführen des Polynukleotids nach einem der Ansprüche 1 bis 5 in eine Zelle umfasst.

9. Verfahren zum Transformieren einer Zelle, das Einführen des Komplements nach Anspruch 7 in eine Zelle umfasst.

10. Verfahren nach Anspruch 8 oder 9, wobei die Zelle eine Pflanzenzelle ist.

11. Verfahren nach Anspruch 10, wobei die Pflanzenzelle eine monokotyle Pflanzenzelle ist.

12. Verfahren nach Anspruch 10, wobei die Pflanzenzelle eine dikotyle Pflanzenzelle ist.

13. Isoliertes Polypeptid, das durch das Polynukleotid nach einem der Ansprüche 1 bis 6 codiert wird.

14. Polypeptid nach Anspruch 13, welches ein Befruchtungs-unabhängiges Endospermprotein ist.

15. Chimäres Gen, in welchem das Polynukleotid nach einem der Ansprüche 1 bis 6 funktionell mit einem Promotor verknüpft ist.

16. Plasmidvektor, der ein isoliertes Polynukleotid nach einem der Ansprüche 1 bis 6 oder ein chimäres Gen nach Anspruch 15 umfasst.

17. Pflanzenexpressionsvektor, der ein Polynukleotid nach einem der Ansprüche 1 bis 6 unter der transkriptionalen Kontrolle von 5'- und 3'-regulatorischen Sequenzen und einen dominanten selektierbaren Marker umfasst.

18. Wirtszelle, die ein Polynukleotid nach einem der Ansprüche 1 bis 6 oder ein chimeres Gen nach Anspruch 15 umfasst.

19. Wirtszelle nach Anspruch 18, die eine Pflanzenzelle ist.

20. Wirtspflanzenzelle nach Anspruch 19, die
(a) eine monokotyle Pflanzenzelle oder
(b) eine dikotyle Pflanzenzelle ist.

21. Verfahren zum Transformieren einer Wirtspflanze, umfassend den Transfer eines Polynukleotids nach einem der Ansprüche 1 bis 6 in die genannte Wirtspflanze, was in einer stabilen Vererbung resultiert.

22. Verfahren nach Anspruch 21, wobei die Pflanze
(a) eine Monokotyle oder
(b) eine Dikotyle ist.

23. Transgene Pflanze, die stabil mit einem Polynukleotid nach einem der Ansprüche 1 bis 6 transformiert ist.

24. Transgene Pflanze nach Anspruch 23, in der ein Polypeptid nach Anspruch 13 oder 14 in höheren Konzentrationen als normal oder in Zelltypen oder Entwicklungsstufen, in denen es normalerweise nicht gefunden wird, vorhanden ist, und wobei das Polypeptid den Effekt einer Veränderung der Endospermbildung hat.

25. Pflanze, in welcher eine Expression eines endogenen Gens, das für ein Polypeptid codiert, das in Anspruch 13 oder 14 definiert ist, verringert oder eliminiert ist, sodass das Polypeptid in geringeren Konzentrationen als normal vorhanden ist und eine Endospermbildung verändert ist; wobei die Reduzierung oder Eliminierung über Antisense-Inhibierung oder -Cosuppresion ist.

26. Pflanze nach Anspruch 24 oder 25, welche eine monokotyle Pflanze ist.

27. Pflanze nach Anspruch 24 oder 25, welche eine dikotyle Pflanze ist.

## Revendications

1. Polynucléotide isolé codant pour un polypeptide qui a pour effet d'altérer la formation d'endosperme, comprenant au moins 250 acides aminés, dans lequel :
(a) la séquence d'acides aminés du polypeptide et SEQ ID NO :2 ont au moins 80 % d'identité sur la base de la méthode d'alignement Clustal ; ou
(b) la séquence nucléotidique du polynucléotide et SEQ ID NO :1 ont au moins 80 % d'identité sur la base de la méthode d'alignement Clustal.

2. Polynucléotide isolé de la revendication 1, dans lequel:
(a) la séquence d'acides aminés du polypeptide et SEQ ID NO :2 ont au moins 85 % d'identité sur la base de la méthode d'alignement Clustal ; ou
(b) la séquence nucléotidique et SEQ ID NO :1 ou SEQ ID NO :29 ont au moins 85 % d'identité sur la base de la méthode d'alignement Clustal.

3. Polynucléotide isolé de la revendication 1, dans lequel :
(a) la séquence d'acides aminés du polypeptide et SEQ ID NO :2 ont au moins 95 % d'identité sur la base de la méthode d'alignement Clustal ; ou
(b) la séquence nucléotidique et SEQ ID NO :1 ou SEQ ID NO :29 ont au moins 95 % d'identité sur la base de la méthode d'alignement Clustal.

4. Polynucléotide isolé de la revendication 1, dans lequel le polypeptide comprend SEQ ID NO : 2.

5. Polynucléotide isolé de la revendication 1 comprenant SEQ ID NO : 1 ou SEQ ID NO : 29.

6. Polynucléotide isolé de l'une quelconque des revendications 1 à 5, dans lequel le polypeptide est une protéine d'endosperme indépendante de la fécondation.

7. Complément du polynucléotide de l'une quelconque des revendications 1 à 6, dans lequel le complément et le polynucléotide contiennent le même nombre de nucléotides et sont complémentaires à 100 %.

8. Méthode de transformation d'une cellule comprenant l'introduction du polynucléotide de l'une quelconque des revendications 1 à 5 dans une cellule.

9. Méthode de transformation d'une cellule comprenant l'introduction du complément de la revendication 7 dans une cellule.

10. Méthode de la revendication 8 ou 9 dans laquelle ladite cellule est une cellule de plante.

11. Méthode de la revendication 10 dans laquelle ladite cellule de plante est une cellule de plante monocotylédone.

12. Méthode de la revendication 10 dans laquelle ladite cellule de plante est une cellule de plante dicotylédone.

13. Polypeptide isolé codé par le polynucléotide de l'une quelconque des revendications 1 à 6.

14. Polypeptide de la revendication 13 qui est une protéine d'endosperme indépendante de la fécondation.

15. Gène chimère dans lequel le polynucléotide de l'une quelconque des revendications 1 à 6 est lié de manière fonctionnelle à un promoteur.

16. Vecteur plasmidique comprenant un polynucléotide isolé de l'une quelconque des revendications 1 à 6 ou un gène chimère de la revendication 15.

17. Vecteur d'expression de plante comprenant un polynucléotide de l'une quelconque des revendications 1 à 6 sous le contrôle transcriptionnel de séquences régulatrices 5' et 3' et un marqueur de sélection dominant.

18. Cellule hôte comprenant un polynucléotide de l'une quelconque des revendications 1 à 6 ou un gène chimère de la revendication 15.

19. Cellule hôte de la revendication 18 qui est une cellule de plante.

20. Cellule hôte de plante de la revendication 19 qui est :
(a) une cellule de plante monocotylédone ; ou
(b) une cellule de plante dicotylédone.

21. Méthode de transformation d'une plante hôte comprenant le transfert d'un polynucléotide de l'une quelconque des revendications 1 à 6 dans ladite plante hôte, résultant en une descendance stable.

22. Méthode de la revendication 21 dans laquelle ladite plante est :
(a) une monocotylédone ; ou
(b) une dicotylédone.

23. Plante transgénique transformée de manière stable avec un polynucléotide de l'une quelconque des revendications 1 à 6.

24. Plante transgénique de la revendication 23 dans laquelle un polypeptide de la revendication 13 ou 14 est présent à des taux plus élevés que la normale ou dans des types cellulaires ou à des stades de développement dans lesquels il n'est normalement pas trouvé ; et dans laquelle ledit polypeptide a un effet d'altération de la formation de l'endosperme.

25. Plante dans laquelle l'expression d'un gène endogène codant pour un polypeptide défini dans la revendication 13 ou 14 est réduite ou éliminée, de telle sorte que ledit polypeptide soit présent à des taux plus faibles que la normale et que la formation de l'endosperme soit altérée ; dans laquelle ladite réduction ou élimination se fait par inhibition antisens ou par co-suppression.

26. Plante de la revendication 24 à 25 qui est une plante monocotylédone.

27. Plante de la revendication 24 ou 25 qui une plante dicotylédone.


### SEQUENCE LISTING

### SEQUENCE LISTING

<110> E.I. du Pont de Nemours and Company
<120> Plant Reproduction Proteins
<130> BB1388 PCT
<140>
   <141>
<150> 60/151,575
   1999-08-31
<160> 72
<170> Microsoft Office 97
<210> 1
   <211> 1643
   <212> DNA
   <213> Zea mays
<400> 1
   gcacgaggcc ggaagaagtc gccgcgtgag gtcagtgtcc ccgttgctgc cgcctctaac   60
   ccgaagccta ggccgctgcc ggtgcataac aaggagaatc aggcggaggg gaaagtagca  120
   gaggaggggg cagcaactga ggagggggag aagtaccggg cggaaccgga aatcttgccg  180
   ctgccgccgg ccatggcgaa gctgggcccg gggcaggggc tcgggtgcga ggcggcggag  240
   gggtcgctcg tgcccagccg gaagcgggag tacaagccct gcggcaagca cactgagggg  300
   aagcgcccgc tatatgctat cgggttcaac ttcatggacg cgcgctacta cgacgtcttc  360
   gccaccgtcg gcggcaaccg cgtgacaact taccgctgcc ttgagaatgg tagtttcgct  420
   cttctacaag cttacgttga tgaggataag gatgagtcgt tctatactct aagctgggct  480
   cgtgaccatg ttgatggctc accactgctg gtggcagcag gaagcaatgg gatcattcgg  540
   gtcatcaatt gtgctacaga aaagttagct aagagctttg ttggccatgg cgactcaata  600
   aatgagataa gaactcaacc gttgaagcct tcgctcatca tttctgcaag caaggatgaa  660
   tctgttaggc tatggaatgt ccatacaggg atctgtatct tgatatttgc tggagctgga  720
   ggtcatcgca atgaagtatt gagtgttgac ttccatccta gtgatattga acgttttgca  780
   agttgtggca tggacaacac tgtgaaaatc tggtcaatga aagaattttg gctatatgtt  840
   gacaaatcat attcatggac tgaccttcca tcaaagtttc caacaaaata tgtccagttt  900
   ccagtcttga ttgctgcagt acactctaac tatgttgatt gtacaagatg gcttggtgac  960
   ttcatcctat caaagagtgt tgacaatgaa attgtgcttt gggaaccgaa gacaaaagaa 1020
   cagagtcctg gggagggaag catcgatatc cttcagaagt atcctgtccc agaatgtgac 1080
   atttggttta tcaaattttc atgtgatttt cacttcaatc agttggcgat aggcaaccgt 1140
   gaaggcaaaa tctacgtgtg ggaagtacag tccagccctc ctgtcctcat tgctcggctg 1200
   tataatcagc agtgtaaatc gccgataaga caaactgcag tgtccttcga tggaagcaca 1260
   atccttggag ctggtgaaga cggcaccatc tggcggtggg atgaagtgga ccatccgagc 1320
   tccagaaact gaagaagtgt tgccgctcaa tgctggactg atggttacgc tcggttgggg 1380
   ttgcgatggt tgaatccgtt ggtggaaagt gccacctggt gttttttcta gtcaaaatgg 1440
   ttggtgttaa cagaatattg aatgcttcga atgttgaaag ttgggatgct tgtgctggta 1500
   ctctgctccg tggacgagtg aacttaggtg ccgtttggtt cacatatttg taacgtaatg 1560
   ggtaacagat aacgttaaat catgtttgtt ttatttcaac cgtaatcaga taccacatta 1620
   aaattaaaaa aaaaaaaaaa aaa                                          1643
<210> 2
   <211> 379
   <212> PRT
   <213> Zea mays
<400> 2
   Met ala Lys Leu Gly Pro Gly Gln Gly Leu Gly Cys Glu Ala Ala Glu
    1               5                  10                  15
   Gly Ser Leu Val Pro Ser Arg Lys Arg Glu Tyr Lys Pro Cys Gly Lys
               20                  25                  30
   His Thr Glu Gly Lys Arg Pro Leu Tyr Ala Ile Gly Phe Asn Phe Met
           35                  40                  45
   Asp Ala Arg Tyr Tyr Asp Val Phe Ala Thr Val Gly Gly Asn Arg Val
       50                  55                  60
   Thr Thr Tyr Arg Cys Leu Glu Asn Gly Ser Phe Ala Leu Leu Gln Ala
   65                  70                  75                  80
   Tyr Val Asp Glu Asp Lys Asp Glu Ser Phe Tyr Thr Leu Ser Trp Ala
                   85                  90                  95
   Arg Asp His Val Asp Gly Ser Pro Leu Leu Val Ala Ala Gly Ser Asn
               100                 105                 110
   Gly Ile Ile Arg Val Ile Asn Cys Ala Thr Glu Lys Leu Ala Lys Ser
           115                 120                 125
   Phe Val Gly His Gly Asp Ser Ile Asn Glu Ile Arg Thr Gln Pro Leu
       130                 135                 140
   Lys Pro Ser Leu Ile Ile Ser Ala Ser Lys Asp Glu Ser Val Arg Leu
   145                 150                 155                 160
   Trp Asn Val His Thr Gly Ile Cys Ile Leu Ile Phe Ala Gly Ala Gly
                   165                 170                 175
   Gly His Arg Asn Glu Val Leu Ser Val Asp Phe His Pro Ser Asp Ile
               180                 185                 190
   Glu Arg Phe Ala Ser Cys Gly Met Asp Asn Thr Val Lys Ile Trp Ser
           195                 200                 205
   Met Lys Glu Phe Trp Leu Tyr Val Asp Lys Ser Tyr Ser Trp Thr Asp
       210                 215                 220
   Leu Pro Ser Lys Phe Pro Thr Lys Tyr Val Gln Phe Pro Val Leu Ile
   225                 230                 235                 240
   Ala Ala Val His Ser Asn Tyr Val Asp Cys Thr Arg Trp Leu Gly Asp
                   245                 250                 255
   Phe Ile Leu Ser Lys Ser Val Asp Asn Glu Ile Val Leu Trp Glu Pro
               260                 265                 270
   Lys Thr Lys Glu Gln Ser Pro Gly Glu Gly Ser Ile Asp Ile Leu Gln
           275                 280                 285
   Lys Tyr Pro Val Pro Glu Cys Asp Ile Trp Phe Ile Lys Phe Ser Cys
       290                 295                 300
   Asp Phe His Phe Asn Gln Leu Ala Ile Gly Asn Arg Glu Gly Lys Ile
   305                 310                 315                 320
   Tyr Val Trp Glu Val Gln Ser Ser Pro Pro Val Leu Ile Ala Arg Leu
                   325                 330                 335
   Tyr Asn Gln Gln Cys Lys Ser Pro Ile Arg Gln Thr Ala Val Ser Phe
               340                 345                 350
   Asp Gly Ser Thr Ile Leu Gly Ala Gly Glu Asp Gly Thr Ile Trp Arg
           355                 360                 365
   Trp Asp Glu Val Asp His Pro Ser Ser Arg Asn
       370                 375
<210> 3
   <211> 348
   <212> DNA
   <213> Zea mays
<400> 3
   gccatcgcca gttgcaatag tgtgataatt taccgatgcc ttgagaatgg tggttttggt  60
   cttctacaaa attatgttga tgaggataag gatgagtcat tctacactct aagctggacc 120
   atcgatcaag ttgatagctc accgctgttg gtggccgcag gaagcaatcg gatcattcgg 180
   gtcatcaatt gtgctaccga aaagttagat aagagcttag ttggccatgg tggttcaata 240
   catgagataa ggactcatgc ctcgaagcca tcactcatca tttctgccag caaggatgaa 300
   tctattaggc tatggaatgt ccatactggg atttgcatct tagtcttt              348
<210> 4
   <211> 461
   <212> PRT
   <213> Zea mays
<400> 4
   Met Pro Pro Ser Lys Ala Arg Arg Lys Arg Ser Leu Arg Asp Ile Thr
   1               5                   10                  15
   Ala Thr Val Ala Thr Gly Thr Val Ala Asn Ser Lys Pro Gly Ser Ser
               20                  25                  30
   Ser Thr Asn Glu Gly Lys Gln Gln Asp Lys Lys Lys Glu Gly Pro Gln
           35                  40                  45
   Glu Pro Asp Ile Pro Pro Leu Pro Pro Val Val Val Asn Ile Val Pro
       50                  55                  60
   Arg Gln Gly Leu Gly Cys Glu Val Val Glu Gly Leu Leu Val Pro Ser
   65                  70                  75                  80
   Arg Lys Arg Glu Tyr Lys Pro Asn Ser Lys Tyr Thr Val Gly Asn His
                   85                  90                  95
   Pro Ile Tyr Ala Ile Gly Phe Asn Phe Ile Asp Met Arg Tyr Tyr Asp
               100                 105                 110
   Val Phe Ala Ile Ala Ser Cys Asn Ser Val Ile Ile Tyr Arg Cys Leu
           115                 120                 125
   Glu Asn Gly Gly Phe Gly Leu Leu Gln Asn Tyr Val Asp Glu Asp Lys
   130 135 140
   Asp Glu Ser Phe Tyr Thr Leu Ser Trp Thr Ile Asp Gln Val Asp Ser
   145 150 155 160
   Ser Pro Leu Leu Val Ala Ala Gly Ser Asn Arg Ile Ile Arg Val Ile
   165 170 175
   Asn Cys Ala Thr Glu Lys Leu Asp Lys Ser Leu Val Gly His Gly Gly
   180 185 190
   Ser Ile His Glu Ile Arg Thr His Ala Ser Lys Pro Ser Leu Ile Ile
   195 200 205
   Ser Ala Ser Lys Asp Glu Ser Ile Arg Leu Trp Asn Val His Thr Gly
   210 215 220
   Ile Cys Ile Leu Val Phe Ala Gly Ala Gly Gly His Arg His Asp Val
   225 230 235 240
   Leu Ser Val Asp Phe His Pro Thr Glu Val Gly Ile Phe Ala Ser Cys
   245 250 255
   Gly Met Asp Asn Thr Val Lys Ile Trp Ser Met Lys Glu Phe Trp Ile
   260 265 270
   Tyr Val Glu Lys Ser Tyr Ser Trp Thr Gly His Pro Ser Lys Phe Pro
   275 280 285
   Thr Arg Asn Ile Gln Phe Pro Val Leu Thr Ala Ala Val His Ser Asp
   290 295 300
   Tyr Val Asp Cys Thr Arg Trp Leu Gly Asp Phe Ile Leu Ser Lys Ser
   305 310 315 320
   Val Lys Asn Ala Val Leu Leu Trp Glu Pro Lys Pro Asp Lys Arg Arg
   325 330 335
   Pro Gly Glu Gly Ser Val Asp Val Leu Gln Lys Tyr Pro Val Pro Lys
   340 345 350
   Cys Ser Leu Trp Phe Met Lys Phe Ser Cys Asp Phe Tyr Ser Asn Gln
   355 360 365
   Met ala Ile Gly Asn Asn Lys Gly Glu Ile Tyr Val Trp Glu Val Gln
   370 375 380
   Ser Ser Pro Pro Val Leu Ile Asp Arg Leu Cys Asn Gln Glu Cys Lys
   385 390 395 400
   Ser Pro Ile Arg Gln Thr Ala Val Ser Phe Asp Gly Ser Thr Ile Leu
   405 410 415
   Gly Ala Ala Asp Asp Gly Ala Ile Trp Arg Trp Asp Glu Val Asp Pro
   420 425 430
   Ala Ala Ser Ser Ser Lys Pro Asp Gln Ala Ala Ala Pro Ala Ala Gly
   435 440 445
   Val Gly Ala Gly Ala Gly Ala Asp Ala Asp Ala Asp Ala
   450 455 460
<210> 5
   <211> 1749
   <212> DNA
   <213> Zea mays
<400> 5
   gcacgaggaa ccgccgccat cacatccact gcctcaacta gtgttaccac ctatggttca   60
   ttgttgtgtc tgcttcttgt agcactgttg gtctacaaac attcatattt ctctcaacat  120
   ctggcacagc atgccgcctt ccaaagcacg ccgaaagagg tcacttcgtg atatcactgc  180
   caccgttgcc actgggactg ttgccaactc gaaacctggc tcatcatcga cgaacgaggg  240
   gaagcaacag gacaagaaaa aggagggtcc acaggaaccg gacatcccac cattaccgcc  300
   ggtggtggtg aatatagtcc cacgacaagg attaggatgt gaagtagtgg aagggctact  360
   cgtgcctagt cggaagcgag agtacaagcc caatagcaag tatactgtgg gaaatcaccc  420
   gatctatgcc atcgggttca atttcattga catgcgctac tatgatgtct ttgccatcgc  480
   cagttgcaat agtgtgataa tttaccgatg ccttgagaat ggtggttttg gtcttctaca  540
   aaattatgtt gatgaggata aggatgagtc attctacact ctaagctgga ccatcgatca  600
   agttgatagc tcaccgctgt tggtggccgc aggaagcaat cggatcattc gggtcatcaa  660
   ttgtgctacc gaaaagttag ataagagctt agttggccat ggtggttcaa tacatgagat  720
   aaggactcat gcctcgaagc catcactcat catttctgcc agcaaggatg aatctattag  780
   gctatggaat gtccatactg ggatttgcat cttagtcttt gcaggggctg gaggccatcg  840
   acatgatgtg ttgagtgttg acttccaccc taccgaggtt gggatttttg caagttgtgg  900
   catggacaat actgtgaaaa tttggtcaat gaaagaattt tggatatatg ttgaaaaatc  960
   atattcatgg actggccatc catcaaagtt tccaacgagg aatatccagt ttccggtctt 1020
   gactgctgca gtacactctg actatgttga ttgtacaaga tggcttggtg acttcatcct 1080
   atcaaagagt gtaaagaatg cagttttgct ttgggaacca aaaccagaca agcgtaggcc 1140
   tggggagggg agtgttgatg ttcttcagaa gtacccggtg ccaaagtgtt cattatggtt 1200
   tatgaaattt tcatgtgatt tttactccaa ccagatggca ataggcaaca ataaaggcga 1260
   gatctatgtc tgggaagtgc agtccagccc gcccgtctta attgaccggc tgtgcaacca 1320
   ggaatgcaag tcgccgataa ggcagaccgc agtgtcattc gacggaagca cgatccttgg 1380
   agccgccgac gacggcgcga tctggcggtg ggacgaagtg gaccctgctg cttccagctc 1440
   caaacctgat caagctgctg cgcccgccgc cggtgtcggt gccggtgccg gtgccgacgc 1500
   cgacgccgac gcctgagcga gaggaccgtc gccgcccgcc ggttcacatc gatcgtactc 1560
   cgtgctggtt gattagcttt acccattggt atgttttggt tcagagtcgc cagatctagt 1620
   gtgtggctga acgttgaatg ttaggatgct gctgtttgtt atgctctgag tcttgagttc 1680
   actttgttaa tttgcaccgt ggatgagatg aataacttga cgttgcaaaa aaaaaaaaaa 1740
   aaaaaaaaa                                                         1749
<210> 6
   <211> 461
   <212> PRT
   <213> Zea mays
<400> 6
   Met Pro Pro Ser Lys Ala Arg Arg Lys Arg Ser Leu Arg Asp Ile Thr
    1               5                  10                  15
   Ala Thr Val Ala Thr Gly Thr Val Ala Asn Ser Lys Pro Gly Ser Ser
               20                  25                  30
   Ser Thr Asn Glu Gly Lys Gln Gln Asp Lys Lys Lys Glu Gly Pro Gln
           35                  40                  45
   Glu Pro Asp Ile Pro Pro Leu Pro Pro Val Val Val Asn Ile Val Pro
       50                  55                  60
   Arg Gln Gly Leu Gly Cys Glu Val Val Glu Gly Leu Leu Val Pro Ser
   65                  70                  75                  80
   Arg Lys Arg Glu Tyr Lys Pro Asn Ser Lys Tyr Thr Val Gly Asn His
                   85                  90                  95
   Pro Ile Tyr Ala Ile Gly Phe Asn Phe Ile Asp Met Arg Tyr Tyr Asp
               100                 105                 110
   Val Phe Ala Ile Ala Ser Cys Asn Ser Val Ile Ile Tyr Arg Cys Leu
           115                 120                 125
   Glu Asn Gly Gly Phe Gly Leu Leu Gln Asn Tyr Val Asp Glu Asp Lys
       130                 135                 140
   Asp Glu Ser Phe Tyr Thr Leu Ser Trp Thr Ile Asp Gln Val Asp Ser
   145                 150                 155                 160
   Ser Pro Leu Leu Val Ala Ala Gly Ser Asn Arg Ile Ile Arg Val Ile
                   165                 170                 175
   Asn Cys Ala Thr Glu Lys Leu Asp Lys Ser Leu Val Gly His Gly Gly
               180                 185                 190
   Ser Ile His Glu Ile Arg Thr His Ala Ser Lys Pro Ser Leu Ile Ile
           195                 200                 205
   Ser Ala Ser Lys Asp Glu Ser Ile Arg Leu Trp Asn Val His Thr Gly
       210                 215                 220
   Ile Cys Ile Leu Val Phe Ala Gly Ala Gly Gly His Arg His Asp Val
   225                 230                 235                 240
   Leu Ser Val Asp Phe His Pro Thr Glu Val Gly Ile Phe Ala Ser Cys
                   245                 250                 255
   Gly Met Asp Asn Thr Val Lys Ile Trp Ser Met Lys Glu Phe Trp Ile
               260                 265                 270
   Tyr Val Glu Lys Ser Tyr Ser Trp Thr Gly His Pro Ser Lys Phe Pro
           275                 280                 285
   Thr Arg Asn Ile Gln Phe Pro Val Leu Thr Ala Ala Val His Ser Asp
       290                 295                 300
   Tyr Val Asp Cys Thr Arg Trp Leu Gly Asp Phe Ile Leu Ser Lys Ser
   305                 310                 315                 320
   Val Lys Asn Ala Val Leu Leu Trp Glu Pro Lys Pro Asp Lys Arg Arg
                   325                 330                 335
   Pro Gly Glu Gly Ser Val Asp Val Leu Gln Lys Tyr Pro Val Pro Lys
               340                 345                 350
   Cys Ser Leu Trp Phe Met Lys Phe Ser Cys Asp Phe Tyr Ser Asn Gln
           355                 360                 365
   Met ala Ile Gly Asn Asn Lys Gly Glu Ile Tyr Val Trp Glu Val Gln
       370                 375                 380
   Ser Ser Pro Pro Val Leu Ile Asp Arg Leu Cys Asn Gln Glu Cys Lys
   385                 390                 395                 400
   Ser Pro Ile Arg Gln Thr Ala Val Ser Phe Asp Gly Ser Thr Ile Leu
                   405                 410                 415
   Gly Ala Ala Asp Asp Gly Ala Ile Trp Arg Trp Asp Glu Val Asp Pro
               420                 425                 430
   Ala Ala Ser Ser Ser Lys Pro Asp Gln Ala Ala Ala Pro Ala Ala Gly
           435                 440                 445
   Val Gly Ala Gly Ala Gly Ala Asp Ala Asp Ala Asp Ala
       450                 455                 460
<210> 7
   <211> 765
   <212> DNA
   <213> Zea mays
<400> 7
   tggcacgaaa agtttccaac aaaatatgtc cagtttccag tcttgattgc tgcagtacac   60
   tctaactatg ttgattgtac aagatggctt ggtgacttca tcctatcaaa gagtgttgac  120
   aatgaaattg tgctttggga accgaagaca aaagaacaga gtcctgggga gggaagcatc  180
   gatatccttc agaagtatcc tgtcccagaa tgtgacattt ggtttatcaa attttcatgt  240
   gattttcact tcaatcagtt ggcgataggc aaccgtgaag gcaaaatcta cgtgtgggaa  300
   gtacagtcca gccctcctgt cctcattgct cggctgtata atcagcagtg taaatcgccg  360
   ataagacaaa ctgcagtgtc cttcgatgga agcacaatcc ttggagctgg tgaagacggc  420
   accatctggc ggtgggatga agtggaccat ccgagctcca gaagctgaag aagtgttgcc  480
   gctcaatgct ggactgatgg ttacgctcgg ttggggttgt gatggttgaa tccgttggcg  540
   gaaagtgcca cctggtgttt tttctagtca aaatggttgg tgttaacaga atattgaatg  600
   cttcgaatgt tgaaagttgg gatgcttgtg ctggtactct gctccgcgga cgagtgaact  660
   tagtttgttg caactttggg aaccgttgtc atctgtttgt tctgcatttc taaaaagaga  720
   gcaaatttca ggataaaaaa aaaaaaaaaa aactcgagac tagtt                  765
<210> 8
   <211> 155
   <212> PRT
   <213> Zea mays
<400> 8
   Trp His Glu Lys Phe Pro Thr Lys Tyr Val Gln Phe Pro Val Leu Ile
    1               5                  10                  15
   Ala Ala Val His Ser Asn Tyr Val Asp Cys Thr Arg Trp Leu Gly Asp
               20                  25                  30
   Phe Ile Leu Ser Lys Ser Val Asp Asn Glu Ile Val Leu Trp Glu Pro
           35                  40                  45
   Lys Thr Lys Glu Gln Ser Pro Gly Glu Gly Ser Ile Asp Ile Leu Gln
       50                  55                  60
   Lys Tyr Pro Val Pro Glu Cys Asp Ile Trp Phe Ile Lys Phe Ser Cys
   65                  70                  75                  80
   Asp Phe His Phe Asn Gln Leu Ala Ile Gly Asn Arg Glu Gly Lys Ile
                   85                  90                  95
   Tyr Val Trp Glu Val Gln Ser Ser Pro Pro Val Leu Ile Ala Arg Leu
               100                 105                 110
   Tyr Asn Gln Gln Cys Lys Ser Pro Ile Arg Gln Thr Ala Val Ser Phe
           115                 120                 125
   Asp Gly Ser Thr Ile Leu Gly Ala Gly Glu Asp Gly Thr Ile Trp Arg
       130                 135                 140
   Trp Asp Glu Val Asp His Pro Ser Ser Arg Ser
   145                 150                 155
<210> 9
   <211> 1626
   <212> DNA
   <213> Eucalyptus grandis
<400> 9
   gcaccagctc gttcgccgtt cggcgtcttc accggcggcg cgcgccgcac tgcgtaccca   60
   ccggctgtcg cgttctcgcg gatcgaactc gaggaaaagg catcggcggc ggatcggggc  120
   aaatggcgaa gatcgcgccc gggtgcgaac cggtggcggg gacgctgacc ccgtcgaaga  180
   agagggagta cagggtcacc aacaggctcc aggaggggaa gcgtcccctc tatgccgtcg  240
   tcttcaactt catcgactcc cgctacttca acgtattcgc caccgtcggc ggcaaccggg  300
   ttactgttta tcagtgtctc gaagggggag taatagctgt gttgcagtca tacattgatg  360
   aagataagga cgagtcgttt tacacggtca gctgggcgtg caacattgat agaaccccat  420
   ttgtggtggc gggaggaatc aatggtatca tccgtgtaat tgatgctggc aatgagaaga  480
   tacacaggag ttttgtaggc catggggatt caataaatga aatcaggact caaccattga  540
   acccatccct catcgtgtct gctagcaaag atgaatccgt taggctctgg aacgttcata  600
   cgggaatttg tatcctgata tttgctggag ctgggggtca tcgcaatgaa gttttgagtg  660
   tggacttcca tccttccgac aagtaccgta ttgcaagttg tggtatggac aatacggtta  720
   aaatctggtc aatgaaagag ttctggacat atgtggagaa gtcatttaca tggacagatc  780
   ttccatcgaa gtttcccacc aaatacgtgc agtttccagt tttcatagct ccagttcatt  840
   caaactatgt tgactgcaac aggtggcttg gtgattttgt tctgtcaaag agtgttgaca  900
   acgagattgt gctttgggaa cccaaaatga aggaacaatc tccgggagag ggatcggtgg  960
   atatccttca gaaatatcca gttccagagt gtgacatttg gttcatcaaa ttttcctgtg 1020
   actttcatta tcactcaatt gctataggaa atagggaagg gaagatctac gtatgggagc 1080
   tgcagagtag ccctcctgtt ctaattgcaa agttgtctca ttcccaatca aaatccccaa 1140
   tcagacagac cgccatgtca tttgatggga gcacaatcct gagctgctgt gaggatggta 1200
   ctatatggcg ctgggatgca attacggcat caacatccta agccttccat ggcagatgga 1260
   ctggagaact ccgtttgtaa ttaggaatcc ctcttgtgtg ggcatgttcc ccaccatgta 1320
   tcagctaaat gggagctgct tcaacctctt atctcgatgg agactcgaat agcatcaccg 1380
   cacaggtgca agcggacaac tgctttttgg taacgaagaa agcaagtgga tgatttggtt 1440
   gtgcatcagt ctgaacgatt tatgaagtta ctttttggtg tcaaatgtac tctccgtgaa 1500
   tcatttcact tcgcaaactg ggatttgtac cttagaaaca tccattttaa tctaccttaa 1560
   cttcccagaa aaaaaaaaaa aaaaaaaaaa aaaaaaaaaa aaaaaaaaaa aaaaaaaaaa 1620
   aaaaaa                                                            1626
<210> 10
   <211> 372
   <212> PRT
   <213> Eucalyptus grandis
<400> 10
   Met ala Lys Ile Ala Pro Gly Cys Glu Pro Val Ala Gly Thr Leu Thr
    1               5                  10                  15
   Pro Ser Lys Lys Arg Glu Tyr Arg Val Thr Asn Arg Leu Gln Glu Gly
   20 25 30
   Lys Arg Pro Leu Tyr Ala Val Val Phe Asn Phe Ile Asp Ser Arg Tyr
   35 40 45
   Phe Asn Val Phe Ala Thr Val Gly Gly Asn Arg Val Thr Val Tyr Gln
   50 55 60
   Cys Leu Glu Gly Gly Val Ile Ala Val Leu Gln Ser Tyr Ile Asp Glu
   65 70 75 80
   Asp Lys Asp Glu Ser Phe Tyr Thr Val Ser Trp Ala Cys Asn Ile Asp
   85 90 95
   Arg Thr Pro Phe Val Val Ala Gly Gly Ile Asn Gly Ile Ile Arg Val
   100 105 110
   Ile Asp Ala Gly Asn Glu Lys Ile His Arg Ser Phe Val Gly His Gly
   115 120 125
   Asp Ser Ile Asn Glu Ile Arg Thr Gln Pro Leu Asn Pro Ser Leu Ile
   130 135 140
   Val Ser Ala Ser Lys Asp Glu Ser Val Arg Leu Trp Asn Val His Thr
   145 150 155 160
   Gly Ile Cys Ile Leu Ile Phe Ala Gly Ala Gly Gly His Arg Asn Glu
   165 170 175
   Val Leu Ser Val Asp Phe His Pro Ser Asp Lys Tyr Arg Ile Ala Ser
   180 185 190
   Cys Gly Met Asp Asn Thr Val Lys Ile Trp Ser Met Lys Glu Phe Trp
   195 200 205
   Thr Tyr Val Glu Lys Ser Phe Thr Trp Thr Asp Leu Pro Ser Lys Phe
   210 215 220
   Pro Thr Lys Tyr Val Gln Phe Pro Val Phe Ile Ala Pro Val His Ser
   225 230 235 240
   Asn Tyr Val Asp Cys Asn Arg Trp Leu Gly Asp Phe Val Leu Ser Lys
   245 250 255
   Ser Val Asp Asn Glu Ile Val Leu Trp Glu Pro Lys Met Lys Glu Gln
   260 265 270
   Ser Pro Gly Glu Gly Ser Val Asp Ile Leu Gln Lys Tyr Pro Val Pro
   275 280 285
   Glu Cys Asp Ile Trp Phe Ile Lys Phe Ser Cys Asp Phe His Tyr His
   290 295 300
   Ser Ile Ala Ile Gly Asn Arg Glu Gly Lys Ile Tyr Val Trp Glu Leu
   305 310 315 320
   Gln Ser Ser Pro Pro Val Leu Ile Ala Lys Leu Ser His Ser Gln Ser
   325 330 335
   Lys Ser Pro Ile Arg Gln Thr Ala Met Ser Phe Asp Gly Ser Thr Ile
   340 345 350
   Leu Ser Cys Cys Glu Asp Gly Thr Ile Trp Arg Trp Asp Ala Ile Thr
   355 360 365
   Ala Ser Thr Ser
   370
<210> 11
   <211> 620
   <212> DNA
   <213> Helianthus sp.
<400> 11
   ccacgcgtcc gcttggtgat ttcatactat ctaagagtgt agacaatgag ttcatattgt  60
   gggagccgaa gatgaaagag cagtctccag gagagggcac ggtggatatt cttcagaaat 120
   atcctgtacc tgattgtgac atctggttta taaagctttc ctgtgatttc cattacaatg 180
   cagcagctat tggtaacaga gaaggaaaaa tctatgtatg ggaattgcag actagcccgc 240
   cttctcttat tgcaaggtta tctcatattc agtccaaatc gccaatcagg caaactgcta 300
   tgtcatttga tggaagcaca attctgagtt gctgtgaaga tggcaccatc tggcgttggg 360
   atactgttgc aacgtcgtag cttgtgttgg tttgaaacaa gtcatgttgt gtaccatgta 420
   tattccttca gcaatttcgt ttgttttccg tggtgatgat gaggcatttt aatttgttct 480
   ttattaaact atgatagtag gatgtattcg tttagtgact ggccaacttg atatatgttt 540
   gtcggtgtta agcttttaaa aaaaaaaaaa aaaaaaaaaa aaaaaaaaaa aaaaaaaaaa 600
   aaaaaaaaaa aaaaaaaaag                                             620
<210> 12
   <211> 125
   <212> PRT
   <213> Helianthus sp.
<400> 12
   Thr Arg Pro Leu Gly Asp Phe Ile Leu Ser Lys Ser Val Asp Asn Glu
    1               5                  10                  15
   Phe Ile Leu Trp Glu Pro Lys Met Lys Glu Gln Ser Pro Gly Glu Gly
               20                  25                  30
   Thr Val Asp Ile Leu Gln Lys Tyr Pro Val Pro Asp Cys Asp Ile Trp
           35                  40                  45
   Phe Ile Lys Leu Ser Cys Asp Phe His Tyr Asn Ala Ala Ala Ile Gly
       50                  55                  60
   Asn Arg Glu Gly Lys Ile Tyr Val Trp Glu Leu Gln Thr Ser Pro Pro
   65                  70                  75                  80
   Ser Leu Ile Ala Arg Leu Ser His Ile Gln Ser Lys Ser Pro Ile Arg
                   85                  90                  95
   Gln Thr Ala Met Ser Phe Asp Gly Ser Thr Ile Leu Ser Cys Cys Glu
               100                 105                 110
   Asp Gly Thr Ile Trp Arg Trp Asp Thr Val Ala Thr Ser
           115                 120                 125
<210> 13
   <211> 1428
   <212> DNA
   <213> Catalpa speciosa
<400> 13
   gcacgagggc atacaggcgg tgctaatctg caggtaagga gatggcaaaa attccgttgg   60
   gttgtgagcc catggtgggt tccttaacgc cgtcgaagaa acgggagtat agggtcacca  120
   acaggctcca ggaaggcaaa cgccccattt acgccgtcgt tttcaacttc attgactccc  180
   gttacttcaa cgctttcgcc actgccggtg gcaatcgcgt gactgtatac cagtgcctag  240
   aaggtggtgt tatagctgta ctacagtcct acattgatga agataaagat gaatctttct  300
   acactgtaag ttgggcttgc aatattgatg gaactccatt cttggtggct ggaggactta  360
   atggaattat tcgagttatt gatactggca atgagaagat atacaagagt tttgtgggtc  420
   atggggattc aataaacgaa attcgaactc agccgctgaa accatcactt gttgtgtcag  480
   caagcaaaga tgaatctgta cgcctgtgga atattcatac tgggatatgc attttgatat  540
   tttctggtgc tggtggtcat cgcaatgaag ttcttagtgt ggacttccat ccttctgaca  600
   tctaccgtat tgcaagctgt ggaatggata acactgtcaa gatctggtca atgaaagaat  660
   tttggacata tgtagagaaa tcttttactt ggactgatct tccttctaag ttccccacaa  720
   aatatgtgca gttcccaata tttattgctt cagtgcatac gaactatgtt gattgcaacc  780
   ggtggattgg tgattttatg ctctccaaga gcgttgataa tgaactcgta ttatgggaac  840
   caaaaatgaa agaacagtct cctggagagg gtacagtcga cattcttcaa aagtatcctg  900
   ttcccgaatg cgatatttgg tttatcaaat tttcctgcga tttccattac aagacagcag  960
   cagtagggaa cagggaagga aagatatatg tatgggaagt gcaagccaac cccccggttc 1020
   tcattgcaag attatctcat attcagtcga aatctccaat tagattgact gccatgtcct 1080
   atgatgggag cacgattctc tgctgttgtg aagatggaac gatatggcga tgggatgtgg 1140
   tagcaagttc ttgagcttct ctaacacccg tttgatggtt atacttatac catgattgat 1200
   cacaaagctg taattgtact cacacaagct gcagcagaaa agcactgggt gctgcccttt 1260
   taacttattt caccagaata ttggttgtca ttgtaaaacg tatcaattgt cattcagttc 1320
   ttcgtttatt cgtaccttcc atcatttcta tggtctcttt tcttgttgat gtttcacagc 1380
   tcaccaaaca tgaaaaggta acagcgggta tagttgtgtt tccatctc             1428
<210> 14
   <211> 370
   <212> PRT
   <213> Catalpa speciosa
<400> 14
   Met ala Lys Ile Pro Leu Gly Cys Glu Pro Met Val Gly Ser Leu Thr
   1               5                   10                  15
   Pro Ser Lys Lys Arg Glu Tyr Arg Val Thr Asn Arg Leu Gln Glu Gly
               20                  25                  30
   Lys Arg Pro Ile Tyr Ala Val Val Phe Asn Phe Ile Asp Ser Arg Tyr
           35                  40                  45
   Phe Asn Ala Phe Ala Thr Ala Gly Gly Asn Arg Val Thr Val Tyr Gln
       50                  55                  60
   Cys Leu Glu Gly Gly Val Ile Ala Val Leu Gln Ser Tyr Ile Asp Glu
   65                  70                  75                  80
   Asp Lys Asp Glu Ser Phe Tyr Thr Val Ser Trp Ala Cys Asn Ile Asp
                   85                  90                  95
   Gly Thr Pro Phe Leu Val Ala Gly Gly Leu Asn Gly Ile Ile Arg Val
               100                 105                 110
   Ile Asp Thr Gly Asn Glu Lys Ile Tyr Lys Ser Phe Val Gly His Gly
           115                 120                 125
   Asp Ser Ile Asn Glu Ile Arg Thr Gln Pro Leu Lys Pro Ser Leu Val
       130                 135                 140
   Val Ser Ala Ser Lys Asp Glu Ser Val Arg Leu Trp Asn Ile His Thr
   145                 150                 155                 160
   Gly Ile Cys Ile Leu Ile Phe Ser Gly Ala Gly Gly His Arg Asn Glu
                   165                 170                 175
   Val Leu Ser Val Asp Phe His Pro Ser Asp Ile Tyr Arg Ile Ala Ser
               180                 185                 190
   Cys Gly Met Asp Asn Thr Val Lys Ile Trp Ser Met Lys Glu Phe Trp
           195                 200                 205
   Thr Tyr Val Glu Lys Ser Phe Thr Trp Thr Asp Leu Pro Ser Lys Phe
       210                 215                 220
   Pro Thr Lys Tyr Val Gln Phe Pro Ile Phe Ile Ala Ser Val His Thr
   225                 230                 235                 240
   Asn Tyr Val Asp Cys Asn Arg Trp Ile Gly Asp Phe Met Leu Ser Lys
                   245                 250                 255
   Ser Val Asp Asn Glu Leu Val Leu Trp Glu Pro Lys Met Lys Glu Gln
               260                 265                 270
   Ser Pro Gly Glu Gly Thr Val Asp Ile Leu Gln Lys Tyr Pro Val Pro
           275                 280                 285
   Glu Cys Asp Ile Trp Phe Ile Lys Phe Ser Cys Asp Phe His Tyr Lys
       290                 295                 300
   Thr Ala Ala Val Gly Asn Arg Glu Gly Lys Ile Tyr Val Trp Glu Val
   305                 310                 315                 320
   Gln Ala Asn Pro Pro Val Leu Ile Ala Arg Leu Ser His Ile Gln Ser
                   325                 330                 335
   Lys Ser Pro Ile Arg Leu Thr Ala Met Ser Tyr Asp Gly Ser Thr Ile
               340                 345                 350
   Leu Cys Cys Cys Glu Asp Gly Thr Ile Trp Arg Trp Asp Val Val Ala
           355                 360                 365
   Ser Ser
       370
<210> 15
   <211> 831
   <212> DNA
   <213> Zea mays
<220>
   <221> unsure
   <222> (578)
<220>
   <221> unsure
   <222> (611)
<220>
   <221> unsure
   <222> (630)
<220>
   <221> unsure
   <222> (641)
<220>
   <221> unsure
   <222> (660)
<220>
   <221> unsure
   <222> (682)
<220>
   <221> unsure
   <222> (685)
<220>
   <221> unsure
   <222> (703)
<220>
   <221> unsure
   <222> (743)
<220>
   <221> unsure
   <222> (752)
<220>
   <221> unsure
   <222> (761)
<220>
   <221> unsure
   <222> (784)
<220>
   <221> unsure
   <222> (794)
<220>
   <221> unsure
   <222> (802)
<220>
   <221> unsure
   <222> (825)
<400> 15
   tttagcacaa gcatcccaac tttcaacatt cgaagcattc aatattctgt taacaccaac  60
   cattttgact agaaaaaaca ccaggtggca ctttccacca acggattcaa ccatcgcaac 120
   cccaaccgag cgtaaccatc agtccagcat tgagcggcaa cacttcttca gtttctggag 180
   ctcggatggt ccacttcatc ccaccgccag atggtgccgt cttcaccagc tccaaggatt 240
   gtgcttccat cgaaggacac tgcagtttgt cttatcggcg atttacactg ctgattatac 300
   agccgagcaa tgaggacagg agggctggac tgtacttccc acacgtagat tttgccttca 360
   cggttgccta tcgccaactg attgaagtga aaatcacatg aaaatttgat aaaccaaatg 420
   tcacattctg ggacaggata cttctgaagg atatcgatgc ttccctcccc aggactctgt 480
   tcttttgtct tcggttccca aagcacaatt tcattgtcaa cactctttga taaggatgaa 540
   atcccaagcc atcttgttac aatcaacata gttagaantg tactgcagca atcaagaatg 600
   gaaactggac ntttttgttg gactctgccn aattccgcag nccgggggat catatttcan 660
   aacggcccca ccgcggtgaa cncanctttg ttcccttagt ganggttatt ccaactggct 720
   tatcaggtct accgttcccg tgnaatgtac cncccattcc ncactacaac cgaactaatt 780
   taancggggt ccanatgaca ancataatgc ttccccgccc tcatngaact t          831
<210> 16
   <211> 121
   <212> PRT
   <213> Zea mays
<400> 16
   Arg Trp Leu Gly Ile Ser Ser Leu Ser Lys Ser Val Asp Asn Glu Ile
   1               5                   10                  15
   Val Leu Trp Glu Pro Lys Thr Lys Glu Gln Ser Pro Gly Glu Gly Ser
               20                  25                  30
   Ile Asp Ile Leu Gln Lys Tyr Pro Val Pro Glu Cys Asp Ile Trp Phe
           35                  40                  45
   Ile Lys Phe Ser Cys Asp Phe His Phe Asn Gln Leu Ala Ile Gly Asn
       50                  55                  60
   Arg Glu Gly Lys Ile Tyr Val Trp Glu Val Gln Ser Ser Pro Pro Val
   65                  70                  75                  80
   Leu Ile Ala Arg Leu Tyr Asn Gln Gln Cys Lys Ser Pro Ile Arg Gln
                   85                  90                  95
   Thr Ala Val Ser Phe Asp Gly Ser Thr Ile Leu Gly Ala Gly Glu Asp
               100                 105                 110
   Gly Thr Ile Trp Arg Trp Asp Glu Val
           115                 120
<210> 17
   <211> 1486
   <212> DNA
   <213> Zea mays
<400> 17
   ccytctagat gcatgctcga gcggccgcca gtgtgatgga tatctgcaga attcgccctt   60
   gccgctctag aactagtgga tcccccgggc ctgcaggaat tcggcacgag ccggaagcgg  120
   gagtacaagc ctgcggcaag cacactgagg ggaagcgccc gctatatgct atcgggttca  180
   acttcatgga cgcgcgctac tacgacgtct tcgccaccgt cggcggcaac cgcgtgacaa  240
   cttatcgctg ccttgagaat ggtagtttcg ctcttctaca agcttacgtt gatgaggata  300
   aggatgagtc gttctatact ctaagctggg ctcgtgacca tgttgatggc tcaccactgc  360
   tggtggcagc aggaagcaat gggatcattc gggtcatcaa ttgtgctaca gaaaagttag  420
   ctaagagctt tgttggccat ggcgactcaa taaatgagat aagaactcaa ccgttgaagc  480
   cttcgctcat catttctgca agcaaggatg aatctgttag gctatggaat gtccatacag  540
   ggatctgtat cttgatattt gctggagctg gaggtcatcg caatgaagta ttgagtgttg  600
   acttccatcc tagtgatatt gaacgttttg caagttgtgg catggacaac actgtgaaaa  660
   tctggtcaat gaaagaattt tggctatatg ttgacaaatc atattcatgg actgaccttc  720
   catcaaagtt tccaacaaaa tatgtccagt ttccagtctt gattgctgca gtacactcta  780
   actatgttga ttgtacaaga tggcttggtg acttcatcct atcaaagagt gttgacaatg  840
   aaattgtgct ttgggaaccg aagacaaaag aacagatcct gggggaggga agcatcgata  900
   tccttcagaa gtatcctgtc ccagaatgtg acatttggtt tatcaaattt tcatgtgatt  960
   ttcacttcaa tcagttggcg ataggcaacc gtgaaggcaa aatctacgtg tgggaagtac 1020
   agtcagccct cctgtcctca ttgctcggct gtataatcag cagtgtaaat cgccgataag 1080
   acaaactgca gtgtccttcg atggaagcac aatccttgga gctggtgaag acggcaccat 1140
   ctggcggtgg gatgaagtgg accatccgag ctccagaaac tgaagaagtg ttgccgctca 1200
   atgctggact gatggttacg ctcggttggg gttgcgatgg ttgaatccgt tggtggaaag 1260
   tgccacctgg tgttttttct agtcaaaatg gttggtgtta acagaatatt gaatgcttcg 1320
   aatgttgaaa gttgggatgc ttgtgctggt actctgctcc gtggacgagt gaacttaggt 1380
   gccgtttggt tcacatattt gtaacgtaat gggtaacaga taacgttaaa tcatgtttgt 1440
   tttatttcaa ccgtaatcag ataccacatt aaaattaaaa aaaaaa                1486
<210> 18
   <211> 391
   <212> PRT
   <213> Zea mays
<400> 18
   Met His Ala Arg Ala Ala Ala Ser Val Met Asp Ile Cys Arg Ile Arg
   1 5 10 15
   Pro Cys Arg Ser Arg Thr Ser Gly Ser Pro Gly Pro Ala Gly Ile Arg
   20 25 30
   His Glu Pro Glu Ala Gly Val Gln Ala Cys Gly Lys His Thr Glu Gly
   35 40 45
   Lys Arg Pro Leu Tyr Ala Ile Gly Phe Asn Phe Met Asp Ala Arg Tyr
   50 55 60
   Tyr Asp Val Phe Ala Thr Val Gly Gly Asn Arg Val Thr Thr Tyr Arg
   65 70 75 80
   Cys Leu Glu Asn Gly Ser Phe Ala Leu Leu Gln Ala Tyr Val Asp Glu
   85 90 95
   Asp Lys Asp Glu Ser Phe Tyr Thr Leu Ser Trp Ala Arg Asp His Val
   100 105 110
   Asp Gly Ser Pro Leu Leu Val Ala Ala Gly Ser Asn Gly Ile Ile Arg
   115 120 125
   Val Ile Asn Cys Ala Thr Glu Lys Leu Ala Lys Ser Phe Val Gly His
   130 135 140
   Gly Asp Ser Ile Asn Glu Ile Arg Thr Gln Pro Leu Lys Pro Ser Leu
   145 150 155 160
   Ile Ile Ser Ala Ser Lys Asp Glu Ser Val Arg Leu Trp Asn Val His
   165 170 175
   Thr Gly Ile Cys Ile Leu Ile Phe Ala Gly Ala Gly Gly His Arg Asn
   180 185 190
   Glu Val Leu Ser Val Asp Phe His Pro Ser Asp Ile Glu Arg Phe Ala
           195                 200                 205
   Ser Cys Gly Met Asp Asn Thr Val Lys Ile Trp Ser Met Lys Glu Phe
       210                 215                 220
   Trp Leu Tyr Val Asp Lys Ser Tyr Ser Trp Thr Asp Leu Pro Ser Lys
   225                 230                 235                     240
   Phe Pro Thr Lys Tyr Val Gln Phe Pro Val Leu Ile Ala Ala Val His
                   245                 250                 255
   Ser Asn Tyr Val Asp Cys Thr Arg Trp Leu Gly Asp Phe Ile Leu Ser
               260                 265                 270
   Lys Ser Val Asp Asn Glu Ile Val Leu Trp Glu Pro Lys Thr Lys Glu
           275                 280                 285
   Gln Ile Leu Gly Glu Gly Ser Ile Asp Ile Leu Gln Lys Tyr Pro Val
       290                 295                 300
   Pro Glu Cys Asp Ile Trp Phe Ile Lys Phe Ser Cys Asp Phe His Phe
   305                 310                 315                     320
   Asn Gln Leu Ala Ile Gly Asn Arg Glu Gly Lys Ile Tyr Val Trp Glu
                   325                 330                 335
   Val Gln Ser Ser Pro Pro Val Leu Ile Ala Arg Leu Tyr Asn Gln Gln
               340                 345                 350
   Cys Lys Ser Pro Ile Arg Gln Thr Ala Val Ser Phe Asp Gly Ser Thr
           355                 360                 365
   Ile Leu Gly Ala Gly Glu Asp Gly Thr Ile Trp Arg Trp Asp Glu Val
       370                 375                 380
   Asp His Pro Ser Ser Arg Asn
   385                 390
<210> 19
   <211> 1104
   <212> DNA
   <213> Zea mays
<400> 19
   ccacgcgtcc ggaagcaatg ggatcattcg ggtcatcaat tgtgctacag aaaagttagc   60
   taagagcttt gttggccatg gcgactcaat aaatgagata agaactcaac cgttgaagcc  120
   ttcgctcatc atttctgcaa gcaaggatga atctgttagg ctatggaatg tccatacagg  180
   gatctgtatc ttgatatttg ctggagctgg aggtcatcgc aatgaagtat tgagtgttga  240
   cttccatcct agtgatattg aacgttttgc aagttgtggc atggacaaca ctgtgaaaat  300
   ctggtcaatg aaagaatttt ggctatatgt tgacaaatca tattcatgga ctgaccttcc  360
   atcaaagttt ccaacaaaat atgtccagtt tccagtcttg attgctgcag tacactctaa  420
   ctatgttgat tgtacaagat ggcttggtga cttcatccta tcaaagagtg ttgacaatga  480
   aattgtgctt tgggaaccga agacaaaaga acagagtcct ggggagggaa gcatcgatat  540
   ccttcagaag tatcctgtcc cagaatgtga catttggttt atcaaatttt catgtgattt  600
   tcacttcaat cagttggcga taggcaaccg tgaaggcaaa atctacgtgt gggaagtaca  660
   gtccagccct cctgtcctca ttgctcggct gtataatcag cagtgtaaat cgccgataag  720
   acaaactgca gtgtccttcg atggaagcac aatccttgga gctggtgaag acggcaccat  780
   ctggcggtgg gatgaagtgg accatccgag ctccagaaac tgaagaagtg ttgccgctca  840
   atgctggact gatggttacg ctcggttggg gttgcgatgg ttgaatccgt tggtggaaag  900
   tgccacctgg tgttttttct agtcaaaatg gttggtgtta acagaatatt gaatgcttcg  960
   aatgttgaaa gttgggatgc ttgtgctggt actctgctcc gtggacgagt gaacttaggt 1020
   gccgtttggt tcacatattt gtaacgtaat gggtaacaga taacgttaaa tcatgtttgt 1080
   tttatttcaa aaaaaaaaaa aaag                                        1104
<210> 20
   <211> 273
   <212> PRT
   <213> Zea mays
<400> 20
   His Ala Ser Gly Ser Asn Gly Ile Ile Arg Val Ile Asn Cys Ala Thr
    1               5                  10                  15
   Glu Lys Leu Ala Lys Ser Phe Val Gly His Gly Asp Ser Ile Asn Glu
               20                  25                  30
   Ile Arg Thr Gln Pro Leu Lys Pro Ser Leu Ile Ile Ser Ala Ser Lys
           35                  40                  45
   Asp Glu Ser Val Arg Leu Trp Asn Val His Thr Gly Ile Cys Ile Leu
       50                  55                  60
   Ile Phe Ala Gly Ala Gly Gly His Arg Asn Glu Val Leu Ser Val Asp
   65                  70                  75                  80
   Phe His Pro Ser Asp Ile Glu Arg Phe Ala Ser Cys Gly Met Asp Asn
                   85                  90                  95
   Thr Val Lys Ile Trp Ser Met Lys Glu Phe Trp Leu Tyr Val Asp Lys
               100                 105                 110
   Ser Tyr Ser Trp Thr Asp Leu Pro Ser Lys Phe Pro Thr Lys Tyr Val
           115                 120                 125
   Gln Phe Pro Val Leu Ile Ala Ala Val His Ser Asn Tyr Val Asp Cys
       130                 135                 140
   Thr Arg Trp Leu Gly Asp Phe Ile Leu Ser Lys Ser Val Asp Asn Glu
   145                 150                 155                 160
   Ile Val Leu Trp Glu Pro Lys Thr Lys Glu Gln Ser Pro Gly Glu Gly
                   165                 170                 175
   Ser Ile Asp Ile Leu Gln Lys Tyr Pro Val Pro Glu Cys Asp Ile Trp
               180                 185                 190
   Phe Ile Lys Phe Ser Cys Asp Phe His Phe Asn Gln Leu Ala Ile Gly
           195                 200                 205
   Asn Arg Glu Gly Lys Ile Tyr Val Trp Glu Val Gln Ser Ser Pro Pro
       210                 215                 220
   Val Leu Ile Ala Arg Leu Tyr Asn Gln Gln Cys Lys Ser Pro Ile Arg
   225                 230                 235                 240
   Gln Thr Ala Val Ser Phe Asp Gly Ser Thr Ile Leu Gly Ala Gly Glu
                   245                 250                 255
   Asp Gly Thr Ile Trp Arg Trp Asp Glu Val Asp His Pro Ser Ser Arg
               260                 265                 270
   Asn
   273
<210> 21
   <211> 488
   <212> DNA
   <213> Zea mays
<400> 21
   ccacgcgtcc gggcaaaatc tacgtgtggg aagtacagtc cagccctcct gtcctcattg  60
   ctcggctgta taatcagcag tgtaaatcgc cgataagaca aactgcagtg tccttcgatg 120
   gaagcacaat ccttggagct ggtgaagacg gcaccatctg gcggtgggat gaagtggacc 180
   atccgagctc cagaaactga agaagtgttg ccgctcaatg ctggactgat ggttacgctc 240
   ggttggggtt gcgatggttg aatccgttgg tggaaagtgc cacctggtgt tttttctagt 300
   caaaatggtt ggtgttaaca gaatattgaa tgcttcgaat gttgaaagtt gggatgcttg 360
   tgctggtact ctgctccgtg gacgagtgaa cttaggtgcc gtttggttca catatttgta 420
   acgtaatggg taacagataa cgttaaatca tgtttgtttt atttcaaccg taaaaaaaaa 480
   aaaaaaag                                                          488
<210> 22
   <211> 65
   <212> PRT
   <213> Zea mays
<400> 22
   Thr Arg Pro Gly Lys Ile Tyr Val Trp Glu Val Gln Ser Ser Pro Pro
   1               5                   10                  15
   Val Leu Ile Ala Arg Leu Tyr Asn Gln Gln Cys Lys Ser Pro Ile Arg
               20                  25                  30
   Gln Thr Ala Val Ser Phe Asp Gly Ser Thr Ile Leu Gly Ala Gly Glu
           35                  40                  45
   Asp Gly Thr Ile Trp Arg Trp Asp Glu Val Asp His Pro Ser Ser Arg
       50                  55                  60
   Asn
   65
<210> 23
   <211> 1763
   <212> DNA
   <213> Zea mays
<400> 23
   ccacgcgtcc gcaaaatgtg catcgccgcc gccaccatat agaaccactt atcatgaacc  60
   gccgccatca catccactgc ctcaactagt gttaccacct atggttcatt gttgtgtctg 120
   cttcttgtag cactgttggt ctacaaacat tcatatttct ctcaacatct ggcacagcat 180
   gccgccttcc aaagcacgcc gaaagaggtc acttcgtgat atcactgcca ccgttgccac 240
   tgggactgtt gccaactcga aacctggctc atcatcgacg aacgagggga agcaacagga 300
   caagaaaaag gagggtccac aggaaccgga catcccacca ttaccgccgg tggtggtgaa 360
   tatagtccca cgacaaggat taggatgtga agtagtggaa gggctactcg tgcctagtcg 420
   gaagcgagag tacaagccca atagcaagta tactgtggga aatcacccga tctatgccat  480
   cgggttcaat ttcattgaca tgcgctacta tgatgtcttt gccatcgcca gttgcaatag  540
   tgtgataatt taccgatgcc ttgagaatgg tggttttggt cttctacaaa attatgttga  600
   tgaggataag gatgagtcat tctacactct aagctggacc atcgatcaag ttgatagctc  660
   accgctgttg gtggccgcag gaagcaatcg gatcattcgg gtcatcaatt gtgctaccga  720
   aaagttagat aagagcttag ttggccatgg tggttcaata catgagataa ggactcatgc  780
   ctcgaagcca tcactcatca tttctgccag caaggacttc caccctaccg aggttgggat  840
   ttttgcaagt tgtggcatgg acaatactgt gaaaatttgg tcaatgaaag aattttggat  900
   atatgttgaa aaatcatatt catggactgg ccatccatca aagtttccaa cgaggaatat  960
   ccagtttccg gtcttgactg ctgcagtaca ctctgactat gttgattgta caagatggct 1020
   tggtgacttc atcctatcaa agagtgtaaa gaatgcagtt ttgctttggg aaccaaaacc 1080
   agacaagcgt aggcctgggg aggggagtgt tgatgttctt cagaagtacc cggtgccaaa 1140
   gtgttcattt atggtttatg aaattttcat gtgattttta ctccaaccag atggcaatag 1200
   gcaacaataa aggcgagatc tatgtctggg aagtgcagtc cagcccgccc gtcttaattg 1260
   accggctgtg caaccaggaa tgcaagtcgc cgataaggca gaccgcagtg tcattcgacg 1320
   gaagcacgat ccttggagcc gccgacgacg gcgcgatctg gcggtgggac gaagtggacc 1380
   ctgctgcttc cagctccaaa cctgatcaag ctgctgcgcc cgccgccggt gtcggtgccg 1440
   gtgccggtgc cgacgccgac gccgacgcct gagcgagagg accgtcgccg cccgccggtt 1500
   cacatcgatc gtactccgtg ctggttgatt agctttaccc attggtatgt tttggttcag 1560
   agtcgccaga tctagtgtgt ggctgaacgt tgaatgttag gatgctgctg tttgttatgc 1620
   tctgagtctt gagttcactt tgttaatttg caccgtggat gagatgaata acttgacgtt 1680
   gcaactttgc atcccatata tgccgtaaat ctgccgtctg ttgtttgtaa aaaaaaaaaa 1740
   aaaaaaaaaa aaaaaaaaaa aag                                         1763
<210> 24
   <211> 430
   <212> PRT
   <213> Zea mays
<400> 24
   Met Pro Pro Ser Lys Ala Arg Arg Lys Arg Ser Leu Arg Asp Ile Thr
    1               5                  10                  15
   Ala Thr Val Ala Thr Gly Thr Val Ala Asn Ser Lys Pro Gly Ser Ser
               20                  25                  30
   Ser Thr Asn Glu Gly Lys Gln Gln Asp Lys Lys Lys Glu Gly Pro Gln
           35                  40                  45
   Glu Pro Asp Ile Pro Pro Leu Pro Pro Val Val Val Asn Ile Val Pro
       50                  55                  60
   Arg Gln Gly Leu Gly Cys Glu Val Val Glu Gly Leu Leu Val Pro Ser
   65                  70                  75                  80
   Arg Lys Arg Glu Tyr Lys Pro Asn Ser Lys Tyr Thr Val Gly Asn His
                   85                  90                  95
   Pro Ile Tyr Ala Ile Gly Phe Asn Phe Ile Asp Met Arg Tyr Tyr Asp
               100                 105                 110
   Val Phe Ala Ile Ala Ser Cys Asn Ser Val Ile Ile Tyr Arg Cys Leu
           115                 120                 125
   Glu Asn Gly Gly Phe Gly Leu Leu Gln Asn Tyr Val Asp Glu Asp Lys
       130                 135                 140
   Asp Glu Ser Phe Tyr Thr Leu Ser Trp Thr Ile Asp Gln Val Asp Ser
   145                 150                 155                 160
   Ser Pro Leu Leu Val Ala Ala Gly Ser Asn Arg Ile Ile Arg Val Ile
                   165                 170                 175
   Asn Cys Ala Thr Glu Lys Leu Asp Lys Ser Leu Val Gly His Gly Gly
               180                 185                 190
   Ser Ile His Glu Ile Arg Thr His Ala Ser Lys Pro Ser Leu Ile Ile
           195                 200                 205
   Ser Ala Ser Lys Asp Phe His Pro Thr Glu Val Gly Ile Phe Ala Ser
       210                 215                 220
   Cys Gly Met Asp Asn Thr Val Lys Ile Trp Ser Met Lys Glu Phe Trp
   225                 230                 235                 240
   Ile Tyr Val Glu Lys Ser Tyr Ser Trp Thr Gly His Pro Ser Lys Phe
                   245                 250                 255
   Pro Thr Arg Asn Ile Gln Phe Pro Val Leu Thr Ala Ala Val His Ser
               260                 265                 270
   Asp Tyr Val Asp Cys Thr Arg Trp Leu Gly Asp Phe Ile Leu Ser Lys
           275                 280                 285
   Ser Val Lys Asn Ala Val Leu Leu Trp Glu Pro Lys Pro Asp Lys Arg
       290                 295                 300
   Arg Pro Gly Glu Gly Ser Val Asp Val Leu Gln Lys Tyr Pro Val Pro
   305                 310                 315                 320
   Lys Cys His Leu Trp Phe Met Lys Phe Ser Cys Asp Phe Tyr Ser Asn
                   325                 330                 335
   Gln Met ala Ile Gly Asn Asn Lys Gly Glu Ile Tyr Val Trp Glu Val
               340                 345                 350
   Gln Ser Ser Pro Pro Val Leu Ile Asp Arg Leu Cys Asn Gln Glu Cys
           355                 360                 365
   Lys Ser Pro Ile Arg Gln Thr Ala Val Ser Phe Asp Gly Ser Thr Ile
       370                 375                 380
   Leu Gly Ala Ala Asp Asp Gly Ala Ile Trp Arg Trp Asp Glu Val Asp
   385                 390                 395                 400
   Pro Ala Ala Ser Ser Ser Lys Pro Asp Gln Ala Ala Ala Pro Ala Ala
                   405                 410                 415
   Gly Val Gly Ala Gly Ala Gly Ala Asp Ala Asp Ala Asp Ala
               420                 425                 430
<210> 25
   <211> 1803
   <212> DNA
   <213> Zea mays
<220>
   <221> unsure
   <222> (1729)
<220>
   <221> unsure
   <222> (1752)
<220>
   <221> unsure
   <222> (1760)
<220>
   <221> unsure
   <222> (1765)
<400> 25
   gcacgaggct tttgccccgc accgctttcc tacgcttgcc caaacccaca aaaccctggc   60
   cgatcgcgcc gcggaaatgc ctttccggcc gccgcgagcc cgcgacacta gtaacggtct  120
   acaccactag aatgactgaa gaatttgaat tccagcaaaa ttcaagcttt tgttttaagc  180
   caagattttg agatttcgat ttgaagtgtg gaagtcctta caattttgcc aattcctata  240
   tttgatctct gctgtgctgc gttaaatccc taaactttca cagcgcggcg ccgggcccag  300
   ccacgccgga agaagtcgcc gcgtgaggtc agtgtccccg ttgctgccgc ctctaacccg  360
   aagcctaggc cgctgccggt gcataacaag gagaatcagg cggaggggaa agtagcagag  420
   gagggggcag caactgagga gggggagaag taccgggcgg aaccggaaat cttgccgctg  480
   ccgccggcca tggcgaagct gggcccgggg caggggctcg ggtgcgaggc ggcggagggg  540
   tcgctcgtgc ccagccggaa gcgggagtac aagccctgcg gcaagcacac tgaggggaag  600
   cgcccgctat atgctatcgg gttcaacttc atggacgcgc gctactacga cgtcttcgcc  660
   accgtcggcg gcaaccgcgt gacaacttac cgctgccttg agaatggtag tttcgctctt  720
   ctacaagctt acgttgatga ggataaggat gagtcgttct atactctaag ctgggctcgt  780
   gaccatgttg atggctcacc actgctggtg gcagcaggaa gcaatgggat cattcgggtc  840
   atcaattgtg ctacagaaaa gttagctaag agctttgttg gccatggcga ctcaataaat  900
   gagataagaa ctcaaccgtt gaagccttcg ctcatcattt ctgcaagcaa ggatgaatct  960
   gttaggctat ggaatgtcca tacagggatc tgtatcttga tatttgctgg agctggaggt 1020
   catcgcaatg aagtattgag tgttgacttc catcctagtg atattgaacg ttttgcaagt 1080
   tgtggcatgg acaacactgt gaaaatctgg tcaatgaaag aattttggct atatgttgac 1140
   aaatcatatt catggactga ccttccatca aagtttccaa caaaatatgt ccagtttcca 1200
   gtcttgattg ctgcagtaca ctctaactat gttgattgta caagatggct tggtgacttc 1260
   atcctatcaa agagtgttga caatgaattg tgcttttggg aaccgaagac aaaagaacag 1320
   agtcctgggg agggaagcat cgatatcctt cagaagtatc ctgtcccaga atgtgacatt 1380
   tggtttatca aattttcatg tgattttcac ttcaatcagt tggcgatagg caaccgtgaa 1440
   ggcaaaatct acgtgtggga agtacagtcc agccctcctg tcctcattgc tcggctgtat 1500
   aatcagcagt gtaaatcgcc gataagacaa actgcagtgt ccttcgatgg aagcacaatc 1560
   cttggagctg gtgaagacgg caccatctgg cggtgggatg aagtggacca tccgagctcc 1620
   agaaactgaa gaagtgttgc cgctcaatgc tggactgatg gttacgctcg gttggggttg 1680
   cgatggttga atccgttggt ggaaagtgcc acctgggtgt tttttctant caaaatgggt 1740
   tggtgttaac anaatattgn aatgnttcca aatgttgaaa aatttgggat gcttgtgcct 1800
   ggt                                                               1803
<210> 26
   <211> 379
   <212> PRT
   <213> Zea mays
<400> 26
   Met ala Lys Leu Gly Pro Gly Gln Gly Leu Gly Cys Glu Ala Ala Glu
    1               5                  10                  15
   Gly Ser Leu Val Pro Ser Arg Lys Arg Glu Tyr Lys Pro Cys Gly Lys
               20                  25                  30
   His Thr Glu Gly Lys Arg Pro Leu Tyr Ala Ile Gly Phe Asn Phe Met
           35                  40                  45
   Asp Ala Arg Tyr Tyr Asp Val Phe Ala Thr Val Gly Gly Asn Arg Val
       50                  55                  60
   Thr Thr Tyr Arg Cys Leu Glu Asn Gly Ser Phe Ala Leu Leu Gln Ala
   65                  70                  75                  80
   Tyr Val Asp Glu Asp Lys Asp Glu Ser Phe Tyr Thr Leu Ser Trp Ala
                   85                  90                  95
   Arg Asp His Val Asp Gly Ser Pro Leu Leu Val Ala Ala Gly Ser Asn
               100                 105                 110
   Gly Ile Ile Arg Val Ile Asn Cys Ala Thr Glu Lys Leu Ala Lys Ser
           115                 120                 125
   Phe Val Gly His Gly Asp Ser Ile Asn Glu Ile Arg Thr Gln Pro Leu
       130                 135                 140
   Lys Pro Ser Leu Ile Ile Ser Ala Ser Lys Asp Glu Ser Val Arg Leu
   145                 150                 155                 160
   Trp Asn Val His Thr Gly Ile Cys Ile Leu Ile Phe Ala Gly Ala Gly
                   165                 170                 175
   Gly His Arg Asn Glu Val Leu Ser Val Asp Phe His Pro Ser Asp Ile
               180                 185                 190
   Glu Arg Phe Ala Ser Cys Gly Met Asp Asn Thr Val Lys Ile Trp Ser
           195                 200                 205
   Met Lys Glu Phe Trp Leu Tyr Val Asp Lys Ser Tyr Ser Trp Thr Asp
       210                 215                 220
   Leu Pro Ser Lys Phe Pro Thr Lys Tyr Val Gln Phe Pro Val Leu Ile
   225                 230                 235                 240
   Ala Ala Val His Ser Asn Tyr Val Asp Cys Thr Arg Trp Leu Gly Asp
                   245                 250                 255
   Phe Ile Leu Ser Lys Ser Val Asp Asn Glu Leu Cys Phe Trp Glu Pro
               260                 265                 270
   Lys Thr Lys Glu Gln Ser Pro Gly Glu Gly Ser Ile Asp Ile Leu Gln
           275                 280                 285
   Lys Tyr Pro Val Pro Glu Cys Asp Ile Trp Phe Ile Lys Phe Ser Cys
       290                 295                 300
   Asp Phe His Phe Asn Gln Leu Ala Ile Gly Asn Arg Glu Gly Lys Ile
   305                 310                 315                 320
   Tyr Val Trp Glu Val Gln Ser Ser Pro Pro Val Leu Ile Ala Arg Leu
                   325                 330                 335
   Tyr Asn Gln Gln Cys Lys Ser Pro Ile Arg Gln Thr Ala Val Ser Phe
   340 345 350
   Asp Gly Ser Thr Ile Leu Gly Ala Gly Glu Asp Gly Thr Ile Trp Arg
   355 360 365
   Trp Asp Glu Val Asp His Pro Ser Ser Arg Asn
   370 375
<210> 27
   <211> 1642
   <212> DNA
   <213> Zea mays
<400> 27
   tccacgcgtc cgcaacatct ggcacagcat gccgccttcc aaagcacgcc gaaagaggtc   60
   acttcgtgat atcactgcca ccgttgccac tgggactgtt gccaactcga aacctggctc  120
   atcatcgacg aacgagggga agcaacagga caagaaaaag gagggtccac aggaaccgga  180
   catcccacca ttaccgccgg tggtggtgaa tatagtccca cgacaaggat taggatgtga  240
   agtagtggaa gggctactcg tgcctagtcg gaagcgagag tacaagccca atagcaagta  300
   tactgtggga aatcacccga tctatgccat cgggttcaat ttcattgaca tgcgctacta  360
   tgatgtcttt gccatcgcca gttgcaatag tgtgataatt taccgatgcc ttgagaatgg  420
   tggttttggt cttctacaaa attatgttga tgaggataag gatgagtcat tctacactct  480
   aagctggacc atcgatcaag ttgatagctc accgctgttg gtggccgcag gaagcaatcg  540
   gatcattcgg gtcatcaatt gtgctaccga aaagttagat aagagcttag ttggccatgg  600
   tggttcaata catgagataa ggactcatgc ctcgaagcca tcactcatca tttctgccag  660
   caaggatgaa tctattaggc tatggaatgt ccatactggg atttgcatct tagtctttgc  720
   aggggctgga ggccatcgac atgatgtgtt gagtgttgac ttccacccta ccgaggttgg  780
   gatttttgca agttgtggca tggacaatac tgtgaaaatt tggtcaatga aagaattttg  840
   gatatatgtt gaaaaatcat attcatggac tggccatcca tcaaagtttc caacgaggaa  900
   tatccagttt ccggtcttga ctgctgcagt acactctgac tatgttgatt gtacaagatg  960
   gcttggtgac ttcatcctat caaagagtgt aaagaatgca gttttgcttt gggaaccaaa 1020
   accagacaag cgtaggcctg gggaggggag tgttgatgtt cttcagaagt acccggtgcc 1080
   aaagtgttca ttatggttta tgaaattttc atgtgatttt tactccaacc agatggcaat 1140
   aggcaacaat aaaggcgaga tctatgtctg ggaagtgcag tccagcccgc ccgtcttaat 1200
   tgaccggctg tgcaaccagg aatgcaagtc gccgataagg cagaccgcag tgtcattcga 1260
   cggaagcacg atccttggag ccgccgacga cggcgcgatc tggcggtggg acgaagtgga 1320
   ccctgctgct tccagctcca aacctgatca agctgctgcg cccgccgccg gtgtcggtgc 1380
   cggtgccggt gccgacgccg acgccgacgc ctgagcgaga ggaccgtcgc cgcccgccgg 1440
   ttcacatcga tcgtactccg tgctggttga ttagctttac ccattggtat gttttggttc 1500
   agagtcgcca gatctagtgt gtggctgaac gttgaatgtt aggatgctgc tgtttgttat 1560
   gctctgagtc ttgagttcac tttgttaatt tgcaccgtgg atgagatgaa taacttgacg 1620
   ttgcaaaaaa aaaaaaaaaa ag                                           1642
<210> 28
   <211> 461
   <212> PRT
   <213> Zea mays
<400> 28
   Met Pro Pro Ser Lys Ala Arg Arg Lys Arg Ser Leu Arg Asp Ile Thr
   1 5 10 15
   Ala Thr Val Ala Thr Gly Thr Val Ala Asn Ser Lys Pro Gly Ser Ser
   20 25 30
   Ser Thr Asn Glu Gly Lys Gln Gln Asp Lys Lys Lys Glu Gly Pro Gln
   35 40 45
   Glu Pro Asp Ile Pro Pro Leu Pro Pro Val Val Val Asn Ile Val Pro
       50                  55                  60
   Arg Gln Gly Leu Gly Cys Glu Val Val Glu Gly Leu Leu Val Pro Ser
   65                  70                  75                  80
   Arg Lys Arg Glu Tyr Lys Pro Asn Ser Lys Tyr Thr Val Gly Asn His
                   85                  90                  95
   Pro Ile Tyr Ala Ile Gly Phe Asn Phe Ile Asp Met Arg Tyr Tyr Asp
               100                 105                 110
   Val Phe Ala Ile Ala Ser Cys Asn Ser Val Ile Ile Tyr Arg Cys Leu
           115                 120                 125
   Glu Asn Gly Gly Phe Gly Leu Leu Gln Asn Tyr Val Asp Glu Asp Lys
       130                 135                 140
   Asp Glu Ser Phe Tyr Thr Leu Ser Trp Thr Ile Asp Gln Val Asp Ser
   145                 150                 155                 160
   Ser Pro Leu Leu Val Ala Ala Gly Ser Asn Arg Ile Ile Arg Val Ile
                   165                 170                 175
   Asn Cys Ala Thr Glu Lys Leu Asp Lys Ser Leu Val Gly His Gly Gly
               180                 185                 190
   Ser Ile His Glu Ile Arg Thr His Ala Ser Lys Pro Ser Leu Ile Ile
           195                 200                 205
   Ser Ala Ser Lys Asp Glu Ser Ile Arg Leu Trp Asn Val His Thr Gly
       210                 215                 220
   Ile Cys Ile Leu Val Phe Ala Gly Ala Gly Gly His Arg His Asp Val
   225                 230                 235                 240
   Leu Ser Val Asp Phe His Pro Thr Glu Val Gly Ile Phe Ala Ser Cys
                   245                 250                 255
   Gly Met Asp Asn Thr Val Lys Ile Trp Ser Met Lys Glu Phe Trp Ile
               260                 265                 270
   Tyr Val Glu Lys Ser Tyr Ser Trp Thr Gly His Pro Ser Lys Phe Pro
           275                 280                 285
   Thr Arg Asn Ile Gln Phe Pro Val Leu Thr Ala Ala Val His Ser Asp
       290                 295                 300
   Tyr Val Asp Cys Thr Arg Trp Leu Gly Asp Phe Ile Leu Ser Lys Ser
   305                 310                 315                 320
   Val Lys Asn Ala Val Leu Leu Trp Glu Pro Lys Pro Asp Lys Arg Arg
                   325                 330                 335
   Pro Gly Glu Gly Ser Val Asp Val Leu Gln Lys Tyr Pro Val Pro Lys
               340                 345                 350
   Cys Ser Leu Trp Phe Met Lys Phe Ser Cys Asp Phe Tyr Ser Asn Gln
           355                 360                 365
   Met ala Ile Gly Asn Asn Lys Gly Glu Ile Tyr Val Trp Glu Val Gln
       370                 375                 380
   Ser Ser Pro Pro Val Leu Ile Asp Arg Leu Cys Asn Gln Glu Cys Lys
   385                 390                 395                 400
   Ser Pro Ile Arg Gln Thr Ala Val Ser Phe Asp Gly Ser Thr Ile Leu
                   405                 410                 415
   Gly Ala Ala Asp Asp Gly Ala Ile Trp Arg Trp Asp Glu Val Asp Pro
               420                 425                 430
   Ala Ala Ser Ser Ser Lys Pro Asp Gln Ala Ala Ala Pro Ala Ala Gly
           435                 440                 445
   Val Gly Ala Gly Ala Gly Ala Asp Ala Asp Ala Asp Ala
       450                 455                 460
<210> 29
   <211> 1686
   <212> DNA
   <213> Zea mays
<400> 29
   gccgccgcga gcccgcgaca ctagtaacgg tctacaccac tagaatgact gaagaattga   60
   attccagcaa attcaagctt ttgttttagc caagatttga gattcgattt gaagtgtgga  120
   agtccttaca atttgccaat cctatatttg atctctgctg tgctgcgtta aatccctaaa  180
   cttcacagcg cggcgccggc ccagccacgc cggaagaagt cgccgcgtga ggtcagtgtc  240
   cccgttgctg ccgcctctaa cccgaagcct aggccgctgc cggtgcataa caaggagaat  300
   caggcggagg ggaaagtagc agaggagggg gcagcaactg aggaggggga gaagtaccgg  360
   gcggaaccgg aaatcttgcc gctgccgccg gccatggcga agctgggccc ggggcagggg  420
   ctcgggtgcg aggcggcgga ggggtcgctc gtgcccagcc ggaagcggga gtacaagccc  480
   tgcggcaagc acactgaggg gaagcgcccg ctatatgcta tcgggttcaa cttcatggac  540
   gcgcgctact acgacgtctt cgccaccgtc ggcggcaacc gcgtgacaac ttaccgctgc  600
   cttgagaatg gtagtttcgc tcttctacaa gcttacgttg atgaggataa ggatgagtcg  660
   ttctatactc taagctgggc tcgtgaccat gttgatggct caccactgct ggtggcagca  720
   ggaagcaatg ggatcattcg ggtcatcaat tgtgctacag aaaagttagc taagagcttt  780
   gttggccatg gcgactcaat aaatgagata agaactcaac cgttgaagcc ttcgctcatc  840
   atttctgcaa gcaaggatga atctgttagg ctatggaatg tccatacagg gatctgtatc  900
   ttgatatttg ctggagctgg aggtcatcgc aatgaagtat tgagtgttga cttccatcct  960
   agtgatattg aacgttttgc aagttgtggc atggacaaca ctgtgaaaat ctggtcaatg 1020
   aaagaatttt ggctatatgt tgacaaatca tattcatgga ctgaccttcc atcaaagttt 1080
   ccaacaaaat atgtccagtt tccagtcttg attgctgcag tacactctaa ctatgttgat 1140
   tgtacaagat ggcttggtga cttcatccta tcaaagagtg ttgacaatga aattgtgctt 1200
   tgggaaccga agacaaaaga acagagtcct ggggagggaa gcatcgatat ccttcagaag 1260
   tatcctgtcc cagaatgtga catttggttt atcaaatttt catgtgattt tcacttcaat 1320
   cagttggcga taggcaaccg tgaaggcaaa atctacgtgt gggaagtaca gtccagccct 1380
   cctgtcctca ttgctcggct gtataatcag cagtgtaaat cgccgataag acaaactgca 1440
   gtgtccttcg atggaagcac aatccttgga gctggtgaag acggcaccat ctggcggtgg 1500
   gatgaagtgg accatccgag ctccagaaac tgaagaagtg ttgccgctca atgctggact 1560
   gatggttacg ctcggttggg gttgcgatgg ttgaatccgt tggtggaaag tgccacctgg 1620
   tgttttttct agtcaaaatg gttggtgtta acagaatatt gaatgcttcg aatgttgaaa 1680
   gttggg                                                            1686
<210> 30
   <211> 379
   <212> PRT
   <213> Zea mays
<400> 30
   Met ala Lys Leu Gly Pro Gly Gln Gly Leu Gly Cys Glu Ala Ala Glu
    1               5                  10                  15
   Gly Ser Leu Val Pro Ser Arg Lys Arg Glu Tyr Lys Pro Cys Gly Lys
               20                  25                  30
   His Thr Glu Gly Lys Arg Pro Leu Tyr Ala Ile Gly Phe Asn Phe Met
           35                  40                  45
   Asp Ala Arg Tyr Tyr Asp Val Phe Ala Thr Val Gly Gly Asn Arg Val
       50                  55                  60
   Thr Thr Tyr Arg Cys Leu Glu Asn Gly Ser Phe Ala Leu Leu Gln Ala
   65                  70                  75                  80
   Tyr Val Asp Glu Asp Lys Asp Glu Ser Phe Tyr Thr Leu Ser Trp Ala
                   85                  90                  95
   Arg Asp His Val Asp Gly Ser Pro Leu Leu Val Ala Ala Gly Ser Asn
               100                 105                 110
   Gly Ile Ile Arg Val Ile Asn Cys Ala Thr Glu Lys Leu Ala Lys Ser
           115                 120                 125
   Phe Val Gly His Gly Asp Ser Ile Asn Glu Ile Arg Thr Gln Pro Leu
       130                 135                 140
   Lys Pro Ser Leu Ile Ile Ser Ala Ser Lys Asp Glu Ser Val Arg Leu
   145                 150                 155                 160
   Trp Asn Val His Thr Gly Ile Cys Ile Leu Ile Phe Ala Gly Ala Gly
                   165                 170                 175
   Gly His Arg Asn Glu Val Leu Ser Val Asp Phe His Pro Ser Asp Ile
               180                 185                 190
   Glu Arg Phe Ala Ser Cys Gly Met Asp Asn Thr Val Lys Ile Trp Ser
           195                 200                 205
   Met Lys Glu Phe Trp Leu Tyr Val Asp Lys Ser Tyr Ser Trp Thr Asp
       210                 215                 220
   Leu Pro Ser Lys Phe Pro Thr Lys Tyr Val Gln Phe Pro Val Leu Ile
   225                 230                 235                 240
   Ala Ala Val His Ser Asn Tyr Val Asp Cys Thr Arg Trp Leu Gly Asp
                   245                 250                 255
   Phe Ile Leu Ser Lys Ser Val Asp Asn Glu Ile Val Leu Trp Glu Pro
               260                 265                 270
   Lys Thr Lys Glu Gln Ser Pro Gly Glu Gly Ser Ile Asp Ile Leu Gln
           275                 280                 285
   Lys Tyr Pro Val Pro Glu Cys Asp Ile Trp Phe Ile Lys Phe Ser Cys
       290                 295                 300
   Asp Phe His Phe Asn Gln Leu Ala Ile Gly Asn Arg Glu Gly Lys Ile
   305 310 315 320
   Tyr Val Trp Glu Val Gln Ser Ser Pro Pro Val Leu Ile Ala Arg Leu
   325 330 335
   Tyr Asn Gln Gln Cys Lys Ser Pro Ile Arg Gln Thr Ala Val Ser Phe
   340 345 350
   Asp Gly Ser Thr Ile Leu Gly Ala Gly Glu Asp Gly Thr Ile Trp Arg
   355 360 365
   Trp Asp Glu Val Asp His Pro Ser Ser Arg Asn
   370 375
<210> 31
   <211> 595
   <212> DNA
   <213> Zea mays
<400> 31
   ccacgcgtcc gcaatgaaat tgtgctttgg gaaccgaaga caaaagaaca gagtcctggg  60
   gagggaagca tcgatatcct tcagaagtat cctgtcccag aatgtgacat ttggtttatc 120
   aaattttcat gtgattttca cttcaatcag ttggcgatag gcaaccgtga aggcaaaatc 180
   tacgtgtggg aagtacagtc cagccctcct gtcctcattg ctcggctgta taatcagcag 240
   tgtaaatcgc cgataagaca aactgcagtg tccttcgatg gaagcacaat ccttggagct 300
   ggtgaagacg gcaccatctg gcggtgggat gaagtggacc atccgagctc cagaaactga 360
   agaagtgttg ccgctcaatg ctggactgat ggttacgctc ggttggggtt gcgatggttg 420
   aatccgttgg tggaaagtgc cacctggtgt tttttctagt caaaatggtt ggtgttaaca 480
   gaatattgaa tgcttcgaat gttgaaagtt gggatgcttg tgctggtaaa aaaaaaaaaa 540
   aaaaaaaaaa aaaaaaaaaa aaaaaaaaaa aaaaaaaaaa aaaaaaaaaa aaaag      595
<210> 32
   <211> 119
   <212> PRT
   <213> Zea mays
<400> 32
   Pro Arg Val Arg Asn Glu Ile Val Leu Trp Glu Pro Lys Thr Lys Glu
   1 5 10 15
   Gln Ser Pro Gly Glu Gly Ser Ile Asp Ile Leu Gln Lys Tyr Pro Val
   20 25 30
   Pro Glu Cys Asp Ile Trp Phe Ile Lys Phe Ser Cys Asp Phe His Phe
   35 40 45
   Asn Gln Leu Ala Ile Gly Asn Arg Glu Gly Lys Ile Tyr Val Trp Glu
   50 55 60
   Val Gln Ser Ser Pro Pro Val Leu Ile Ala Arg Leu Tyr Asn Gln Gln
   65 70 75 80
   Cys Lys Ser Pro Ile Arg Gln Thr Ala Val Ser Phe Asp Gly Ser Thr
   85 90 95
   Ile Leu Gly Ala Gly Glu Asp Gly Thr Ile Trp Arg Trp Asp Glu Val
   100 105 110
   Asp His Pro Ser Ser Arg Asn
   115
<210> 33
   <211> 1498
   <212> DNA
   <213> Oryza sativa
<220>
   <221> unsure
   <222> (839)
<400> 33
   cgtcctcctt cttccaccgc atcgtcgctc gccgccgtta tgaacttcca aatttggttc   60
   caagcaggaa gagggagtac aaggcgtgca acaagctcac cgaggggaag cggcagctct  120
   acgccatcgg attcaacttc ctcgacttcc actactacga ggtcttcgcc accgtcggcg  180
   gcaaccgcgt gacaacctac agctgcctca aggatggtaa ttttgctatc ctgcaagcat  240
   atattgatga ggataaggat gaatcgttct acacactgag ttgggcttgt gatcttgatg  300
   gcacaccgct gttagtggct gcaggaagca atgggatcat tcgggtcatc aactgtgcca  360
   ctgagaagtt actcaagact tttgttggcc atggcgattc aataaacgag ataagaactc  420
   aagcattaaa gccttcgctc atcatttctg caagcaagga tgaatctgtt aggctgtgga  480
   atgttcacac agggatctgc attttgattt ttgctggagc aggaggtcac cggaatgaag  540
   tattgagtgt tgacttccac ccatctgata tctaccgcat agcaagttgt ggcatggata  600
   acactgttaa aatatggtca atgaaggaat tctggccata tgttgagcaa tcctttacat  660
   ggactgacct tccatcaaaa tttccaacaa aatatgtgca atttccggtc ttggttgctg  720
   tagtacattc taactatgtt gattgtacta gatggcttgg tgacttcatt ctgtcaaaga  780
   gtgttgacaa tgaaattgtg ctgtgggagc caaaaacaaa agaacaaagt cccggggang  840
   gtagcattga tattcttcag aagtatcctg tgccagaatg tgatatctgg tttatcaaat  900
   tctcatgcga ttttcacttc aatcaattgg caataggcaa ccgtgaagga aaagtctttg  960
   tctgggaagt acagtccagt cctcctgttt taactgctcg gctgactaat ccgcaatgca 1020
   aatctgcgat aaggcagact gccgtgtcat ttgatggaag cacaatcctt gcctgcagcg 1080
   aggatggcag catatggcga tgggatgaag tggaccatcc aaaagcatga aaagtaccct 1140
   tatagacaga ccatggcaat gccagattaa gattgacttg ggaattcctg catgtgtact 1200
   ttgttgtggg ggttatagta atcagtctta ctgttgaaaa aaagtgcaat ctgatactct 1260
   gaaattagaa ggattgacag ctgaatgctg gggttaccaa cttgaatgtt gcaaatagga 1320
   tactgcttct gttatatgct gaatgtttca agttagggcc tttttgtaaa tgggaagatt 1380
   cggctatgcc agatttttgg aaaagttgcc atttgctttg ttaccaaagt tgcatggcaa 1440
   agattggccc agctcaaatt tctatagtta taaatgagtt gccaaatatt ttggcttc   1498
<210> 34
   <211> 375
   <212> PRT
   <213> Oryza sativa
<220>
   <221> UNSURE
   <222> (279)
<400> 34
   Ser Ser Phe Phe His Arg Ile Val Ala Arg Arg Arg Tyr Glu Leu Pro
    1               5                  10                  15
   Asn Leu Val Pro Ser Arg Lys Arg Glu Tyr Lys Ala Cys Asn Lys Leu
               20                  25                  30
   Thr Glu Gly Lys Arg Gln Leu Tyr Ala Ile Gly Phe Asn Phe Leu Asp
   35 40 45
   Phe His Tyr Tyr Glu Val Phe Ala Thr Val Gly Gly Asn Arg Val Thr
   50 55 60
   Thr Tyr Ser Cys Leu Lys Asp Gly Asn Phe Ala Ile Leu Gln Ala Tyr
   65 70 75 80
   Ile Asp Glu Asp Lys Asp Glu Ser Phe Tyr Thr Leu Ser Trp Ala Cys
   85 90 95
   Asp Leu Asp Gly Thr Pro Leu Leu Val Ala Ala Gly Ser Asn Gly Ile
   100 105 110
   Ile Arg Val Ile Asn Cys Ala Thr Glu Lys Leu Leu Lys Thr Phe Val
   115 120 125
   Gly His Gly Asp Ser Ile Asn Glu Ile Arg Thr Gln Ala Leu Lys Pro
   130 135 140
   Ser Leu Ile Ile Ser Ala Ser Lys Asp Glu Ser Val Arg Leu Trp Asn
   145 150 155 160
   Val His Thr Gly Ile Cys Ile Leu Ile Phe Ala Gly Ala Gly Gly His
   165 170 175
   Arg Asn Glu Val Leu Ser Val Asp Phe His Pro Ser Asp Ile Tyr Arg
   180 185 190
   Ile Ala Ser Cys Gly Met Asp Asn Thr Val Lys Ile Trp Ser Met Lys
   195 200 205
   Glu Phe Trp Pro Tyr Val Glu Gln Ser Phe Thr Trp Thr Asp Leu Pro
   210 215 220
   Ser Lys Phe Pro Thr Lys Tyr Val Gln Phe Pro Val Leu Val Ala Val
   225 230 235 240
   Val His Ser Asn Tyr Val Asp Cys Thr Arg Trp Leu Gly Asp Phe Ile
   245 250 255
   Leu Ser Lys Ser Val Asp Asn Glu Ile Val Leu Trp Glu Pro Lys Thr
   260 265 270
   Lys Glu Gln Ser Pro Gly Xaa Gly Ser Ile Asp Ile Leu Gln Lys Tyr
   275 280 285
   Pro Val Pro Glu Cys Asp Ile Trp Phe Ile Lys Phe Ser Cys Asp Phe
   290 295 300
   His Phe Asn Gln Leu Ala Ile Gly Asn Arg Glu Gly Lys Val Phe Val
   305 310 315 320
   Trp Glu Val Gln Ser Ser Pro Pro Val Leu Thr Ala Arg Leu Thr Asn
   325 330 335
   Pro Gln Cys Lys Ser Ala Ile Arg Gln Thr Ala Val Ser Phe Asp Gly
   340 345 350
   Ser Thr Ile Leu Ala Cys Ser Glu Asp Gly Ser Ile Trp Arg Trp Asp
           355                 360                 365
   Glu Val Asp His Pro Lys Ala
       370                 375
<210> 35
   <211> 1387
   <212> DNA
   <213> Glycine max
<220>
   <221> unsure
   <222> (14)
<400> 35
   gggaagcgaa cagnagaaga gtagatggtg ggtgaaacgg cggcaacggg gaagtcggtt   60
   ggtttgggtt tgggatgtga cccagtggtg ggatccttgg cttgttcgaa gaagagagaa  120
   tacagagtca ccaatcgcct tcaagaggga aagcgccctc tatacgccgt cattttcaac  180
   ttcatcgact cccgctactt caacgttttc gccactgttg gcggcaatag ggttactgtt  240
   tatcaatgcc ttgatgaagg ggatattgct gttttgcaat cttatgcgga tgaggataag  300
   aatgagtctt tttacaccgt gggttgggca tgcaatgttg acgggacccc acttgttgtg  360
   gctggaggac tcaatggggt aatccgagtc attgatgctg gcagtgagaa gatacataag  420
   agttttgttg gccatggaga ctccataaat gaagtcaaag ctcaaatatt aaatccatca  480
   ctcgtggtat cggcaagcaa agatgaatct attcggttat ggaatgctca tactggaata  540
   tgcattttga tatttgctgg aggcggggga catcgtaatg aagtcttaag tgttgatttt  600
   catccatcgg atatgtatcg tatttgtagt tgtggcatgg atagtactgt aaaaatatgg  660
   tctatgaagg agttctggac atatgtagaa aaatcatcca catggacaga tcttccttcc  720
   aagtttccaa caaaatttgt ccagtttcct gtttacactg cttcagtgca tataaattat  780
   gttgactgta ataggtggtt gggtgatttt atcctctcaa agagtgttga taatgaaatt  840
   atcttgtggg aacctaaagt gaacgaacaa actccaggga agggtgtagt tgatgttctt  900
   cataaatacc ctattcccga ttgcaatatc tggttcatca agttttcttg tgacttccat  960
   ttcaacatag ttacagtggg taacagggaa gggaagattt ttgtttggga attacagtca 1020
   agtcctcccg tacttgctgc aaagttgtca catcctcaat caaaatcccc aatcaggcag 1080
   actgcaacat cctttgatgg aagtactata ttgagttgct gtgaggatgg gacaatatgg 1140
   cgttgggatg tttcaaaacc ctcaacctca acctcaaccg cagcctaact tatcttcgtg 1200
   caacaccaat ctgatgtgca tgtcaaacac aagggcattt gtgatttatc aatttaacca 1260
   gtcatgtaca tcaggaactt gatttattgc atgtttttgt atttgtttat tttggttcgg 1320
   taaggcttat aatgtaaaat gttcaactaa gaactcagtt aaaagttatt taaataaagt 1380
   aaagcca                                                           1387
<210> 36
   <211> 387
   <212> PRT
   <213> Glycine max
<400> 36
   Met Val Gly Glu Thr Ala Ala Thr Gly Lys Ser Val Gly Leu Gly Leu
    1               5                  10                  15
   Gly Cys Asp Pro Val Val Gly Ser Leu Ala Cys Ser Lys Lys Arg Glu
               20                  25                  30
   Tyr Arg Val Thr Asn Arg Leu Gln Glu Gly Lys Arg Pro Leu Tyr Ala
           35                  40                  45
   Val Ile Phe Asn Phe Ile Asp Ser Arg Tyr Phe Asn Val Phe Ala Thr
       50                  55                  60
   Val Gly Gly Asn Arg Val Thr Val Tyr Gln Cys Leu Asp Glu Gly Asp
   65 70 75 80
   Ile Ala Val Leu Gln Ser Tyr Ala Asp Glu Asp Lys Asn Glu Ser Phe
   85 90 95
   Tyr Thr Val Gly Trp Ala Cys Asn Val Asp Gly Thr Pro Leu Val Val
   100 105 110
   Ala Gly Gly Leu Asn Gly Val Ile Arg Val Ile Asp Ala Gly Ser Glu
   115 120 125
   Lys Ile His Lys Ser Phe Val Gly His Gly Asp Ser Ile Asn Glu Val
   130 135 140
   Lys Ala Gln Ile Leu Asn Pro Ser Leu Val Val Ser Ala Ser Lys Asp
   145 150 155 160
   Glu Ser Ile Arg Leu Trp Asn Ala His Thr Gly Ile Cys Ile Leu Ile
   165 170 175
   Phe Ala Gly Gly Gly Gly His Arg Asn Glu Val Leu Ser Val Asp Phe
   180 185 190
   His Pro Ser Asp Met Tyr Arg Ile Cys Ser Cys Gly Met Asp Ser Thr
   195 200 205
   Val Lys Ile Trp Ser Met Lys Glu Phe Trp Thr Tyr Val Glu Lys Ser
   210 215 220
   Ser Thr Trp Thr Asp Leu Pro Ser Lys Phe Pro Thr Lys Phe Val Gln
   225 230 235 240
   Phe Pro Val Tyr Thr Ala Ser Val His Ile Asn Tyr Val Asp Cys Asn
   245 250 255
   Arg Trp Leu Gly Asp Phe Ile Leu Ser Lys Ser Val Asp Asn Glu Ile
   260 265 270
   Ile Leu Trp Glu Pro Lys Val Asn Glu Gln Thr Pro Gly Lys Gly Val
   275 280 285
   Val Asp Val Leu His Lys Tyr Pro Ile Pro Asp Cys Asn Ile Trp Phe
   290 295 300
   Ile Lys Phe Ser Cys Asp Phe His Phe Asn Ile Val Thr Val Gly Asn
   305 310 315 320
   Arg Glu Gly Lys Ile Phe Val Trp Glu Leu Gln Ser Ser Pro Pro Val
   325 330 335
   Leu Ala Ala Lys Leu Ser His Pro Gln Ser Lys Ser Pro Ile Arg Gln
   340 345 350
   Thr Ala Thr Ser Phe Asp Gly Ser Thr Ile Leu Ser Cys Cys Glu Asp
   355 360 365
   Gly Thr Ile Trp Arg Trp Asp Val Ser Lys Pro Ser Thr Ser Thr Ser
   370 375 380
   Thr Ala Ala
   385
<210> 37
   <211> 1518
   <212> DNA
   <213> Triticum aestivum
<400> 37
   gcgtctgcaa aggcggbggc gctgctccaa ggcgtgggcc gagaggagga gaagcaggcg   60
   ccagagcccc aacctctccc ggcgcgcatg gcgaggctgg gcccggggca ggggttaggg  120
   tgcgaggcgg cggtggggtc gctggcgccc agccggagcc gggagtacaa gctctgcagc  180
   aagcacaccg agggcaagcg cccgctctac gccatcggct tcaacttcat cgacgcccgc  240
   tactacgacg tcttcgccac cgtcggcggc aatcgtgtga cgacgtaccg tggcctcccc  300
   gacggtaact tggctgttct gcaagcatac attgatgcgg acgatgctca gtcattctac  360
   actctgagct gggcttgtga ccttgacggc acaccactgc tagtggcagc aggaagcaat  420
   gcggtcattc gggtcatcaa ctgtgccacc gagaagttgt ttaagagttt tcttggccat  480
   ggtgattcaa taaatgagat aagaactcaa ccattgaagc cttcgctctt catttctgca  540
   agcaaggacg agtctgttag gctatggaat gtccatacag gtatctgcat cttgattttt  600
   gctggaggag gaggtcaccg taatgaagta ttgagtgttg acttccaccc ttctgatatc  660
   taccgaattg ccagttgtgg catggataat actgttaaaa tctggtcaat gaaagaattt  720
   tggccatacg tggagaaatc ctttacatgg actgaccttc catcaaaatt tccaacgaaa  780
   tttgttcaat ttccgctcat gacttccgtg gttcattcta actatgttga ctgtactagg  840
   tggcttggtg acttcatcct gtcgaagagt gttgacaatg aaattgttct gtgggagcca  900
   aaaataaaag agcagggtcc cggcgagggt agcattgatg ttcttcagaa gtaccctgtg  960
   cctgattgtg acatttggtt tatcaaattc tcatgtgatt ttcacttcaa tcaattagca 1020
   ataggcaacc gcgaaggcaa aatctatgtg tgggaagtgc aggcgagccc tcctgtgcta 1080
   attacccggc tgagtagtcc acaatgcaaa atgccaataa ggcagactgc agtgtcgttt 1140
   gatggaagca cgatccttgc ctgcggcgag gatggcagca tataccgctg ggatgaagtg 1200
   gaacatcaag ctgcaaaaaa ttgaagcaac tgaaaaccac catccgtgcg gccccatggc 1260
   aatgccagcc agtttgagct tgtcctgggt agttgttgtg ttgcttactt agtgggttgt 1320
   accaattact tagtccagaa gttggggtga atgagcttat aatgttgtaa ggttggatgt 1380
   tgttgattcg atgatttgcc ggatgtttct gtttattaca ttggctgtat catgtaccga 1440
   atgtgggagt taaacttaaa tcctcgttcg cattctaaaa aaaaaaaaaa aaaaaaaaaa 1500
   aaaaaaaaaa aaaaaaaa                                               1518
<210> 38
   <211> 378
   <212> PRT
   <213> Triticum aestivum
<400> 38
   Met ala Arg Leu Gly Pro Gly Gln Gly Leu Gly Cys Glu Ala Ala Val
   1 5 10 15
   Gly Ser Leu Ala Pro Ser Arg Ser Arg Glu Tyr Lys Leu Cys Ser Lys
   20 25 30
   His Thr Glu Gly Lys Arg Pro Leu Tyr Ala Ile Gly Phe Asn Phe Ile
   35 40 45
   Asp Ala Arg Tyr Tyr Asp Val Phe Ala Thr Val Gly Gly Asn Arg Val
   50 55 60
   Thr Thr Tyr Arg Gly Leu Pro Asp Gly Asn Leu Ala Val Leu Gln Ala
   65 70 75 80
   Tyr Ile Asp Ala Asp Asp Ala Gln Ser Phe Tyr Thr Leu Ser Trp Ala
   85 90 95
   Cys Asp Leu Asp Gly Thr Pro Leu Leu Val Ala Ala Gly Ser Asn Ala
   100 105 110
   Val Ile Arg Val Ile Asn Cys Ala Thr Glu Lys Leu Phe Lys Ser Phe
   115 120 125
   Leu Gly His Gly Asp Ser Ile Asn Glu Ile Arg Thr Gln Pro Leu Lys
   130 135 140
   Pro Ser Leu Phe Ile Ser Ala Ser Lys Asp Glu Ser Val Arg Leu Trp
   145 150 155 160
   Asn Val His Thr Gly Ile Cys Ile Leu Ile Phe Ala Gly Gly Gly Gly
   165 170 175
   His Arg Asn Glu Val Leu Ser Val Asp Phe His Pro Ser Asp Ile Tyr
   180 185 190
   Arg Ile Ala Ser Cys Gly Met Asp Asn Thr Val Lys Ile Trp Ser Met
   195 200 205
   Lys Glu Phe Trp Pro Tyr Val Glu Lys Ser Phe Thr Trp Thr Asp Leu
   210 215 220
   Pro Ser Lys Phe Pro Thr Lys Phe Val Gln Phe Pro Leu Met Thr Ser
   225 230 235 240
   Val Val His Ser Asn Tyr Val Asp Cys Thr Arg Trp Leu Gly Asp Phe
   245 250 255
   Ile Leu Ser Lys Ser Val Asp Asn Glu Ile Val Leu Trp Glu Pro Lys
   260 265 270
   Ile Lys Glu Gln Gly Pro Gly Glu Gly Ser Ile Asp Val Leu Gln Lys
   275 280 285
   Tyr Pro Val Pro Asp Cys Asp Ile Trp Phe Ile Lys Phe Ser Cys Asp
   290 295 300
   Phe His Phe Asn Gln Leu Ala Ile Gly Asn Arg Glu Gly Lys Ile Tyr
   305 310 315 320
   Val Trp Glu Val Gln Ala Ser Pro Pro Val Leu Ile Thr Arg Leu Ser
   325 330 335
   Ser Pro Gln Cys Lys Met Pro Ile Arg Gln Thr Ala Val Ser Phe Asp
   340 345 350
   Gly Ser Thr Ile Leu Ala Cys Gly Glu Asp Gly Ser Ile Tyr Arg Trp
   355 360 365
   Asp Glu Val Glu His Gln Ala Ala Lys Asn
   370 375
<210> 39
   <211> 488
   <212> DNA
   <213> Zea mays
<220>
   <221> unsure
   <222> (464)
<220>
   <221> unsure
   <222> (475)
<220>
   <221> unsure
   <222> (488)
<400> 39
   gccggaagaa gtcgccgcgt gaggtcagtg tccccgttgc tgccgcctct aacccgaagc  60
   ctaggccgct gccggtgcat aacaaggaga atcaggcgga ggggaaagta gcagaggagg 120
   gggcagcaac tgaggagggg gagaagtacc gggcggaacc ggaaatcttg ccgctgccgc 180
   cggccatggc gaactgggcc cggggcaggg gctcgggtgc gaggcggcgg aggggtcgct 240
   cgtgcccagc cggaagcggg agtacaagcc ctgcggcaag cacactgagg ggaagcgccc 300
   gctatatgct atcgggttca acttcatgga cgcgcgctac tacgacgtct tcgccaccgt 360
   cggcggcaac cgcgtgaaca acttaccgct gccttgagaa tggtagtttc gctcttctac 420
   aagcttacgt tgatgaggat aaggatgagt cgttctatac tccnaagctg ggccnttgac 480
   catgttgn                                                          488
<210> 40
   <211> 84
   <212> PRT
   <213> Zea mays
<220>
   <221> UNSURE
   <222> (56)
<400> 40
   Leu Gly Cys Glu Ala Ala Glu Gly Ser Leu Val Pro Ser Arg Lys Arg
   1               5                   10                  15
   Glu Tyr Lys Pro Cys Gly Lys His Thr Glu Gly Lys Arg Pro Leu Tyr
               20                  25                  30
   Ala Ile Gly Phe Asn Phe Met Asp Ala Arg Tyr Tyr Asp Val Phe Ala
           35                  40                  45
   Thr Val Gly Gly Asn Arg Val Xaa Thr Thr Tyr Arg Cys Leu Glu Asn
       50                  55                  60
   Gly Ser Phe Ala Leu Leu Gln Ala Tyr Val Asp Glu Asp Lys Asp Glu
   65                  70                  75                  80
   Ser Phe Tyr Thr
               84
<210> 41
   <211> 348
   <212> DNA
   <213> Zea mays
<400> 41
   gccatcgcca gttgcaatag tgtgataatt taccgatgcc ttgagaatgg tggttttggt  60
   cttctacaaa attatgttga tgaggataag gatgagtcat tctacactct aagctggacc 120
   atcgatcaag ttgatagctc accgctgttg gtggccgcag gaagcaatcg gatcattcgg 180
   gtcatcaatt gtgctaccga aaagttagat aagagcttag ttggccatgg tggttcaata 240
   catgagataa ggactcatgc ctcgaagcca tcactcatca tttctgccag caaggatgaa 300
   tctattaggc tatggaatgt ccatactggg atttgcatct tagtcttt              348
<210> 42
   <211> 116
   <212> PRT
   <213> Zea mays
<400> 42
   Ala Ile Ala Ser Cys Asn Ser Val Ile Ile Tyr Arg Cys Leu Glu Asn
    1               5                  10                  15
   Gly Gly Phe Gly Leu Leu Gln Asn Tyr Val Asp Glu Asp Lys Asp Glu
               20                  25                  30
   Ser Phe Tyr Thr Leu Ser Trp Thr Ile Asp Gln Val Asp Ser Ser Pro
           35                  40                  45
   Leu Leu Val Ala Ala Gly Ser Asn Arg Ile Ile Arg Val Ile Asn Cys
       50                  55                  60
   Ala Thr Glu Lys Leu Asp Lys Ser Leu Val Gly His Gly Gly Ser Ile
   65                  70                  75                  80
   His Glu Ile Arg Thr His Ala Ser Lys Pro Ser Leu Ile Ile Ser Ala
                   85                  90                  95
   Ser Lys Asp Glu Ser Ile Arg Leu Trp Asn Val His Thr Gly Ile Cys
               100                 105                 110
   Ile Leu Val Phe
           115
<210> 43
   <211> 488
   <212> DNA
   <213> Zea mays
<220>
   <221> unsure
   <222> (1)
<220>
   <221> unsure
   <222> (454)
<220>
   <221> unsure
   <222> (462)
<220>
   <221> unsure
   <222> (471)
<400> 43
   naagtttcca acaaaatatg tccagtttcc agtcttgatt gctgcagtac actctaacta  60
   tgttgattgt acaagatggc ttggtgactt catcctatca aagagtgttg acaatgaaat 120
   tgtgctttgg gaaccgaaga caaaagaaca gagtcctggg gagggaagca tcgatatcct 180
   tcagaagtat cctgtcccag aatgtgacat ttggtttatc aaattttcat gtgattttca 240
   cttcaatcag ttggcgatag gcaaccgtga aggcaaaatc tacgtgtggg aagtacagtc 300
   cagccctcct gtcctcattg ctcggctgta taatcagcag tgtaaatcgc cgataagaca 360
   aactgcagtg tccttcgatg gaagcacaat ccttggagct ggtgaagacg gcaccatctg 420
   gcggtgggga tgaagtggac catccgagct ccanaagctg angaagtgtt nccggctcaa 480
   tgctggtg                                                          488
<210> 44
   <211> 145
   <212> PRT
   <213> Zea mays
<400> 44
   Lys Phe Pro Thr Lys Tyr Val Gln Phe Pro Val Leu Ile Ala Ala Val
   1               5                   10                  15
   His Ser Asn Tyr Val Asp Cys Thr Arg Trp Leu Gly Asp Phe Ile Leu
               20                  25                  30
   Ser Lys Ser Val Asp Asn Glu Ile Val Leu Trp Glu Pro Lys Thr Lys
           35                  40                  45
   Glu Gln Ser Pro Gly Glu Gly Ser Ile Asp Ile Leu Gln Lys Tyr Pro
       50                  55                  60
   Val Pro Glu Cys Asp Ile Trp Phe Ile Lys Phe Ser Cys Asp Phe His
   65                  70                  75                  80
   Phe Asn Gln Leu Ala Ile Gly Asn Arg Glu Gly Lys Ile Tyr Val Trp
                   85                  90                  95
   Glu Val Gln Ser Ser Pro Pro Val Leu Ile Ala Arg Leu Tyr Asn Gln
               100                 105                 110
   Gln Cys Lys Ser Pro Ile Arg Gln Thr Ala Val Ser Phe Asp Gly Ser
           115                 120                 125
   Thr Ile Leu Gly Ala Gly Glu Asp Gly Thr Ile Trp Arg Trp Asp Glu
       130                 135                 140
   Val
   145
<210> 45
   <211> 460
   <212> DNA
   <213> Eucalyptus grandis
<220>
   <221> unsure
   <222> (319)
<220>
   <221> unsure
   <222> (331)
<220>
   <221> unsure
   <222> (355)
<220>
   <221> unsure
   <222> (387)
<220>
   <221> unsure
   <222> (426)..(427)..(428)
<220>
   <221> unsure
   <222> (437)
<220>
   <221> unsure
   <222> (447)
<220>
   <221> unsure
   <222> (451)
<400> 45
   ctcgttcgcc gttcggcgtc ttcaccggcg gcgcgcgccg cactgcgtac ccaccggctg  60
   tcgcgttctc gcggatcgaa ctcgaggaaa aggcatcggc ggcggatcgg ggcaaatggc 120
   gaagatcgcg cccgggtgcg aaccggtggc ggggacgctg accccgtcga agaagaggga 180
   gtacagggtc accaacaggc tccaggaggg gaagcgtccc ctctatgccg tcgtcttcaa 240
   cttcatcgac tcccgctact tcaacgtatt cgccaccgtc ggcggcaacc ggggttactg 300
   tttatcaagt gtctcgaang gggagtaata nctgtgttgc agtcatacat tgatnaagat 360
   aaggacgagt ccgttttaca cggtcangtg gggcgtgcaa acatttatag aaccccaatt 420
   gtgggnnngc gggaggnaac aattggnaac natcgggtgt                      460
<210> 46
   <211> 59
   <212> PRT
   <213> Eucalyptus grandis
<400> 46
   Met ala Lys Ile Ala Pro Gly Cys Glu Pro Val Ala Gly Thr Leu Thr
    1               5                  10                  15
   Pro Ser Lys Lys Arg Glu Tyr Arg Val Thr Asn Arg Leu Gln Glu Gly
               20                  25                  30
   Lys Arg Pro Leu Tyr Ala Val Val Phe Asn Phe Ile Asp Ser Arg Tyr
           35                  40                  45
   Phe Asn Val Phe Ala Thr Val Gly Gly Asn Arg
       50                  55
<210> 47
   <211> 521
   <212> DNA
   <213> Helianthus sp.
<220>
   <221> unsure
   <222> (390)
<220>
   <221> unsure
   <222> (517)
<220>
   <221> unsure
   <222> (519)
<400> 47
   cttggtgatt tcatactatc taagagtgta gacaatgagt tcatattgtg ggagccgaag  60
   atgaaagagc agtctccagg agagggcacg gtggatattc ttcagaaata tcctgtacct 120
   gattgtgaca tctggtttat aaagctttcc tgtgatttcc attacaatgc agcagctatt 180
   ggtaacagag aaggaaaaat ctatgtatgg gaattgcaga ctagcccgcc ttctcttatt 240
   gcaaggttat ctcatattca agtccaaatc gccaatcagg caaactgcta tgtcatttga 300
   tggaagcaca attctgagtt gctgtgaaga tggcaccatc tggcgttggg atactgttgc 360
   aacgtcgtag cttgtgttgg tttgaaacan gtcatgttgt gtaccatgta tattccttca 420
   gcaatttcgt ttgttttccg tggtgatgat tgagggcatt ttaatttgtt ctttattaaa 480
   ctatgatagt aaggatgtta ttccgtttta gtgaacngnc c                     521
<210> 48
   <211> 119
   <212> PRT
   <213> Helianthus sp.
<220>
   <221> UNSURE
   <222> (87)
<400> 48
   Gly Asp Phe Ile Leu Ser Lys Ser Val Asp Asn Glu Phe Ile Leu Trp
    1               5                  10                  15
   Glu Pro Lys Met Lys Glu Gln Ser Pro Gly Glu Gly Thr Val Asp Ile
               20                  25                  30
   Leu Gln Lys Tyr Pro Val Pro Asp Cys Asp Ile Trp Phe Ile Lys Leu
           35                  40                  45
   Ser Cys Asp Phe His Tyr Asn Ala Ala Ala Ile Gly Asn Arg Glu Gly
       50                  55                  60
   Lys Ile Tyr Val Trp Glu Leu Gln Thr Ser Pro Pro Ser Leu Ile Ala
   65                  70                  75                  80
   Arg Leu Ser His Ile Gln Xaa Ser Lys Ser Pro Ile Arg Gln Thr Ala
                   85                  90                  95
   Met Ser Phe Asp Gly Ser Thr Ile Leu Ser Cys Cys Glu Asp Gly Thr
               100                 105                 110
   Ile Trp Arg Trp Asp Thr Val
           115
<210> 49
   <211> 658
   <212> DNA
   <213> Catalpa speciosa
<220>
   <221> unsure
   <222> (367)
<220>
   <221> unsure
   <222> (445)
<220>
   <221> unsure
   <222> (456)
<220>
   <221> unsure
   <222> (483)
<220>
   <221> unsure
   <222> (492)
<220>
   <221> unsure
   <222> (509)
<220>
   <221> unsure
   <222> (549)
<220>
   <221> unsure
   <222> (554)
<220>
   <221> unsure
   <222> (563)
<220>
   <221> unsure
   <222> (579)
<220>
   <221> unsure
   <222> (584)
<220>
   <221> unsure
   <222> (602)
<220>
   <221> unsure
   <222> (611)
<220>
   <221> unsure
   <222> (648)
<220>
   <221> unsure
   <222> (657)
<400> 49
   ggcatacagg cggtgctaat ctgcaggtaa ggagatggca aaaattccgt tgggttgtga  60
   gcccatggtg ggttccttaa cgccgtcgaa gaaacgggag tatagggtca ccaacaggct 120
   ccaggaaggc aaacgcccca tttacgccgt cgttttcaac ttcattgact cccgttactt 180
   caacgctttc gccactgccg gtggcaatcg cgtgactgta taccaagtgc ctagaaggtg 240
   gtgttatagc tgtactacag tcctacattg atgaagataa agatgaatct ttctacactg 300
   taagttgggc ttgcaatatt gatgggactc cattcttggt ggctggagga cttaatggaa 360
   ttattcnagt tattgatact ggcaatgaga aaatatacaa gagtttgtgg gtcatgggga 420
   atcaataaac gaaatccaac tcaancgctg aaacancact tgttgtgtca acaaacaaag 480
   atnaatctta cnctgtggaa tatcatacng gatatcattt gatatttctg ggctgtggca 540
   tccatgaant ctangggcct cancctctac acacgtatna aacntgaagg taaatgcaga 600
   tnggcatgaa nattggcaag taaaacttct gcgttctcaa tccaaaanca tcaatang   658
<210> 50
   <211> 124
   <212> PRT
   <213> Catalpa speciosa
<220>
   <221> UNSURE
   <222> (64)
<220>
   <221> UNSURE
   <222> (111)
<400> 50
   Met ala Lys Ile Pro Leu Gly Cys Glu Pro Met Val Gly Ser Leu Thr
   1               5                   10                  15
   Pro Ser Lys Lys Arg Glu Tyr Arg Val Thr Asn Arg Leu Gln Glu Gly
               20                  25                  30
   Lys Arg Pro Ile Tyr Ala Val Val Phe Asn Phe Ile Asp Ser Arg Tyr
           35                  40                  45
   Phe Asn Ala Phe Ala Thr Ala Gly Gly Asn Arg Val Thr Val Tyr Xaa
       50                  55                  60
   Cys Leu Glu Gly Gly Val Ile Ala Val Leu Gln Ser Tyr Ile Asp Glu
   65                  70                  75                  80
   Asp Lys Asp Glu Ser Phe Tyr Thr Val Ser Trp Ala Cys Asn Ile Asp
                   85                  90                  95
   Gly Thr Pro Phe Leu Val Ala Gly Gly Leu Asn Gly Ile Ile Xaa Val
               100                 105                 110
   Ile Asp Thr Gly Asn Glu Lys Ile Tyr Lys Ser Leu
           115                 120
<210> 51
   <211> 631
   <212> DNA
   <213> Zea mays
<220>
   <221> unsure
   <222> (466)
<220>
   <221> unsure
   <222> (564)
<220>
   <221> unsure
   <222> (601)
<220>
   <221> unsure
   <222> (603)
<220>
   <221> unsure
   <222> (607)
<400> 51
   ggcacgagtc caacaaaata tgtccagttt ccagtcttga ttgctgcagt acactctaac  60
   tatgttgatt gtacaagatg gcttggtgac ttcatcctat caaagagtgt tgacaatgaa 120
   attgtgcttt gggaaccgaa gacaaaagaa cagagtcctg gggagggaag catcgatatc 180
   cttcagaagt atcctgtccc agaatgtgac atttggttta tcaaattttc atgtgatttt 240
   cacttcaatc agttggcgat aggcaaccgt gaaggcaaaa tctacgtgtg ggaagtacag 300
   tccagccctc ctgtcctcat tgctcggctg tataatcagc agtgtaaatc gccgataaga 360
   caaactgcag tgtccttcga tggaacacaa tccttggagc tggtgaagac gcaccatctg 420
   gcggtgggga tgaagtggac catccgagct ccagaaactg aagaantttg ccgctcaatg 480
   ctggactgat ggttacgctc ggttggggtt gcgatggttg aaccgtggtg gaaatgccac 540
   tggtgttttt caatcaaaat ggtnggtgtt aacagaataa tgaatgctcc aaagttgaaa 600
   ntnggangct gttgctaaaa aaaaaaaaaa a                                631
<210> 52
   <211> 129
   <212> PRT
   <213> Zea mays
<400> 52
   Pro Thr Lys Tyr Val Gln Phe Pro Val Leu Ile Ala Ala Val His Ser
   1               5                   10                  15
   Asn Tyr Val Asp Cys Thr Arg Trp Leu Gly Asp Phe Ile Leu Ser Lys
               20                  25                  30
   Ser Val Asp Asn Glu Ile Val Leu Trp Glu Pro Lys Thr Lys Glu Gln
           35                  40                  45
   Ser Pro Gly Glu Gly Ser Ile Asp Ile Leu Gln Lys Tyr Pro Val Pro
       50                  55                  60
   Glu Cys Asp Ile Trp Phe Ile Lys Phe Ser Cys Asp Phe His Phe Asn
   65                  70                  75                  80
   Gln Leu Ala Ile Gly Asn Arg Glu Gly Lys Ile Tyr Val Trp Glu Val
                   85                  90                  95
   Gln Ser Ser Pro Pro Val Leu Ile Ala Arg Leu Tyr Asn Gln Gln Cys
               100                 105                 110
   Lys Ser Pro Ile Arg Gln Thr Ala Val Ser Phe Asp Gly Thr Gln Ser
           115                 120                 125
   Leu
   129
<210> 53
   <211> 777
   <212> DNA
   <213> Zea mays
<220>
   <221> unsure
   <222> (597)
<220>
   <221> unsure
   <222> (611)
<220>
   <221> unsure
   <222> (639)
<220>
   <221> unsure
   <222> (657)
<220>
   <221> unsure
   <222> (681)
<220>
   <221> unsure
   <222> (692)
<220>
   <221> unsure
   <222> (699)
<220>
   <221> unsure
   <222> (702) ,
<220>
   <221> unsure
   <222> (710)
<220>
   <221> unsure
   <222> (718)
<220>
   <221> unsure
   <222> (798)..(749)
<220>
   <221> unsure .
   <222> (753)
<220>
   <221> unsure
   <222> (772)
<400> 53
   ggcacgaggt tagctaagag ctttgttggc catggcgact caataaatga gataagaact  60
   caaccgttga agccttcgct catcatttct gcaagcaagg atgaatctgt taggctatgg 120
   aatgtccata cagggatctg tatcttgata tttgctggag ctggaggtca tcgcaatgaa 180
   gtattgagtg ttgacttcca tcctagtgat attgaacgtt ttgcaagttg tggcatggac 240
   aacactgtga aaatctggtc aatgaaagaa ttttggctat atgttgacaa atcatattca 300
   tggactgacc ttccatcaaa gtttccaaca aaatatgtcc agtttccagt cttgattgct 360
   gcagtacact ctaactatgt tgattgtaca agatggcttg gtgacttcat cctatcaaag 420
   agtgttgaca atgaaattgt gctttgggaa ccgaagacaa aagacagatc ctgggggaag 480
   gaagcatcga tatccttcag aagtacctgt cccagaatgt gacattgggt ttatcaaatt 540
   ttcatgtgat tttcacttca atcagtggcg ataggcaacc gtgaaagcaa atctacnttt 600
   gggaagtaca nccagccctc tgtcctcatg ctcgctgtnt atcacatgta atcccanaaa 660
   aaacgcatgt ctccatgaac ncatcctgga cnggtaaang cncactgcgn ggaaaatnac 720
   accacccaac gaaaattccc catccganna gtnccgtggg tcaagtaact gngaatc    777
<210> 54
   <211> 158
   <212> PRT
   <213> Zea mays
<400> 54
   Lys Ser Phe Val Gly His Gly Asp Ser Ile Asn Glu Ile Arg Thr Gln
   1               5                   10                  15
   Pro Leu Lys Pro Ser Leu Ile Ile Ser Ala Ser Lys Asp Glu Ser Val
               20                  25                  30
   Arg Leu Trp Asn Val His Thr Gly Ile Cys Ile Leu Ile Phe Ala Gly
           35                  40                  45
   Ala Gly Gly His Arg Asn Glu Val Leu Ser Val Asp Phe His Pro Ser
       50                  55                  60
   Asp Ile Glu Arg Phe Ala Ser Cys Gly Met Asp Asn Thr Val Lys Ile
   65                  70                  75                  80
   Trp Ser Met Lys Glu Phe Trp Leu Tyr Val Asp Lys Ser Tyr Ser Trp
                   85                  90                  95
   Thr Asp Leu Pro Ser Lys Phe Pro Thr Lys Tyr Val Gln Phe Pro Val
               100                 105                 110
   Leu Ile Ala Ala Val His Ser Asn Tyr Val Asp Cys Thr Arg Trp Leu
           115                 120                 125
   Gly Asp Phe Ile Leu Ser Lys Ser Val Asp Asn Glu Ile Val Leu Trp
       130                 135                 140
   Glu Pro Lys Thr Lys Asp Arg Ser Trp Gly Lys Glu Ala Ser
   145                 150                 155
<210> 55
   <211> 466
   <212> DNA
   <213> Zea mays
<220>
   <221> unsure
   <222> (305)
<220>
   <221> unsure
   <222> (441)
<400> 55
   gaagcaatgg gatcattcgg gtcatcaatt gtgctacaga aaagttagct aagagctttg  60
   ttggccatgg cgactcaata aatgagataa gaactcaacc gttgaagcct tcgctcatca 120
   tttctgcaag caaggatgaa tctgttaggc tatggaatgt ccatacaggg atctgtatct 180
   tgatatttgc tggagctgga ggtcatcgca atgaagtatt gagtgttgac ttccatccta 240
   gtgatattga acgttttgca agttgtggca tggacaacac tgtgaaaatc tggtcaatga 300
   aaganttttg gctatatgtt gacaaatcat attcatggac tgaccttcca tcaaagtttc 360
   caacaaaata tgtccagttt ccagtcttga ttgctgcagt acactctaac tatgttgatt 420
   gtacaagatg gcttggtgac ntcatcctat caaagagtgt tgacaa                466
<210> 56
   <211> 152
   <212> PRT
   <213> Zea mays
<220>
   <221> UNSURE
   <222> (99)
<220>
   <221> UNSURE
   <222> (145)
<400> 56
   Gly Ile Ile Arg Val Ile Asn Cys Ala Thr Glu Lys Leu Ala Lys Ser
    1               5                  10                  15
   Phe Val Gly His Gly Asp Ser Ile Asn Glu Ile Arg Thr Gln Pro Leu
               20                  25                  30
   Lys Pro Ser Leu Ile Ile Ser Ala Ser Lys Asp Glu Ser Val Arg Leu
           35                  40                  45
   Trp Asn Val His Thr Gly Ile Cys Ile Leu Ile Phe Ala Gly Ala Gly
       50                  55                  60
   Gly His Arg Asn Glu Val Leu Ser Val Asp Phe His Pro Ser Asp Ile
    65                 70                  75                  80
   Glu Arg Phe Ala Ser Cys Gly Met Asp Asn Thr Val Lys Ile Trp Ser
                   85                  90                  95
   Met Lys Xaa Phe Trp Leu Tyr Val Asp Lys Ser Tyr Ser Trp Thr Asp
               100                 105                 110
   Leu Pro Ser Lys Phe Pro Thr Lys Tyr Val Gln Phe Pro Val Leu Ile
           115                 120                 125
   Ala Ala Val His Ser Asn Tyr Val Asp Cys Thr Arg Trp Leu Gly Asp
       130                 135                 140
   Xaa Ile Leu Ser Lys Ser Val Asp
   145                 150
<210> 57
   <211> 464
   <212> DNA
   <213> Zea mays
<220>
   <221> unsure
   <222> (369)
<220>
   <221> unsure
   <222> (447)
<400> 57
   ggcaaaatct acgtgtggga agtacagtcc agccctcctg tcctcattgc tcggctgtat  60
   aatcagcagt gtaaatcgcc gataagacaa actgcagtgt ccttcgatgg aagcacaatc 120
   cttggagctg gtgaagacgg caccatctgg cggtgggatg aagtggacca tccgagctcc 180
   agaaactgaa gaagtgttgc cgctcaatgc tggactgatg gttacgctcg gttggggttg 240
   cgatggttga atccgttggt ggaaagtgcc acctggtgtt ttttctagtc aaaatggttg 300
   gtgttaacag aatattgaat gcttcgaatg ttgaaagttg ggatgcttgt gctggtactc 360
   tgctccgtng acgagtgaac ttaggtgccg tttggttcac atatttgtaa cgtaatgggt 420
   aacagataac gttaaatcat gtttgtntta attcaaccgt aaaa                 464
<210> 58
   <211> 55
   <212> PRT
   <213> Zea mays
<400> 58
   Gly Lys Ile Tyr Val Trp Glu Val Gln Ser Ser Pro Pro Val Leu Ile
    1               5                  10                  15
   Ala Arg Leu Tyr Asn Gln Gln Cys Lys Ser Pro Ile Arg Gln Thr Ala
               20                  25                  30
   Val Ser Phe Asp Gly Ser Thr Ile Leu Gly Ala Gly Glu Asp Gly Thr
           35                  40                  45
   Ile Trp Arg Trp Asp Glu Val
       50                  55
<210> 59
   <211> 299
   <212> DNA
   <213> Zea mays
<220>
   <221> unsure
   <222> (203)
<220>
   <221> unsure
   <222> (270)
<400> 59
   ggcaaccgtg aaggcaaaat ctacgtgtgg gaagtacagt ccagccctcc tgtcctcatt  60
   gctcggctgt ataatcagca gtgtaaatcg ccgataagac aaactgcagt gtccttcgat 120
   ggaagcacaa tccttggagc tggtgaagac ggtaccatct ggcggtggga tgaagtggac 180
   catccgagct ccagaaactg aanaagtgtt gccgctcaat gctggactga tggttacgct 240
   cggttggggt tgcgatggtt gaatccgttn gtggaaagtg ccacctggtg ttttttcta   299
<210> 60
   <211> 59
   <212> PRT
   <213> Zea mays
<400> 60
   Gly Asn Arg Glu Gly Lys Ile Tyr Val Trp Glu Val Gln Ser Ser Pro
    1               5                  10                  15
   Pro Val Leu Ile Ala Arg Leu Tyr Asn Gln Gln Cys Lys Ser Pro Ile
               20                  25                  30
   Arg Gln Thr Ala Val Ser Phe Asp Gly Ser Thr Ile Leu Gly Ala Gly
           35                  40                  45
   Glu Asp Gly Thr Ile Trp Arg Trp Asp Glu Val
       50                  55
<210> 61
   <211> 450
   <212> DNA
   <213> Zea mays
<220>
   <221> unsure
   <222> (223)
<220>
   <221> unsure
   <222> (259)
<220>
   <221> unsure
   <222> (263)
<220>
   <221> unsure
   <222> (267)
<220>
   <221> unsure
   <222> (288)
<220>
   <221> unsure
   <222> (385)
<220>
   <221> unsure
   <222> (390)
<220>
   <221> unsure
   <222> (428)
<220>
   <221> unsure
   <222> (432)
<220>
   <221> unsure
   <222> (434)
<400> 61
   caacatctgg cacagcatgc cgccttccaa agcacgccga aagaggtcac ttcgtgatat  60
   cactgccacc gttgccactg ggactgttgc caactcgaaa cctggctcat catcgacgaa 120
   cgaggggaag caacaggaca agaaaaagga gggtccacag gaaaccggac atcccaccat 180
   taccgccggt ggtggtgaat atagtcccac gaacaaggat tangatgttg aaattagtgg 240
   aagggctact cgtgcctant ccngaancga aaattacaac cccaatancc aattattctg 300
   ttggggaaat ccacccgatc ttatgccatc cgggtttcca atttcccttg aaaatgccct 360
   actattaaat tttttttggc caccnccccn tttgcaataa ttgtttaaaa attttaccaa 420
   aacccttnaa angngggggt tttggggccc                                   450
<210> 62
   <211> 363
   <212> PRT
   <213> Zea mays
<400> 62
   Leu Gly Cys Glu Val Val Glu Gly Leu Leu Val Pro Ser Arg Lys Arg
    1               5                  10                  15
   Glu Tyr Lys Pro Asn Ser Lys Tyr Thr Val Gly Asn His Pro Ile Tyr
               20                  25                  30
   Ala Ile Gly Phe Asn Phe Ile Asp Met Arg Tyr Tyr Asp Val Phe Ala
           35                  40                  45
   Ile Ala Ser Cys Asn Ser Val Ile Ile Tyr Arg Cys Leu Glu Asn Gly
       50                  55                  60
   Gly Phe Gly Leu Leu Gln Asn Tyr Val Asp Glu Asp Lys Asp Glu Ser
   65                  70                  75                  80
   Phe Tyr Thr Leu Ser Trp Thr Ile Asp Gln Val Asp Ser Ser Pro Leu
   85 90 95
   Leu Val Ala Ala Gly Ser Asn Arg Ile Ile Arg Val Ile Asn Cys Ala
   100 105 110
   Thr Glu Lys Leu Asp Lys Ser Leu Val Gly His Gly Gly Ser Ile His
   115 120 125
   Glu Ile Arg Thr His Ala Ser Lys Pro Ser Leu Ile Ile Ser Ala Ser
   130 135 140
   Lys Asp Glu Ser Ile Arg Leu Trp Asn Val His Thr Gly Ile Cys Ile
   145 150 155 160
   Leu Val Phe Ala Gly Ala Gly Gly His Arg His Asp Val Leu Ser Val
   165 170 175
   Asp Phe His Pro Thr Glu Val Gly Ile Phe Ala Ser Cys Gly Met Asp
   180 185 190
   Asn Thr Val Lys Ile Trp Ser Met Lys Glu Phe Trp Ile Tyr Val Glu
   195 200 205
   Lys Ser Tyr Ser Trp Thr Gly His Pro Ser Lys Phe Pro Thr Arg Asn
   210 215 220
   Ile Gln Phe Pro Val Leu Thr Ala Ala Val His Ser Asp Tyr Val Asp
   225 230 235 240
   Cys Thr Arg Trp Leu Gly Asp Phe Ile Leu Ser Lys Ser Val Lys Asn
   245 250 255
   Ala Val Leu Leu Trp Glu Pro Lys Pro Asp Lys Arg Arg Pro Gly Glu
   260 265 270
   Gly Ser Val Asp Val Leu Gln Lys Tyr Pro Val Pro Lys Cys Ser Leu
   275 280 285
   Trp Phe Met Lys Phe Ser Cys Asp Phe Tyr Ser Asn Gln Met ala Ile
   290 295 300
   Gly Asn Asn Lys Gly Glu Ile Tyr Val Trp Glu Val Gln Ser Ser Pro
   305 310 315 320
   Pro Val Leu Ile Asp Arg Leu Cys Asn Gln Glu Cys Lys Ser Pro Ile
   325 330 335
   Arg Gln Thr Ala Val Ser Phe Asp Gly Ser Thr Ile Phe Gly Ala Ala
   340 345 350
   Asp Asp Gly Ala Ile Trp Arg Trp Asp Glu Val
   355 360
<210> 63
   <211> 524
   <212> DNA
   <213> Zea mays
<220>
   <221> unsure
   <222> (297)
<220>
   <221> unsure
   <222> (323)
<220>
   <221> unsure
   <222> (351)
<220>
   <221> unsure
   <222> (354)
<220>
   <221> unsure
   <222> (390)
<220>
   <221> unsure
   <222> (404)
<220>
   <221> unsure
   <222> (427)
<220>
   <221> unsure
   <222> (450)
<220>
   <221> unsure
   <222> (473)
<220>
   <221> unsure
   <222> (481)
<220>
   <221> unsure
   <222> (486)
<400> 63
   gcaatgaaat tgtgctttgg gaaccgaaga caaaagaaca gagtcctggg gagggaagca  60
   tcgatatcct tcagaagtat cctgtcccag aatgtgacat ttggtttatc aaattttcat 120
   gtgattttca cttcaatcag ttggcgatag gcaaccgtga aggcaaaatc tacgtgtggg 180
   aagtacagtc cagccctcct gtcctcattg ctcggctgta taatcagcag tgtaaatcgc 240
   cgataagaca aactgcagtg tccttcgatg gaagcacaat ccttggagct ggtgaanacg 300
   caccatctgg cggtgggatg aantggacca tccgagctcc agaaactgaa naantgttgc 360
   cgctcaatgc tggactgatg gttacgctcn gttggggttg ccanggttga atccgttggt 420
   ggaaaantgc cacctgggtg ttttttctan tcaaaatggg ttggtgttaa canaatattg 480
   naatgnttcc aaatgttgaa aaatttggga tgcttgtgcc tggt                 524
<210> 64
   <211> 98
   <212> PRT
   <213> Zea mays
<210> 65
   <211> 513
   <212> DNA
   <213> Oryza sativa
<220>
   <221> unsure
   <222> (364)
<220>
   <221> unsure
   <222> (452)
<220>
   <221> unsure
   <222> (458)
<220>
   <221> unsure
   <222> (480)
<220>
   <221> unsure
   <222> (499)
<400> 65
   gtggaatgtt cacacaggga tctgcatttt gatttttgct ggagcaggag gtcaccggaa  60
   tgaagtattg agtgttgact tccacccatc tgatatctac cgcatagcaa gttgtggcat 120
   ggataacact gttaaaatat ggtcaatgaa ggaattctgg ccatatgttg agcaatcctt 180
   tacatggact gaccttccat caaaatttcc aacaaaatat gtgcaatttc cggtcttggt 240
   tgctgtagta cattctaact atgttgattg tactagatgg cttggtgact tcattctgtc 300
   aaagagtgtt gacaatgaaa ttgtgctgtg ggagccaaaa acaaaagaac aaagtcccgg 360
   gganggtagc attgatattc ttcagaagta tcctgtgcca gaatgtgata tctgggttat 420
   caaatctcat gcgattcact caatcaattg gnataggnac cgtgaaggaa aatcttgtcn 480
   ggaatacatc aatcctccng tttaacgccc gcg                               513
<210> 66
   <211> 141
   <212> PRT
   <213> Oryza sativa
<220>
   <221> UNSURE
   <222> (121)
<400> 66
   Trp Asn Val His Thr Gly Ile Cys Ile Leu Ile Phe Ala Gly Ala Gly
   1               5                   10                  15
   Gly His Arg Asn Glu Val Leu Ser Val Asp Phe His Pro Ser Asp Ile
               20                  25                  30
   Tyr Arg Ile Ala Ser Cys Gly Met Asp Asn Thr Val Lys Ile Trp Ser
           35                  40                  45
   Met Lys Glu Phe Trp Pro Tyr Val Glu Gln Ser Phe Thr Trp Thr Asp
       50                  55                  60
   Leu Pro Ser Lys Phe Pro Thr Lys Tyr Val Gln Phe Pro Val Leu Val
   65                  70                  75                  80
   Ala Val Val His Ser Asn Tyr Val Asp Cys Thr Arg Trp Leu Gly Asp
                   85                  90                  95
   Phe Ile Leu Ser Lys Ser Val Asp Asn Glu Ile Val Leu Trp Glu Pro
               100                 105                 110
   Lys Thr Lys Glu Gln Ser Pro Gly Xaa Gly Ser Ile Asp Ile Leu Gln
           115                 120                 125
   Lys Tyr Pro Val Pro Glu Cys Asp Ile Trp Val Ile Lys
       130                 135                 140
<210> 67
   <211> 534
   <212> DNA
   <213> Glycine max
<400> 67
   taagagtttt gttggccatg gagactccat aaatgaagtc aaagctcaaa tattaaatcc  60
   atcactcgtg gtatcggcaa gcaaagatga atctattcgg ttatggaatg ctcatactgg 120
   aatatgcatt ttgatatttg ctggaggcgg gggacatcgt aatgaagtct taagtgttga 180
   ttttcatcca tcggatatgt atcgtatttg tagttgtggc atggatagta ctgtaaaaat 240
   atggtctatg aaggagttct ggacatatgt agaaaaatca tccacatgga cagatcttcc 300
   ttccaagttt ccaacaaaat ttgtccagtt tcctgtttac actgcttcag tgcatataaa 360
   ttatgttgac tgtaataggt ggttgggtga ttttatcctc tcaaagagtg ttgataatga 420
   aattatcttg tgggaaccta aagtgaacga accaactcca gggaagggtg tagttgatgt 480
   cctcataaat acccatttcc gattgcaata tctgggtcat cagttttctt gtga       534
<210> 68
   <211> 162
   <212> PRT
   <213> Glycine max
<400> 68
   Lys Ser Phe Val Gly His Gly Asp Ser Ile Asn Glu Val Lys Ala Gln
   1               5                   10                  15
   Ile Leu Asn Pro Ser Leu Val Val Ser Ala Ser Lys Asp Glu Ser Ile
               20                  25                  30
   Arg Leu Trp Asn Ala His Thr Gly Ile Cys Ile Leu Ile Phe Ala Gly
           35                  40                  45
   Gly Gly Gly His Arg Asn Glu Val Leu Ser Val Asp Phe His Pro Ser
       50                  55                  60
   Asp Met Tyr Arg Ile Cys Ser Cys Gly Met Asp Ser Thr Val Lys Ile
   65                  70                  75                  80
   Trp Ser Met Lys Glu Phe Trp Thr Tyr Val Glu Lys Ser Ser Thr Trp
                   85                  90                  95
   Thr Asp Leu Pro Ser Lys Phe Pro Thr Lys Phe Val Gln Phe Pro Val
               100                 105                 110
   Tyr Thr Ala Ser Val His Ile Asn Tyr Val Asp Cys Asn Arg Trp Leu
           115                 120                 125
   Gly Asp Phe Ile Leu Ser Lys Ser Val Asp Asn Glu Ile Ile Leu Trp
       130                 135                 140
   Glu Pro Lys Val Asn Glu Pro Thr Pro Gly Lys Gly Val Val Asp Val
   145                 150                 155                 160
   Leu Ile
       162
<210> 69
   <211> 584
   <212> DNA
   <213> Triticum aestivum
<220>
   <221> unsure
   <222> (350)
<220>
   <221> unsure
   <222> (366)
<220>
   <221> unsure
   <222> (429)
<220>
   <221> unsure
   <222> (440)
<220>
   <221> unsure
   <222> (469)
<220>
   <221> unsure
   <222> (476)
<220>
   <221> unsure
   <222> (493)
<220>
   <221> unsure
   <222> (496)
<220>
   <221> unsure
   <222> (511)
<220>
   <221> unsure
   <222> (555)
<220>
   <221> unsure
   <222> (562)
<400> 69
   ccggagccgg gagtacaagc tctgcagcaa gcacaccgag ggcaagcgcc cgctctacgc  60
   catcggcttc aacttcatcg acgcccgcta ctacgacgtc ttcgccaccg tcggcggcaa 120
   tcgtgtgacg acgtaccgtg gcctccccga cggtaacttg gctgttctgc aagcatacat 180
   tgatgcggac gatgctcagt cattctacac tctgagctgg gcttgtgacc ttgacggcac 240
   accactgcta gtggcagcag gaagcaatgc ggtcattcgg gtcatcaact gtgccaaccg 300
   agaatttgtt aagagtttcc tgggcaatgg ggaatcatta attgggatan gatccaacca 360
   ttgaancttc gtcttaattc tgcaagcaag gacaatctgt tagctatgga atgtcaatac 420
   aaggtatcng atcttgattn gctgggagga agaagtcacc gtaatgaant attgantgtt 480
   gactcaacct tcnganatca acgaattgca ntgtggaagg taatacgtta aatcgggcaa 540
   tgaaaaattt ggcanactgg anaatcttta atgacgactc acaa                  584
<210> 70
   <211> 111
   <212> PRT
   <213> Triticum aestivum
<400> 70
   Arg Glu Tyr Lys Leu Cys Ser Lys His Thr Glu Gly Lys Arg Pro Leu
    1               5                  10                  15
   Tyr Ala Ile Gly Phe Asn Phe Ile Asp Ala Arg Tyr Tyr Asp Val Phe
               20                  25                  30
   Ala Thr Val Gly Gly Asn Arg Val Thr Thr Tyr Arg Gly Leu Pro Asp
           35                  40                  45
   Gly Asn Leu Ala Val Leu Gln Ala Tyr Ile Asp Ala Asp Asp Ala Gln
       50                  55                  60
   Ser Phe Tyr Thr Leu Ser Trp Ala Cys Asp Leu Asp Gly Thr Pro Leu
   65                  70                  75                  80
   Leu Val Ala Ala Gly Ser Asn Ala Val Ile Arg Val Ile Asn Cys Ala
                   85                  90                  95
   Asn Arg Glu Phe Val Lys Ser Phe Leu Gly Asn Gly Glu Ser Leu
               100                 105                 110
<210> 71
   <211> 4735
   <212> DNA
   <213> Zea mays
<400> 71
   aagcttttgt tttagccaag atttgagatt cgatttgaag tgtggaagtc cttccaattt   60
   gccaatccta tatttgatct ctgctgtgct gcgttaaatc cctaaacttc acagcgcggc  120
   gccggcccag ccacgccgga agaggtcgcc gcgtgaggtc agtgtccccg ttgctgccgc  180
   ctctaacccg aagcctaggc cgctgccggt gcataacaag gagaatcagg cggaggggaa  240
   agtagcagag gagggggcag caactgagga gggggagaag taccgggcgg aaccggaaat  300
   cttgccgctg ccgccggcca tggcgaagct gggcccgggg caggggctcg ggtgcgaggc  360
   ggcggagggg tcgctcgtgc ccagccggaa gcgggagtac caagccctgc ggcaagcaca  420
   ctgaggggaa gcgcccgcta tatgctatcg ggttcaactt catggacgcg cgctactacg  480
   acgtcttcgc caccgtcggc ggcaaccgcg taagccatcg actgctctct cctgtcgtcc  540
   tttttttgtt tctactgagg tttggggagt tcttgttgat taatggcaag gtaaaactac  600
   gttgtttttt tttgtgattt tggtggtcgg ttttaggaag cggtcgcttt tgattcaaat  660
   ttgatctaaa gctgaggcat tcggttgttt ttattgggga cttgaggtct gtaatgttcc  720
   gactattgtg atttgttttg ccgaaacatg gagtttgcta gttcatttga tgaaaagctg  780
   caacctttga caaagaattt gtatcacttg ggaaagtata gtgaggtgtg gggaatcaga  840
   tagtaccaat attactttga ctatgattat aagataatct tttaatgtcc tttgtaacga  900
   ccatgctgct tttcgcttat cttgcctatt gatcttgcag gtgacaactt accgctgcct  960
   tgagaatggt agtttcgctc ttctacaagc ttacgttgat gaggatgtaa gaaagacaat 1020
   gctcaatgac aatgcttttg cttgctgatt taatattgat aatattcttt ctctaattct 1080
   tgtgacgcct atttacctca gaaggatgag tcgttctata ctctaagctg ggctcgtgac 1140
   catgttgatg gctcaccact gctggtggca gcaggaagca atgggatcat tcgggtcatc 1200
   aattgtgcta cagaaaagtt agctaaggta atctaccctt atatttgtat gtgttcctat 1260
   ggtaaacttg aatgaagcct tatttgcata attcaatatt tcagttgttt atttgacata 1320
   tatcacttta tttatgatat ctgatccaga aggtcttttg gatttgcttt agttaaggaa 1380
   tggtgcttgc tacgcattaa taccataagc aaactgtacc ttttgctcac agaatattgt 1440
   taattttgac tacttcagta tgtccgttgt agtaaaaaca aatcaacttg gtgtatctat 1500
   tttttccttg cttatacata gccaggagat tgggcatgtg gcatgtcaat aaatactatc 1560
   ctataccatt tgataggaca cgcactgtgt cttatttggt agctctgttt acgtgattct 1620
   gcagagcttt gttggccatg gcgactcaat aaatgtgata agaactcaac cgttgaagcc 1680
   ttcgctcatc atttctgcaa gcaaggttat gcgatagtct gttcttaggt tcatgtacct 1740
   ttttattttt ataatctttc tgaatttgga caccatttca tatggcatta tctaatagga 1800
   tgaatctgtt aggctatgga atgtccatac agggatctgt atcttgatat ttgctggagc 1860
   tggaggtcat cgcaatgaag tattgagtgt tgtaagtagt gcctgctatt atgacattgt 1920
   gcccttcaaa aaaaacatta ttatgacatt atttttagaa cattactagg ttaaggtgcc 1980
   tttaatatgg cgcactcttt cagctcctga tattaccatt tgttattgag cgttacatca 2040
   gagataaaat aaggctacct aatgactgct actgcttttg tactttgatt acattagtca 2100
   taaatgtact gatgaataca ttattttgtc ttaaggactt ccatcctagt gatattgaac 2160
   gttttgcaag ttgtggcatg gacaacactg tgaaaatctg gtcaatgaaa ggttagaaag 2220
   ctacttcaaa gttgcttcat atttgcatgt tgcgtgtcat tgagttcacc aatgttgtcg 2280
   cagaattttg gctatatgtt gacaaatcat attcatggac tgaccttcat caaagttcca 2340
   caaaatatgg ccagtttcca gtatgtttca caatgcctat atccaattat cctggcaagg 2400
   tcctgttggt gtctaatcct catgccatca gactgacctg tttctttttg tttcaggtct 2460
   tgattgctgc agtacactct aactatgttg attgaacaag atggcttggt gacttcatcc 2520
   tatcaaaggt gaaatttctg attcgtttaa atggatacaa atttctgtag cacggttgtc 2580
   actcttttgt gggtttgaca tgccactgtc ttggttcatc tattgctgta ccgtgcaagt 2640
   gttcagtttt ttcaatcttt tttctcagtg cttaatgagg ggagattcta tttgcagagt 2700
   gttgtcaatg aaattgtgct ttgggaaccg aagacaaaag aacagagtcc tggggaggta 2760
   attcagttta actttcccag aattgtattc ctattataat gccatatatt tacgcacagt 2820
   tgtaaactat ttccagatcc ttagatttca aggtactggc tgccaatatt aaatatgttc 2880
   cactgaagta atatgatttt ctgttgcctc atagggaagc atcgatatcc ttcagaagta 2940
   tcctgtccca gaatgtgaca tttggtttat caaattttca tgtgattttc acttcaatca 3000
   gttggcgata ggtaatatct ctcatcagga ttgtttctgg tagaagtttt atttaagatt 3060
   ttttttgctc tgtaaaattt cacacacgca cacatgcacc cccacacaca cacacatgca 3120
   cgcacacccc cacccacctg cacgcgcgcg tacacacaca ccgcacacat atatatgact 3180
   ttttttccca cacaaatatt tgctgtgtga gatatcagca aataaattcg tatgtttgat 3240
   tatattcaga gatataggaa aattgagtgc tctaataccc catccactac ttcaaacagg 3300
   caaccgtgaa ggcaaaatct acgtgtggaa aaatacagtc cagccctcct gtcctcattg 3360
   ctcggtagtt ttcactggaa gagtttcagt tattcttgtc tcccacttgt atcgtcgcat 3420
   gcttctggat gccaatgctt catcattttc aggctgtata atcagcagtg taaatcgccg 3480
   ataagacaaa ctgcagtgtc cttcgatgga aggtacctca ctctaatcca tgctcaattt 3540
   ggtgtactgt ctattctagc acttgctttt ttcttggttc tgcttgagaa attctcgatt 3600
   gcatgtcata tgctggtgca ttttcttttt tctgtttccg tggcggattg gtaaaatgcg 3660
   acgatgcctt ccttatctag cacaatcctt ggagctggtg aagacggcac catctggcgg 3720
   tgggatgaag tggaccatcc gagctccaga aactgaagaa gtgttgccgc tcaatgctgg 3780
   actgatggtt acgctcggtt ggggttgtga tggttgaatc cgttggcgga aagtgccacc 3840
   tggtgttttt ttctagtcaa aatggttgat gttaacagaa tattgaatgc ttcgaatgtt 3900
   gaaagttggg atgcttgtgc tggtactctg ctccgcggac gagtgaactt agtttgttgc 3960
   aactttggga accgttgtca tctgtttgtt ctgcatttct aaaaagagag caaatttcag 4020
   gatacatgtt cttttttttc agtacaggaa aactaaggtt gaggtattgc tttgcaattt 4080
   actctctctc tctctctctc ttaaaaaaac tggatcttgc ttcaacgatg cattccttgg 4140
   gtcatcggtt ttacttttga aatcttgata gctgggccta aagttaccaa gcccactagt 4200
   atcagaagta ataatatgat ggctcctccc ctgccttact gtcacgtgta aactttcgaa 4260
   actagcagga ctgtagcatt tagcgagctg gttgtttggg ttagagctca gcgtcgcaac 4320
   ttatggtacc gaggtcagtg tcaagatcta tggcaccatg gttcaatcac agttttagtc 4380
   ccaccaaaaa tataaaggtg aagtttcgac aaaaaatggc tagaataaaa aaaaacaggt 4440
   ccacatactg aggagaacac atgacagatt caccaaggat tttgaattga aagaggctaa 4500
   tgattgacag gatttgatct tcaattccac ctcccgttgt cctgcttcta ctctaaagtt 4560
   caagcgtggc tcagtttggc tatctgttat aatttcaaga aatcctgatt tctgttagca 4620
   gtttactagg ctattaggag gagctgggac aaaagaaaaa cgagaattga cgaggacaaa 4680
   ttcgcaatta gttgggaaat tgggggcaca attttcaatg cccacaaaat tcact      4735
<210> 72
   <211> 7525
   <212> DNA
   <213> Zea mays
<220>
   <221> unsure
   <222> (5878)
<220>
   <221> unsure
   <222> (5975)
<400> 72
   aagctttgag acttgatttg aagtattaaa taaacccttc aaatttcttt ctaactttga   60
   taatacacta ttcaatgaca atgcacttcc ttaaatccct atacttcaca gcatgccgcc  120
   ttccaaagca cgccgaaaga ggtcacttcg tgatatcact gccaccgttg ccactgggcc  180
   tgttgccaac tcgaaacctg gctcatcatc gacgaacgag gggaagcaac atgacaagaa  240
   aaaggagggt ccacaggaac cggacatccc accattaccg ccggtggtgg tgaatatagt  300
   cccacgacaa ggattaggat gtgaagtagt ggaagggcta ctcgtgccta gtcggaagcg  360
   agagtacaag cccaatagca agtatactgt gggaaatcac ccgatctatg ccatcgggtt  420
   caatttcatt gacatgcgct actatgatgt ctttgccatc gccagttgca atagtgtaag  480
   caaccgactt ctccctacct cttgtttgct atccatttat cctattgagg tttggggagt  540
   tctatatggt gaacgaaaat ggaagttatg attttggtgg gattggatct tggtttataa  600
   ctagaaaagg atttgagtac aggttatgat gtgtggcttt atggtaggga aacttaatat  660
   cttttcctat tttgtttttt ggcatcacga gtaatggttt gggaaataaa agggaaaatg  720
   atttaaaatt atttctcaat agagcatgcc cttttacata gggacatttt agtcatttta  780
   cacacacttt agtcatttta cacaccgtaa ttatgtcaca atcaaagaat cattccttgg  840
   ttcaattgaa tgagatgatt caactagttc acatctctat acctaacaat atagtttttc  900
   ataactagaa ttcttaaaaa gaattaatat gaacctaaat attatttcac tttcttgccc  960
   cttataatat aatacatttg tcactcccat tttggcaagg gtggtgggta ttttggggga 1020
   tggaatgtta ctatttttaa tttgattaga agctataagc tttggctata tttttattag 1080
   gaatttgatg ttcattttca atatattgtg atctattttc ttaaaatgtg aatttgttgt 1140
   gtattttgat tagttcgatg aagagtgttt ataagatatg atttttaaat tctcttacga 1200
   cgaaacaata ttatgttact ttcatctatt catcttgagg aatcacctac ctcacttctt 1260
   gatcttgcag gtgataattt accgatgcct tgagaatggt ggttttggtc ttctacaaaa 1320
   ttatgttgat gaggatgtga gaaagacaat gcctggtgca tgtggttgtt aatgttaatt 1380
   tgataatatg cttttatcta atgtctgtgg tgcctattta tctcagaagg atgagtcatt 1440
   ctacactcta agctggacca tcgatcaagt tgatagctca ccgctgttgg tggccgctgg 1500
   aagcaatcgg atcattcggg tcatcaattg tgctaccgaa aagttagata aggtccctgc 1560
   ccctgtgctt actctatgtt tgtatggaaa agttgattga acgttgatgt tcacatatca 1620
   atatttcagt agtttagttg aaatacaatt tatttatgct ctctattctt gaacatcagt 1680
   tgactttgct ttgattaagc aatggtcttg ctcatacaat attctaggag ttgaatattc 1740
   aatatgcctg ttacatgata gcaaatacat agtgaactag gacatgtact aaatatttaa 1800
   tttcccttta tgacattctc tagagcttag ttggccatgg tggttcaata catgagataa 1860
   ggactcatgc ctcgaagcca tcactcatca tttctgccag caaggttagt aataaatttg 1920
   tcgtgtgtcg atttttttac actttttaac atgacattat tctataggat gaatctatta 1980
   ggctatggaa tgtccatact gggatttgca tcttagtctt tgcaggggct ggaggccatc 2040
   gacatgatgt gttgagtgtt gtaagtatcg attgcatctt gtctagacat tgttttaaat 2100
   atcacttgcc ccgaagataa cactcattag aattctaatg ttaccatttg ttattgagca 2160
   tgccaaattt caattttaac atcatagata aaataagacc ccacaattac ttttactgtt 2220
   tatctacttc cattacatta ggcataaagt tactgataaa aaagacaatc ttttatctga 2280
   aggacttcca ccctaccgag gttgggattt ttgcaagttg tggcatggac aatactgtga 2340
   agatttggtc aatgaaaggt ttgggaacta ctttaaacta gcttcatgtt tacattttgt 2400
   gttgtatgtt gcatatcatc gacaaatatt gccaatgttg tcacagaatt ttggatatat 2460
   gttgaaaaat catattcatg gactggccat ccatcaaagt ttccaacgag gaatatccag 2520
   tttccggtat gttaagtagc tataatcacc tgagctcctt tctttttttg caaactattg 2580
   ttggtgttca gttttcatgc cattcaagca tacatgtttc ttttctttta ggtcttgact 2640
   gctgcagtac actctgacta tgttgattgt accaagatgg cttggtgact tcatcctatc 2700
   aaaaggtaaa ttcttcattt gttaaatggc tatacatttt tttataaagg aaatttttta 2760
   ttaatttcaa gcactttaga ttgaaataat acaaaatctt aaaaaacatt tttggcctcc 2820
   atttaaacaa gcacaaatcc aacaaaaatg agtaaaccaa cccattctag tgaatattaa 2880
   tgcataaact agattgctac ccatatgtct agaaaaagta gccttgaccg cgtatcttaa 2940
   ttgtcaccat gccgccacaa ccaaaccgtg caaatatggt ttttggagaa tggaccaagt 3000
   aagaaaccaa tcaataattg agtatatagc atgcacagga gaaatagatc tcttattttc 3060
   aagaacaatg gtatttttta ttaaccatag gaccaacaag tagcgactac ccatagcaaa 3120
   actaatggct tcagattatt actggttgtt gaagtgtata cgtggtttgc ctactttctc 3180
   ccaatagttt aagcttttgg attgaatcga ttagtgcgtt cactcttaca tggtatcaaa 3240
   gttagcaatt ttgggtttga atcctaacgg aagctttatt tgtgacttca cctcttgttt 3300
   tccatttcct ttctacctgc acgtgagtgg gggtgttgaa gtgtataagt ggattgccta 3360
   ccttatcaac cttttggatt aaactggtta ttggttagtg tgttcactcc tacacctaag 3420
   tatgaggttt agttatccag tagccaatta gattatgcac agtggacact tcacatgtgc 3480
   aactagcact caaaacataa gtctttaatt gtctcatctt atgacaaaac aacatatttc 3540
   actaccattc tataacatct tgatttgtac atcagtcttg ttaatgctaa atagtgagat 3600
   ttgatcgtca attggccagt tggatgtaaa ttccagtgaa atacatcttg accttgggtt 3660
   aaatggacat tagcaatgtg tgggaacaaa ttgttggttt gggtacacca aactgttggt 3720
   ttttaattag tagattagtt tgtaacacat ttccttttat cagtgttagt attggtttat 3780
   tatgcatagg gaaggatctg atatgtgata attaacatgg atttgcagag tgtaaagaat 3840
   gcagttttgc tttgggaacc aaaaccagac aagcgtaggc ctggggaggt gacacgcttt 3900
   accttctcgt cccgaattct gcacctattt ttatattact atcatactca tctacagttt 3960
   aaaacttgtc ccgcaatctt ttcagtttct gagcactaaa tttatacctc tgaatcagta 4020
   tagtcgtttt ctctttgttc gtatagggga gtgttgatgt tcttcagaag tacccggtgc 4080
   caaagtgttc attatggttt atgaaatttt catgtgattt ttactccaac cagatggcaa 4140
   taggtaatgc ctttaatttt gtgaagactg ttttggcact aaagctttac gtacgtaata 4200
   ttagttttat atcttgtaca ttgatggaaa atagattgct caatatctat atatatgact 4260
   atatcttggg ttagattcta aggaacaaac tctcccagag tacggttctg aataacaacc 4320
   atctgctgct gctgcttaat gcgaacaggc aacaataaag gcgagatcta tgtctgggaa 4380
   gtgcagtcca gcccgcccgt cttaattgac cggtaaattt ccagttcttc tcctcctcgc 4440
   atcggttcct gcatgggtag ctagctagta actccgacgc ttctgctgga tgcaaacact 4500
   tgtgcatttt caggctgtgc aaccaggaat gcaagtcgcc gataaggcag accgcagtgt 4560
   cattcgacgg aaggcacgta cgcactacga ctctcactat ctgctcatgc atgcattcac 4620
   cgcacgtacg tgtgatgtgc tcgctcgctt cctccttttg tgatggtgtc tctctcactt 4680
   gcccagcacg atcttggagc cgccgacgac ggcggatctg gcgcggtggg acgaagtgga 4740
   ccctgctgct tccagctcca aacctgatca agctgctgcg cccgccgccg gtgcgggtgc 4800
   cgacgccgac gccgacgcct gagcgagagg accgtcgtcg cccgccggtt cacatcgatc 4860
   gtactccgtg ctggctgatt acctttaccc attgggatgt tttggttcag agtcgccaga 4920
   tctagtgtgt ggctgaacgt tgaatgttag gatgctgctg cttgttatgc tctgagtctt 4980
   gagttctctt tgttaatttg caccgtggat gagatgaata acttgacgtt gcaactttgc 5040
   atcccatata tgccgtaaat ctgccgtctg ttgtttgttc tgcgttgtct agaattagtg 5100
   gagatgtgct ggatacaatg tatgctagtc tattaaaccg tgctccactc tgagataatc 5160
   gaccaacttg tcttattatt gaaagaactg tggaaaaaac caaaaaaagt cgttgtggtt 5220
   ttgtttatta tcaaatatat tttacataag acttaaaagt tttcattttt tcatgaattt 5280
   tttgaataaa ccgagtagtc aaagctaggg tcaaaaaggc aaacatatta tattttaaaa 5340
   tggagagaga gtacattgtt ttaagacgaa ttgtttaata caactcgaga atattctgat 5400
   acattaatcc tatgatatta ccataaaaaa cattaatcct atgatagagt gtataattac 5460
   aaatgcacaa aggttctttt catgtgaaat cgtattatag ataggggtca tagcgcgccc 5520
   ttgtccctac aacttacgat gttcatgagt taggttagaa aaaggttaga gcaagtatac 5580
   taaagtgaca tatgcaggct acaaggaatg ccacatcaga tttttggtga cgttgaagga 5640
   agaaaaatag agggagaaaa aagcgaacca attgcgaagg tgccttcttc caagggcacg 5700
   gtccatggag tgtggtagcc gacatcaagg tagaggatta tggtaaagtt atttgagcaa 5760
   gtgtctgaca actagcatga aggcttagga ttttctaaat gcatctttga gcgctattga 5820
   tgtagatgtt aatgattttt agggctgatg accaaaccaa agatgaacat gggaacgnaa 5880
   ggaaggttac tgaaagtgta taggccccta gtttagtctt cagtgactaa tgataatata 5940
   tattattgtg actaacaagt gttttataga aacanggaaa gttagatcac aataatagat 6000
   atgatcagga ttattatgtg gtacccatcc cttattgatg aaaatcaatg gttggttctc 6060
   ataggataat cgaaaaggtt aaggatcaac tgtaaatgga gttgttggac acttagagta 6120
   gtgatttgac cttttttctt tggtagtact ataaacggac atgaaatgcg tagctttacc 6180
   taaacaagtc tagttaagta tgatgatgca cacttgtgaa tactagtgct aggtaaaccc 6240
   atgagatctc atgtgaagtt cgaaacaaaa cctaattcga aaagtgatta aaacatgtga 6300
   cttaacaatg ttgtagtagc attggtcgag tttgatgggc acctgatatg ggtcactaga 6360
   catgagtgtg ccctgttgtg tttgagtgaa gcactagcat atcaggtgtg caacagatat 6420
   ggtgcaccca ggcaggacac ccaaagagct tgcaaaatta gcctaaaaca cttagtgctc 6480
   accagacata tctagtgtac tactagttat tctcgttata tatgaaccct attagttatt 6540
   cttgaattgc ttcgatcttt tacaaaggaa gtagtttttc cttcatctcc ataaactgtg 6600
   gttttccaaa ggcattaata ataagattta gtatattaaa ttcaaagttg aggtacttta 6660
   ttatcgtgaa accaacatta atactataga cttaactaag gagtctattg gtgcttcctt 6720
   ctcatgtatt ttcttcttga agtgttcctt catcttggtg ctaacgacga cattcaacaa 6780
   tgtgtgctct tacttgattg gtttgtatat atggtggtgt tcctttactt agtggcaaca 6840
   taccttatcg ataactaacc cttagtgaaa gaaatgaaaa tgtacatccc actgggaaat 6900
   cactcatacc cctaagagct aacttaatgg aacatcactc atagccctaa gggctagttg 6960
   gaagtacttt ctcatttcct gtataagggc tagttcatga ttcaacttct tctccatttc 7020
   ttggtgaact atcttagcac gattcctata aaaacatata caactaaaca aagggtggtg 7080
   gtactgaaca cagtggaccc aagcactcgg aaatgggaag gacaagttgc atggaaaaaa 7140
   cgacaggctg ggaactattg tgtcttgtca agcgtgttcg tccagctata ggacatgggt 7200
   atttataggg caactagagg ttggtatcct aaaatatgtc cagaccccta gttatcaact 7260
   acgttcctag ataatactgt acaacaaggt aattatagaa tagtaagttt gttattctaa 7320
   ctccaccccg acaggtgggt ccgttgtcgc ccggttgaga gtgggccctg ctcggccagg 7380
   tcattggcat tgtccgtgca gacgtgttcc caatatcgag gcaatgaagt tgtttgacac 7440
   ttcttcggga gtcggcgtga ggccttcgct tgctagcgcg aacttgccca cgagcgtcct 7500
   caccatgggc cccgctgaca agctt 7525